# EUROPEAN PATENT APPLICATION

(11) **EP 3 205 718 A1**
(43) Date of publication of application: **16.08.2017**
(21) Application number: 16155748.3
(22) Date of filing: 15.02.2016
(51) Int. Cl.: C12N 5/078, C12N 5/077, C12N 5/0789, C12N 5/071, C07K 14/47, C07K 14/46

(54) **MEANS AND METHODS FOR CELL DIFFERENTIATION**

(71) Applicant: Max-Planck-Gesellschaft zur Förderung der Wissenschaften e.V., 80539 München (DE)
(72) Inventor: REISCHAUER, Sven, 61231 Bad Nauheim (DE); STAINIER, Didier, 61381 Friedrichsdorf (DE)
(74) Representative: Vossius & Partner Patentanwälte Rechtsanwälte mbB

(57) **Abstract**

The present invention relates to a method for producing endothelial cells from progenitor cells, said method comprising inducing NPAS4 function in the progenitor cells. The present invention also encompasses a method for producing hematopoietic cells from progenitor cells, said method comprising inducing NPAS4 function in the progenitor cells. The present invention furthermore pertains to a method for producing cardiomyocytes from progenitor cells, said method comprising inhibiting NPAS4 function in the progenitor cell.

SEQUENCE LISTING

<110> Max-Planck-Gesellschaft zur Förderung der Wissenschaften e.V.

<120> MEANS AND METHODS FOR CELL DIFFERENTIATION

<130> Y3215 EP

<160> 88

<170> BiSSAP 1.3

<210> 1
<211> 802
<212> PRT
<213> Homo sapiens

<400> 1
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu
            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Thr Pro Thr Met Leu Pro Ser Phe Pro Glu Asn Ile Leu Ser
            340                 345                 350
Gln Glu Glu Cys Ser Ser Thr Asn Pro Leu Phe Thr Ala Ala Leu Gly

355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400
Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asp Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe


<210> 2
<211> 802
<212> PRT
<213> Pan troglodytes

<400> 2
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu
            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Thr Pro Thr Met Leu Pro Ser Phe Pro Glu Asn Ile Leu Ser
            340                 345                 350
Gln Glu Gln Cys Ser Ser Thr Asn Pro Leu Phe Thr Ala Ala Leu Gly
        355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400
Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495

Tyr Glu Asp Gln Leu Thr Pro Cys Thr Ser Thr Phe Pro Asp Gln Leu
                500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asp Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
                580                 585                 590
Gln Leu Arg Gly Pro Leu Ser Val Asp Val Pro Leu Val Pro Glu Gly
                595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
                660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
                740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 3
<211> 802
<212> PRT
<213> Gorilla gorilla

<400> 3
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125

Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu
            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Thr Pro Thr Met Leu Pro Ser Phe Pro Glu Asn Ile Leu Ser
            340                 345                 350
Gln Glu Gln Cys Ser Ser Thr Asn Pro Leu Phe Thr Ala Ala Leu Gly
        355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400
Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asp Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser

625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 4
<211> 802
<212> PRT
<213> Pongo abelii

<400> 4
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu

            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Thr Pro Thr Met Leu Pro Ser Phe Pro Glu Asn Ile Leu Ser
            340                 345                 350
Gln Glu Gln Cys Ser Ser Thr Asn Pro Leu Phe Thr Ala Ala Leu Gly
        355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400
Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala Gln
        515                 520                 525
Glu Gln Leu Thr Pro Pro Ser Thr Ala Phe Gln Ala His Leu Asp Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765

Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe



<210> 5
<211> 802
<212> PRT
<213> Nomascus leucogenys

<400> 5
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu
            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Thr Pro Thr Met Leu Pro Ser Phe Pro Glu Asn Ile Leu Ser
            340                 345                 350
Gln Glu Gln Cys Ser Ser Thr Asn Pro Leu Phe Thr Ala Ala Leu Gly
        355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400

Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Asn Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asp Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 6
<211> 802
<212> PRT
<213> Macaca mulatta

<400> 6
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30

Asp Lys Val Arg Leu Ser Tyr Leu His Ile Met Ser Leu Ala Cys Ile
            35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu
            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Ala Pro Thr Met Leu Pro Ser Phe Pro Glu Asn Ile Leu Ser
            340                 345                 350
Gln Glu Gln Cys Pro Ser Thr Asn Pro Leu Phe Thr Thr Ala Leu Gly
        355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400
Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asp Ser

530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 7
<211> 802
<212> PRT
<213> Chlorocebus sabaeus

<400> 7
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His

                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Ala His Ala Ser Ala Gln His Tyr Arg Leu Leu Ala Glu
            260                 265                 270
Ser Gly Asp Ile Gln Ala Glu Met Val Val Arg Leu Gln Ala Lys Thr
        275                 280                 285
Gly Gly Trp Ala Trp Ile Tyr Cys Leu Leu Tyr Ser Glu Gly Pro Glu
    290                 295                 300
Gly Pro Ile Thr Ala Asn Asn Tyr Pro Ile Ser Asp Met Glu Ala Trp
305                 310                 315                 320
Ser Leu Arg Gln Gln Leu Asn Ser Glu Asp Thr Gln Ala Ala Tyr Val
                325                 330                 335
Leu Gly Thr Pro Thr Met Leu Pro Ser Phe Pro Glu Asn Ile Leu Ser
            340                 345                 350
Gln Glu Gln Cys Pro Ser Thr Asn Pro Leu Phe Thr Thr Ala Leu Gly
        355                 360                 365
Ala Pro Arg Ser Thr Ser Phe Pro Ser Ala Pro Glu Leu Ser Val Val
    370                 375                 380
Ser Ala Ser Glu Glu Leu Pro Arg Pro Ser Lys Glu Leu Asp Phe Ser
385                 390                 395                 400
Tyr Leu Thr Phe Pro Ser Gly Pro Glu Pro Ser Leu Gln Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Thr Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Val Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asp Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670

Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Met Phe
    690                 695                 700
Leu Glu Glu Thr Pro Val Glu Asp Ile Phe Met Asp Leu Ser Thr Pro
705                 710                 715                 720
Asp Pro Ser Glu Glu Trp Gly Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 8
<211> 800
<212> PRT
<213> Callithrix jacchus

<400> 8
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ser Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Ala
        195                 200                 205
Ser Leu Phe Leu Ala Met Phe Gln Ser Arg His Ala Lys Asp Leu Ala
    210                 215                 220
Leu Leu Asp Ile Ser Glu Ser Val Leu Ile Tyr Leu Gly Phe Glu Arg
225                 230                 235                 240
Ser Glu Leu Leu Cys Lys Ser Trp Tyr Gly Leu Leu His Pro Glu Asp
                245                 250                 255
Leu Ala His Ala Ser Ala Gln His Tyr Arg Leu Leu Ala Glu Ser Gly
            260                 265                 270
Asp Ile Gln Ala Glu Met Val Val Arg Leu Gln Ala Lys Thr Gly Gly
        275                 280                 285
Trp Ala Trp Ile Tyr Cys Leu Leu Tyr Ser Glu Gly Pro Glu Gly Pro
    290                 295                 300

Ile Thr Ala Asn Asn Tyr Pro Ile Ser Asp Met Glu Ala Trp Ser Leu
305                 310                 315                 320
Arg Gln Gln Leu Asn Ser Glu Asp Asn Gln Ala Ala Tyr Val Leu Gly
                325                 330                 335
Thr Pro Thr Met Leu Pro Ser Phe Pro Glu Asn Ile Leu Ser Gln Glu
            340                 345                 350
Gln Cys Ser Ser Thr Asn Pro Leu Phe Thr Ala Ala Leu Gly Ala Pro
        355                 360                 365
Arg Ser Thr Ser Phe Pro Ser Ala Pro Glu Leu Ser Val Val Ser Ala
    370                 375                 380
Ser Glu Glu Leu Pro Arg Pro Ser Lys Glu Leu Asp Phe Ser Tyr Leu
385                 390                 395                 400
Ala Phe Pro Ser Gly Pro Glu Pro Ser Leu Gln Ala Glu Leu Ser Lys
                405                 410                 415
Asp Leu Val Cys Thr Pro Pro Tyr Thr Pro His Gln Pro Gly Gly Cys
            420                 425                 430
Ala Phe Leu Phe Ser Leu His Glu Pro Phe Gln Thr His Leu Pro Ala
        435                 440                 445
Pro Ser Ser Thr Leu Gln Glu Gln Leu Thr Pro Ser Thr Ala Thr Phe
    450                 455                 460
Ser Asp Gln Leu Thr Pro Ser Ser Ala Thr Phe Pro Asp Pro Leu Thr
465                 470                 475                 480
Ser Pro Leu Gln Gly Gln Leu Thr Glu Thr Ser Ala Arg Ser Tyr Glu
                485                 490                 495
Asp Gln Leu Thr Pro Cys Thr Ser Thr Phe Pro Asp Gln Leu Leu Pro
            500                 505                 510
Ser Thr Ala Thr Phe Pro Glu Pro Val Gly Ser Pro Ala His Glu Gln
        515                 520                 525
Leu Thr Pro Pro Ser Thr Ala Phe Gln Ala His Leu Asp Ser Pro Ser
    530                 535                 540
Gln Thr Phe Pro Glu Gln Leu Ser Pro Asn Pro Thr Lys Thr Tyr Phe
545                 550                 555                 560
Ala Gln Glu Gly Cys Ser Phe Leu Tyr Glu Lys Leu Pro Pro Ser Pro
                565                 570                 575
Ser Ser Pro Gly Asn Gly Asp Cys Thr Leu Leu Ala Leu Ala Gln Leu
            580                 585                 590
Arg Gly Pro Leu Ser Val Asp Val Pro Leu Val Pro Glu Gly Leu Leu
        595                 600                 605
Thr Pro Glu Ala Ser Pro Val Lys Gln Ser Phe Phe His Tyr Ser Glu
    610                 615                 620
Lys Glu Gln Asn Glu Ile Asp Arg Leu Ile Gln Gln Ile Ser Gln Leu
625                 630                 635                 640
Ala Gln Gly Met Asp Arg Pro Phe Ser Ala Glu Ala Gly Thr Gly Gly
                645                 650                 655
Leu Glu Pro Leu Gly Gly Leu Glu Pro Leu Asp Ser Asn Leu Ser Leu
            660                 665                 670
Ser Gly Ala Gly Pro Pro Val Leu Ser Leu Asp Leu Lys Pro Trp Lys
        675                 680                 685
Cys Gln Glu Leu Asp Phe Leu Ala Asp Pro Asp Asn Met Phe Leu Glu
    690                 695                 700
Glu Thr Pro Val Glu Asp Ile Phe Met Asp Leu Ser Thr Pro Asp Pro
705                 710                 715                 720
Asn Glu Glu Trp Gly Ser Gly Asp Pro Glu Ala Glu Gly Pro Gly Gly
                725                 730                 735
Ala Pro Ser Pro Cys Asn Asn Leu Ser Pro Glu Asp His Ser Phe Leu
            740                 745                 750
Glu Asp Leu Ala Thr Tyr Glu Thr Ala Phe Glu Thr Gly Val Ser Ala
        755                 760                 765
Phe Pro Tyr Asp Gly Phe Thr Asp Glu Leu His Gln Leu Gln Ser Gln
    770                 775                 780
Val Gln Asp Ser Phe His Glu Asp Gly Ser Gly Gly Glu Pro Thr Phe
785                 790                 795                 800

<210> 9
<211> 804
<212> PRT
<213> Otolemur garnettii

<400> 9
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Glu Gly
    50                  55                  60
Pro Ser Gly Leu Leu Ser Ala Glu Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Asn Asp
    130                 135                 140
Arg Leu Phe Arg Cys Arg Phe Asn Thr Ser Lys Ser Leu Arg Arg Gln
145                 150                 155                 160
Ser Thr Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Val Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Ala His Ala Ser Ala Gln His Tyr Arg Leu Leu
            260                 265                 270
Ala Glu Ser Gly Asp Ile Gln Ala Glu Met Val Val Arg Leu Gln Ala
        275                 280                 285
Lys Pro Gly Gly Trp Ala Trp Ile Tyr Cys Leu Leu Tyr Ser Glu Gly
    290                 295                 300
Pro Glu Gly Pro Ile Thr Ala Asn Asn Tyr Pro Ile Ser Asp Met Glu
305                 310                 315                 320
Ala Trp Ser Leu Arg Gln Gln Leu Asn Ser Glu Asp Thr Gln Ala Ala
                325                 330                 335
Tyr Val Leu Gly Thr Pro Thr Met Leu Pro Ser Phe Pro Glu Asn Ile
            340                 345                 350
Leu Ala Gln Glu Gln Cys Ser Ser Thr Asn Pro Leu Phe Thr Pro Ala
        355                 360                 365
Leu Gly Ala Pro Arg Ser Thr Thr Phe Pro Ser Ala Pro Glu Leu Ser
    370                 375                 380
Val Val Ser Ala Ser Glu Asp Ile Pro Arg Pro Pro Lys Gly Ile Asp
385                 390                 395                 400
Phe Ser Tyr Leu Thr Phe Pro Ser Gly Pro Glu Pro Ser Leu Gln Gly
                405                 410                 415
Asp Leu Ser Lys Asp Leu Val Cys Thr Pro Pro Tyr Thr Pro His Gln
            420                 425                 430
Pro Gly Gly Cys Ala Phe Leu Phe Ser Leu His Glu Pro Phe Gln Thr

435                 440                 445
His Leu Pro Thr Pro Ser Thr Ser Leu Gln Glu Gln Leu Thr Pro Ser
    450                 455                 460
Thr Ala Thr Phe Ser Asp Gln Leu Thr Pro Ser Ser Ala Thr Phe Pro
465                 470                 475                 480
Asp Pro Leu Thr Ser Pro Leu Gln Gly Leu Leu Thr Glu Thr Ser Ala
                485                 490                 495
Arg Ser Tyr Glu Asp Gln Leu Thr Pro Cys Thr Ser Thr Phe Pro Asp
            500                 505                 510
Gln Leu Leu Pro Ser Thr Ala Thr Phe Pro Glu Pro Leu Gly Ser Pro
        515                 520                 525
Ser Arg Glu Gln Leu Thr Pro Pro Ser Thr Ala Phe Gln Ala His Leu
    530                 535                 540
Asn Ser Pro Ser Gln Thr Phe Pro Glu Gln Leu Ser Pro Asn Pro Thr
545                 550                 555                 560
Lys Thr Tyr Phe Ala Gln Glu Gly Cys Ser Phe Leu Tyr Glu Lys Leu
                565                 570                 575
Pro Pro Ser Pro Ser Ser Pro Gly Asn Gly Asp Cys Thr Leu Leu Ala
            580                 585                 590
Leu Ala Gln Leu Arg Gly Pro Leu Ser Val Asp Val Pro Leu Val Pro
        595                 600                 605
Glu Gly Leu Leu Thr Pro Glu Ala Ser Pro Val Lys Gln Ser Phe Phe
    610                 615                 620
His Tyr Ser Glu Lys Glu Gln Asn Glu Ile Asp Arg Leu Ile Arg Gln
625                 630                 635                 640
Ile Ser Gln Leu Ala Gln Gly Met Asp Arg Pro Phe Ser Ala Glu Ala
                645                 650                 655
Gly Thr Gly Gly Leu Glu Pro Leu Gly Gly Leu Glu Pro Leu Asp Ser
            660                 665                 670
Asn Leu Ser Leu Pro Gly Ala Gly Pro Pro Val Leu Ser Leu Asp Leu
        675                 680                 685
Lys Pro Trp Lys Cys Gln Glu Leu Asp Phe Leu Ala Asp Pro Asp Asn
    690                 695                 700
Met Phe Leu Glu Glu Thr Pro Val Glu Asp Ile Phe Met Asp Leu Ser
705                 710                 715                 720
Thr Pro Asp Pro Ser Glu Glu Trp Gly Ser Gly Asp Pro Glu Ala Glu
                725                 730                 735
Gly Pro Gly Gly Pro Pro Ser Pro Cys Asn Asn Leu Ser Pro Glu Asp
            740                 745                 750
His Ser Phe Leu Glu Asp Leu Ala Thr Tyr Glu Thr Ala Phe Glu Thr
        755                 760                 765
Gly Val Ser Ala Phe Pro Tyr Asp Gly Phe Thr Asp Glu Leu His Gln
    770                 775                 780
Leu Gln Ser Gln Val Gln Asp Ser Phe His Glu Asp Gly Ser Gly Gly
785                 790                 795                 800
Glu Pro Thr Phe


<210> 10
<211> 802
<212> PRT
<213> Mus musculus

<400> 10
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala

65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Met Pro Ser Ala Leu Asp Ala Asp
    130                 135                 140
Arg Leu Phe Arg Cys Arg Phe Asn Thr Ser Lys Ser Leu Arg Arg Gln
145                 150                 155                 160
Ser Ser Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Ala Leu Leu Asp Val Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Ala Gln Ala Ser Ser Gln His Tyr Arg Leu Leu
            260                 265                 270
Ala Glu Ser Gly Asp Ile Gln Ala Glu Met Val Val Arg Leu Gln Ala
        275                 280                 285
Lys His Gly Gly Trp Thr Trp Ile Tyr Cys Met Leu Tyr Ser Glu Gly
    290                 295                 300
Pro Glu Gly Pro Phe Thr Ala Asn Asn Tyr Pro Ile Ser Asp Thr Glu
305                 310                 315                 320
Ala Trp Ser Leu Arg Gln Gln Leu Asn Ser Glu Asp Thr Gln Ala Ala
                325                 330                 335
Tyr Val Leu Gly Thr Pro Ala Val Leu Pro Ser Phe Ser Glu Asn Val
            340                 345                 350
Phe Ser Gln Glu Gln Cys Ser Asn Pro Leu Phe Thr Pro Ser Leu Gly
        355                 360                 365
Thr Pro Arg Ser Ala Ser Phe Pro Arg Ala Pro Glu Leu Gly Val Ile
    370                 375                 380
Ser Thr Pro Glu Glu Leu Pro Gln Pro Ser Lys Glu Leu Asp Phe Ser
385                 390                 395                 400
Tyr Leu Pro Phe Pro Ala Arg Pro Glu Pro Ser Leu Gln Ala Asp Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Pro Pro Ser Ser Ser Leu Gln Glu Gln Leu Thr Pro Ser Thr Val
    450                 455                 460
Thr Phe Ser Glu Gln Leu Thr Pro Ser Ser Ala Thr Phe Pro Asp Pro
465                 470                 475                 480
Leu Thr Ser Ser Leu Gln Gly Gln Leu Thr Glu Ser Ser Ala Arg Ser
                485                 490                 495
Phe Glu Asp Gln Leu Thr Pro Cys Thr Ser Ser Phe Pro Asp Gln Leu
            500                 505                 510
Leu Pro Ser Thr Ala Thr Phe Pro Glu Pro Leu Gly Ser Pro Ala His
        515                 520                 525
Glu Gln Leu Thr Pro Pro Ser Thr Ala Phe Gln Ala His Leu Asn Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575

Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Thr Glu Lys Glu Gln Asn Glu Ile Asp Arg Leu Ile Gln Gln Ile Ser
625                 630                 635                 640
Gln Leu Ala Gln Gly Val Asp Arg Pro Phe Ser Ala Glu Ala Gly Thr
                645                 650                 655
Gly Gly Leu Glu Pro Leu Gly Gly Leu Glu Pro Leu Asn Pro Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Val Asp Pro Asp Asn Leu Phe
    690                 695                 700
Leu Glu Glu Thr Pro Val Glu Asp Ile Phe Met Asp Leu Ser Thr Pro
705                 710                 715                 720
Asp Pro Asn Gly Glu Trp Gly Ser Gly Asp Pro Glu Ala Glu Val Pro
                725                 730                 735
Gly Gly Thr Leu Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Thr Phe Pro Tyr Glu Gly Phe Ala Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe


<210> 11
<211> 802
<212> PRT
<213> Rattus norvegicus

<400> 11
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Met Pro Ser Ala Leu Asp Ala Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205

Pro Gly Pro Ala Ser Leu Phe Leu Ala Met Phe Gln Ser Arg His Ala
    210                 215                 220
Lys Asp Leu Ala Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Ala His Ala Ser Ser Gln His Tyr Arg Leu Leu
            260                 265                 270
Ala Glu Asn Gly Asp Ile Gln Ala Glu Met Val Val Arg Leu Gln Ala
        275                 280                 285
Lys His Gly Gly Trp Thr Trp Ile Tyr Cys Met Leu Tyr Ser Asp Gly
    290                 295                 300
Pro Glu Gly Pro Ile Thr Ala Asn Asn Tyr Pro Ile Ser Asp Thr Glu
305                 310                 315                 320
Ala Trp Ser Leu Arg Gln Gln Leu Asn Ser Glu Asn Thr Gln Ala Ala
                325                 330                 335
Tyr Val Leu Gly Thr Pro Ala Val Leu Pro Ser Phe Ser Glu Asn Val
            340                 345                 350
Phe Ser Gln Glu His Cys Ser Asn Pro Leu Phe Thr Pro Ala Leu Gly
        355                 360                 365
Thr Pro Arg Ser Ala Ser Phe Pro Arg Ala Pro Glu Leu Gly Val Ile
    370                 375                 380
Ser Thr Ser Glu Glu Leu Ala Gln Pro Ser Lys Glu Leu Asp Phe Ser
385                 390                 395                 400
Tyr Leu Pro Phe Pro Ala Arg Pro Glu Pro Ser Leu Gln Ala Asp Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Pro Pro Ser Ser Ser Leu Gln Glu Gln Leu Thr Pro Ser Thr Val
    450                 455                 460
Thr Phe Ser Glu Gln Leu Thr Pro Ser Ser Ala Thr Phe Pro Asp Pro
465                 470                 475                 480
Leu Thr Ser Ser Leu Gln Gly Gln Leu Thr Glu Ser Ser Ala Arg Ser
                485                 490                 495
Phe Glu Glu Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Thr His
        515                 520                 525
Glu Gln Leu Thr Pro Pro Ser Thr Ala Phe Gln Ala His Leu Asn Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Thr Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asn Pro Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Val Asp Pro Asp Asn Leu Phe
    690                 695                 700
Leu Glu Glu Thr Pro Val Glu Asp Ile Phe Met Asp Leu Ser Thr Pro

705                 710                 715                 720
Asp Pro Asn Gly Glu Trp Gly Ser Gly Asp Pro Glu Ala Glu Val Pro
                725                 730                 735
Gly Gly Thr Leu Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Thr Phe Pro Tyr Glu Gly Phe Ala Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 12
<211> 802
<212> PRT
<213> Cavia porcellus

<400> 12
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Thr Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Ala Leu Leu Asp Val Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Ala His Ala Ser Ala Gln His Tyr Arg Leu Leu
            260                 265                 270
Ala Glu Ser Gly Asp Ile Gln Ala Glu Met Val Val Arg Leu Gln Ser
        275                 280                 285
Lys His Gly Gly Trp Ala Trp Ile Tyr Cys Leu Leu Tyr Ser Glu Gly
    290                 295                 300
Pro Glu Gly Pro Ile Thr Thr Asn Asn Tyr Pro Ile Ser Asp Met Glu
305                 310                 315                 320
Ala Trp Ser Leu Arg Gln Gln Leu Asn Ser Glu Asp Thr Gln Ala Ala
                325                 330                 335
Tyr Val Leu Gly Asn Pro Thr Val Leu Pro Ser Phe Pro Glu Asn Val

            340                 345                 350
Leu Ser Gln Glu Gln Cys Ser Asn Pro Leu Phe Thr Pro Pro Leu Gly
        355                 360                 365
Ala Pro Arg Ser Thr Ser Phe Pro Ser Ala Pro Gln Leu Gly Val Val
    370                 375                 380
Ser Thr Ser Glu Glu Leu Pro Arg Ser Leu Gln Lys Leu Asp Phe Ser
385                 390                 395                 400
Tyr Leu Pro Phe Pro Ser Gly Pro Glu Pro Ser Leu Gln Ala Asp Leu
                405                 410                 415
Ser Lys Asp Phe Val Cys Thr Pro Pro Tyr Thr Pro His Lys Pro Gly
            420                 425                 430
Gly Cys Ala Phe Leu Phe Ser Leu His Glu Pro Phe Gln Thr His Leu
        435                 440                 445
Pro Pro Pro Ser Ser Ser Phe Gln Glu Gln Leu Thr Pro Ser Thr Ser
    450                 455                 460
Thr Phe Ser Asp Gln Leu Thr Pro Ser Ser Ala Thr Phe Pro Asp Pro
465                 470                 475                 480
Leu Ile Ser Pro Leu Gln Gly Gln Leu Thr Glu Ile Ser Thr Arg Asn
                485                 490                 495
Tyr Glu Asp Gln Pro Thr Pro Cys Thr Ser Thr Phe Ser Asp Gln Leu
            500                 505                 510
Leu Pro Ser Thr Ala Thr Phe Pro Glu Pro Leu Gly Ser Pro Thr His
        515                 520                 525
Glu Arg Leu Thr Pro Pro Thr Ala Ala Phe Gln Ala Pro Leu Asn Ser
    530                 535                 540
Pro Ser Gln Thr Phe Pro Glu Gln Leu Ser Pro Asn Ser Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln His Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Ser Ile Phe
    690                 695                 700
Leu Glu Glu Met Pro Val Glu Asp Ile Phe Met Asp Leu Ser Thr Pro
705                 710                 715                 720
Asp Pro Asn Gly Glu Trp Asp Ser Gly Asp Ser Glu Ala Glu Gly Pro
                725                 730                 735
Arg Arg Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Ser Val
        755                 760                 765
Ser Thr Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 13
<211> 792
<212> PRT

<213> Ochotona princeps

<400> 13
Met Tyr Arg Ser Thr Lys Gly Ala Ser Lys Ala Arg Arg Asp Gln Ile
1               5                   10                  15
Asn Ala Glu Ile Arg Ser Leu Lys Glu Leu Leu Pro Leu Ala Glu Ala
            20                  25                  30
Asp Lys Val Arg Leu Ser Tyr Leu His Ile Met Ser Leu Cys Ile Tyr
        35                  40                  45
Thr Arg Lys Gly Val Phe Phe Ala Gly Gly Thr Ala Leu Glu Gly Pro
    50                  55                  60
Thr Gly Leu Leu Ser Ala Gln Glu Leu Glu Asp Ile Val Ala Ala Leu
65                  70                  75                  80
Pro Gly Phe Leu Leu Val Phe Thr Ala Glu Gly Lys Leu Leu Tyr Leu
                85                  90                  95
Ser Glu Ser Val Ser Glu His Leu Gly His Ser Met Val Asp Leu Val
            100                 105                 110
Ala Gln Gly Asp Ser Ile Tyr Asp Ile Ile Asp Pro Ala Asp His Leu
        115                 120                 125
Thr Val Arg Gln Gln Leu Ala Met Pro Ser Ala Leu Asn Thr Asp Arg
    130                 135                 140
Leu Phe Arg Cys Arg Phe Asn Thr Ser Lys Ser Leu Arg Arg Gln Ser
145                 150                 155                 160
Ala Gly Asn Lys Leu Val Leu Ile Arg Gly Arg Phe His Ala His Pro
                165                 170                 175
Pro Gly Ala Tyr Trp Ala Gly Asn Pro Val Phe Thr Ala Phe Cys Ala
            180                 185                 190
Pro Leu Glu Pro Arg Pro Arg Pro Gly Pro Gly Pro Gly Pro Gly Pro
        195                 200                 205
Ala Ser Leu Phe Leu Ala Met Phe Gln Ser Arg His Ala Lys Asp Leu
    210                 215                 220
Ala Ile Leu Asp Ile Ser Glu Ser Val Leu Ile Tyr Leu Gly Phe Glu
225                 230                 235                 240
His Thr Glu Leu Leu Cys Lys Ser Trp Tyr Gly Leu Leu His Pro Glu
                245                 250                 255
Asp Leu Thr His Ala Ser Ala Gln His Tyr Arg Leu Leu Thr Glu Ser
            260                 265                 270
Arg Asp Ile Gln Ala Glu Met Val Val Arg Leu Gln Ala Lys Ser Gly
        275                 280                 285
Gly Trp Ala Trp Ile Tyr Cys Leu Leu Tyr Ser Glu Gly Pro Glu Gly
    290                 295                 300
Pro Ile Thr Ala Asn Asn Tyr Pro Ile Ser Asp Pro Glu Ala Trp Ser
305                 310                 315                 320
Leu Arg Gln Gln Leu Ser Ser Glu Asp Thr Leu Ala Ser Tyr Val Leu
                325                 330                 335
Ser Ala Pro Ala Met Arg Thr Ser Phe Pro Glu Asn Ile Leu Ser His
            340                 345                 350
Glu Gln Cys Ser Asn Pro Asn Pro Leu Phe Ala Thr Ala Leu Gly Ala
        355                 360                 365
Pro Arg Ser Thr Ser Phe Pro Ser Ala Ala Glu Leu Gly Val Ala Ser
    370                 375                 380
Gly Ser Lys Glu Leu Pro Gln Pro Pro Lys Glu Val Asp Phe Arg Tyr
385                 390                 395                 400
Leu Thr Phe Ser Ser Gly Pro Ala Leu Pro Leu Gln Ala Asp Met Ser
                405                 410                 415
Lys Asp Leu Val Cys Thr Pro Leu Tyr Thr Pro His Gln Pro Gly Gly
            420                 425                 430
Cys Thr Phe Leu Phe Ser Leu His Glu Pro Phe Gln Thr Arg Cys Pro
        435                 440                 445
Pro Pro Ser Ser Ser Gln Glu Gln Leu Thr Ser Ser Thr Val Thr Phe
    450                 455                 460
Phe Glu Gln Leu Thr Pro Ser Ser Ala Phe Asp Pro Leu His Ser Pro
465                 470                 475                 480

Leu Gln Gln Leu Thr Glu Ala Gln Arg Ser Tyr Glu Gln Leu Thr Pro
                485                 490                 495
Cys Thr Thr Phe Asp Leu Leu Pro Ser Thr Ala Thr Phe Pro Glu Pro
            500                 505                 510
Leu Gly Ser Pro Gly His Glu Gln Leu Thr Pro Pro Ser Thr Ala Phe
        515                 520                 525
Gln Ala Gln Leu Ser Ser Pro Ser Gln Thr Phe Pro Glu Gln Leu Ser
    530                 535                 540
Pro Ser Ala Thr Lys Thr Tyr Phe Thr Gln Glu Gly Cys Ser Phe Leu
545                 550                 555                 560
Tyr Glu Lys Leu Pro Pro Ser Pro Ser Ser Pro Gly Asn Gly Asp Cys
                565                 570                 575
Thr Leu Leu Ala Leu Ala Gln Leu Arg Gly Pro Leu Ser Val Asp Val
            580                 585                 590
Pro Leu Val Pro Glu Gly Leu Leu Thr Pro Glu Ala Ser Pro Val Lys
        595                 600                 605
Gln Ser Phe Phe His Tyr Ser Glu Lys Gln Gln Asn Gly Ile Asp Arg
    610                 615                 620
Leu Ile Gln Gln Ile Ser Gln Leu Ala Gln Gly Met Asp Arg Pro Phe
625                 630                 635                 640
Ser Ala Glu Ala Gly Thr Gly Gly Leu Glu Pro Leu Gly Gly Leu Glu
                645                 650                 655
Pro Leu Asp Ser Asn Leu Ser Leu Pro Gly Ala Gly Pro Pro Val Leu
            660                 665                 670
Ser Leu Asp Leu Lys Pro Trp Lys Cys Gln Glu Leu Asp Phe Leu Val
        675                 680                 685
Asp Pro Glu Asn Ile Phe Leu Asp Glu Thr Pro Val Glu Asp Ile Phe
    690                 695                 700
Met Asp Leu Ser Thr Pro Asp Pro Asn Gly Glu Trp Ser Ser Gly Asp
705                 710                 715                 720
Pro Glu Ala Glu Gly Pro Gly Gly Ala Pro Ser Pro His Asn Asn Leu
                725                 730                 735
Ser Pro Glu Asp His Ser Phe Leu Glu Asp Leu Ala Thr Tyr Glu Thr
            740                 745                 750
Ala Phe Glu Thr Gly Val Ser Ala Phe Pro Tyr Asp Gly Phe Thr Asp
        755                 760                 765
Glu Leu His Gln Leu Gln Ser Gln Val Gln Asp Ser Phe His Glu Asp
    770                 775                 780
Gly Ser Gly Gly Glu Pro Thr Phe
785                 790

<210> 14
<211> 802
<212> PRT
<213> Ictidomys tridecemlineatus

<400> 14
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125

Leu Thr Val Arg Gln Gln Leu Thr Leu Pro Ser Ala Leu Asp Thr Asp
    130                 135                 140
Arg Leu Phe Arg Cys His Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Ala Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Ala His Ala Ser Ala Gln His Tyr Arg Leu Leu
            260                 265                 270
Ala Glu Ser Gly Asp Ile Gln Ala Glu Met Val Val Arg Leu Gln Ala
        275                 280                 285
Lys Asn Gly Gly Trp Ala Trp Ile Tyr Cys Leu Leu Tyr Ser Glu Gly
    290                 295                 300
Pro Glu Gly Pro Ile Thr Ala Asn Asn Tyr Pro Ile Ser Asp Ser Glu
305                 310                 315                 320
Ala Trp Ser Leu Arg Gln Gln Leu Asn Ser Glu Asp Thr Gln Ala Ala
                325                 330                 335
Tyr Val Leu Gly Thr Pro Thr Met Leu Pro Ser Phe Pro Glu Asn Ile
            340                 345                 350
Leu Ser Gln Glu Gln Cys Ser Asn Pro Leu Phe Thr Pro Pro Leu Gly
        355                 360                 365
Ala Pro Arg Ser Thr Gly Phe Pro Asn Thr Pro Gly Leu Gly Val Val
    370                 375                 380
Ser Ala Ser Glu Glu Leu Pro Arg Pro Pro Lys Glu Leu Asp Phe Ser
385                 390                 395                 400
Tyr Leu Pro Phe Pro Ser Gly Pro Glu Pro Ser Leu His Ala Glu Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Ala Pro Ser Ser Ser Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Pro Gly Gln Leu Thr Glu Thr Ser Ala Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Ser Ser Pro Ser His
        515                 520                 525
Glu Gln Leu Thr Pro Pro Ser Thr Ala Phe Gln Ala Pro Leu Asn Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser

625                 630                 635                 640
Gln Leu Ala Gln Gly Met Asp Arg Pro Phe Ser Ala Glu Ala Gly Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Asn Ile Phe
    690                 695                 700
Leu Glu Glu Thr Pro Val Glu Asp Ile Phe Met Asp Leu Ser Thr Pro
705                 710                 715                 720
Asp Pro Asn Gly Asp Trp Asp Ser Gly Asp Pro Glu Ala Glu Gly Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Ala Gln Asp Ser Phe His Glu Asp Gly Ser Gly Gly Glu Pro
785                 790                 795                 800
Thr Phe


<210> 15
<211> 798
<212> PRT
<213> Pteropus vampyrus

<400> 15
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Arg Asn Lys Leu Val Leu Ile Arg Gly Arg Phe His Ala His
                165                 170                 175
Pro Pro Gly Ala Tyr Trp Ala Gly Ile Pro Asp Phe Thr Ala Phe Cys
            180                 185                 190
Ala Pro Leu Pro Arg Pro Arg Pro Gly Pro Gly Pro Gly Leu Ala Ser
        195                 200                 205
Leu Phe Leu Val Met Gln Ser Arg His Ala Lys Asp Leu Val Leu Leu
    210                 215                 220
Asp Ile Ser Glu Ser Val Leu Ile Tyr Leu Gly Phe Glu Arg Ser Glu
225                 230                 235                 240
Leu Leu Cys Lys Ser Trp Tyr Gly Leu Leu His Pro Glu Asp Leu Gly
                245                 250                 255
His Ala Ser Ala Gln His Tyr Arg Leu Leu Thr Glu Ser Gly Asp Ile

            260                 265                 270
Gln Ala Glu Met Val Val Arg Leu Gln Ala Lys Pro Gly Gly Trp Ala
        275                 280                 285
Trp Ile Tyr Cys Leu Leu Tyr Ser Glu Gly Pro Glu Gly Pro Ile Thr
    290                 295                 300
Ala Asn Asn Tyr Pro Ile Ser Asp Thr Glu Ala Trp Ser Leu Arg Gln
305                 310                 315                 320
Gln Leu Asn Ser Glu Asp Thr Gln Ala Ala Tyr Val Leu Gly Thr Pro
                325                 330                 335
Thr Met Leu Pro Ser Phe Pro Glu Asn Ile Leu Ser Gln Glu Gln Cys
            340                 345                 350
Ser Ser Thr His Pro Leu Phe Thr Pro Ala Leu Gly Ala Pro Arg Ser
        355                 360                 365
Thr Asn Phe Pro Ser Ala Pro Gln Leu Gly Ala Val Ser Thr Ser Glu
    370                 375                 380
Glu Leu Pro Arg Pro Pro Lys Glu Leu Gly Phe Ser Tyr Leu Thr Phe
385                 390                 395                 400
Pro Ser Gly Pro Glu Pro Ser Leu Gln Ala Asp Leu Ser Lys Asp Ile
                405                 410                 415
Val Cys Thr Pro Pro Tyr Thr Pro His Gln Pro Gly Gly Cys Ala Phe
            420                 425                 430
Leu Phe Ser Leu His Glu Pro Phe Gln Thr Arg Leu Pro Thr Pro Ser
        435                 440                 445
Ser Ser Leu Gln Glu Gln Leu Thr Pro Ser Thr Met Thr Phe Ser Glu
    450                 455                 460
Gln Leu Thr Pro Ser Ser Ala Thr Phe Pro Asp Pro Leu Thr Ser Ser
465                 470                 475                 480
Leu Gln Ser Gln Leu Thr Glu Thr Ser Ala Arg Ser Tyr Glu Asp Gln
                485                 490                 495
Leu Thr Pro Cys Thr Ser Asn Phe Pro Glu Gln Leu Leu Pro Ser Thr
            500                 505                 510
Ala Thr Phe Pro Glu Pro Leu Gly Ser Pro Ala His Glu Gln Leu Thr
        515                 520                 525
Pro Pro Gly Thr Ala Phe Gln Ala His Leu Asn Ser Pro Gly Gln Thr
    530                 535                 540
Phe Pro Glu Gln Leu Ser Pro Asn Pro Thr Lys Thr Tyr Phe Ala Gln
545                 550                 555                 560
Glu Gly Cys Ser Phe Leu Tyr Glu Lys Leu Pro Pro Ser Pro Ser Ser
                565                 570                 575
Pro Gly Asn Gly Asp Cys Thr Leu Leu Ala Leu Ala Gln Leu Arg Gly
            580                 585                 590
Pro Leu Ser Val Asp Val Pro Leu Val Pro Glu Gly Leu Leu Thr Pro
        595                 600                 605
Glu Ala Ser Pro Val Lys Gln Ser Phe Phe His Tyr Ser Glu Lys Glu
    610                 615                 620
Gln Asn Glu Ile Asp Arg Leu Ile Gln Gln Ile Ser Gln Leu Ala His
625                 630                 635                 640
Gly Met Asp Arg Pro Phe Ser Ala Glu Ala Gly Thr Gly Gly Leu Glu
                645                 650                 655
Pro Leu Gly Gly Leu Glu Pro Leu Asp Ser Asn Leu Ser Leu Ser Gly
            660                 665                 670
Ala Gly Pro Pro Val Leu Ser Leu Asp Leu Lys Pro Trp Lys Cys Gln
        675                 680                 685
Glu Leu Asp Phe Leu Val Asp Pro Asp Asn Ile Phe Leu Glu Glu Thr
    690                 695                 700
Pro Val Glu Asp Ile Phe Met Asp Leu Ser Thr Pro Asp Pro Asn Gly
705                 710                 715                 720
Glu Trp Ser Leu Glu Asp Pro Glu Ala Ala Val Pro Gly Gly Ala Pro
                725                 730                 735
Ser Pro Cys Asn Asn Leu Ser Pro Glu Asp His Ser Phe Leu Glu Asp
            740                 745                 750
Leu Ala Thr Tyr Glu Thr Ala Phe Glu Thr Gly Val Ser Ala Phe Pro
        755                 760                 765

Tyr Asp Gly Phe Thr Asp Glu Leu His Gln Leu Gln Ser Gln Val Gln
    770                 775                 780
Asp Ser Phe His Glu Asp Gly Ser Gly Gly Glu Pro Thr Phe
785                 790                 795

<210> 16
<211> 802
<212> PRT
<213> Sus scrofa

<400> 16
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Ser Thr Gly Leu Leu Ser Ala Gln Glu Leu Glu Glu Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Ser Leu Pro Ser Ser Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Thr Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Ala Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Gly His Ala Ser Ala Gln His Tyr Arg Leu Leu
            260                 265                 270
Ala Glu Ser Gly Asp Ile Gln Ala Glu Met Val Val Arg Leu Leu Ala
        275                 280                 285
Lys Pro Gly Gly Trp Ala Trp Ile Tyr Cys Leu Leu Tyr Ser Glu Gly
    290                 295                 300
Pro Glu Gly Pro Ile Thr Ala Asn Asn Tyr Pro Ile Ser Asp Ser Glu
305                 310                 315                 320
Ala Trp Ser Leu Arg Gln Gln Leu Asn Ser Glu Asp Thr Gln Ala Ala
                325                 330                 335
Tyr Val Leu Gly Thr Pro Thr Met Leu Pro Ser Phe Pro Glu Asn Ile
            340                 345                 350
Leu Ser Gln Glu Gln Cys Ser Ser Pro Asn Pro Leu Phe Thr Met Gly
        355                 360                 365
Pro Pro Arg Ser Thr Asp Phe Pro Arg Ala Pro Glu Pro Ser Ala Val
    370                 375                 380
Ser Thr Ser Glu Glu Leu Leu Arg Pro Thr Lys Asp Leu Gly Phe Gly
385                 390                 395                 400
Tyr Leu Thr Phe Pro Ser Gly Pro Glu Pro Ser Leu Gln Ala Asp Leu
                405                 410                 415

Ser Lys Asp Phe Val Cys Thr Pro Pro Tyr Thr Pro His Gln Pro Gly
            420                 425                 430
Gly Cys Ala Phe Leu Phe Ser Leu His Glu Pro Phe Gln Pro His Leu
        435                 440                 445
Pro Ile Pro Pro Ser Ser Leu Gln Glu Gln Leu Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Ala Arg Ser
                485                 490                 495
Tyr Glu Asp Gln Leu Thr Pro Cys Thr Ser Thr Phe Pro Asp Gln Leu
            500                 505                 510
Leu Pro Ser Thr Ala Thr Phe Pro Glu Pro Leu Gly Ser Pro Ala Arg
        515                 520                 525
Glu Gln Ser Thr Pro Pro Ser Thr Ala Phe Gln Ala His Leu Asn Ser
    530                 535                 540
Pro Ser Gln Thr Phe Pro Glu Gln Leu Ser Pro Asn Pro Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Val Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Gly Thr
                645                 650                 655
Gly Gly Leu Glu Ser Leu Gly Gly Leu Glu Pro Leu Asp Ser Asn Leu
            660                 665                 670
Ser Leu Ser Gly Ala Gly Pro Pro Val Leu Ser Leu Glu Leu Lys Pro
        675                 680                 685
Trp Lys Cys Gln Asp Leu Asp Phe Leu Ala Asp Pro Asp Asn Ile Phe
    690                 695                 700
Leu Glu Glu Thr Ser Val Glu Asp Ile Phe Met Asp Leu Ser Thr Pro
705                 710                 715                 720
Asp Pro Asn Gly Glu Trp Ser Ser Gly Asp Pro Glu Ala Ala Val Pro
                725                 730                 735
Gly Gly Ala Pro Ser Pro Cys Asn Asn Leu Ser Pro Glu Asp Arg Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Thr Asp Glu Leu His Gln Leu Gln
    770                 775                 780
Ser Gln Val Gln Asp Ser Phe His Glu Asp Gly Ser Gly Gly Glu Pro
785                 790                 795                 800
Thr Phe

<210> 17
<211> 804
<212> PRT
<213> Dasypus novemcinctus

<400> 17
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45

Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Pro His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Gly Pro Ala Ser Leu Phe Leu Ala Met Phe Gln Ser His His Ala
    210                 215                 220
Lys Asp Leu Ala Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Ala His Ala Ser Ala Gln His Tyr Arg Leu Leu
            260                 265                 270
Ala Glu Ser Gly Asp Ile Gln Ala Glu Met Val Val Arg Leu Gln Ala
        275                 280                 285
Lys Pro Gly Gly Trp Ala Trp Ile Tyr Cys Leu Leu Tyr Ser Glu Gly
    290                 295                 300
Pro Glu Gly Pro Ile Thr Ala Asn Asn Tyr Pro Ile Ser Asp Ser Glu
305                 310                 315                 320
Ala Trp Ser Leu Arg Gln Gln Leu Asn Ser Glu Asp Thr Gln Ala Ala
                325                 330                 335
Tyr Val Leu Gly Thr Ser Thr Met Leu Pro Ser Phe Ser Glu Asn Ile
            340                 345                 350
Leu Ser Gln Glu Gln Cys Ser Ser Ser Asn Pro Leu Phe Thr Pro Ala
        355                 360                 365
Leu Ala Thr Ala Arg Ser Thr Ser Phe Pro Ser Ala Thr Glu Leu Gly
    370                 375                 380
Val Ala Ser Thr Ser Glu Glu Leu Pro Gln Pro Leu Lys Glu Leu Asp
385                 390                 395                 400
Phe Arg Tyr Leu Pro Phe Pro Ala Gly Pro Glu Pro Ser Leu Gln Ala
                405                 410                 415
Asp Met Ser Lys Asp Leu Val Cys Thr Pro Pro Tyr Thr Pro His Gln
            420                 425                 430
Pro Gly Gly Cys Ala Phe Leu Phe Ser Leu His Glu Pro Phe Gln Thr
        435                 440                 445
His Leu Ala Thr Pro Ser Ser Ser Leu Gln Glu Gln Leu Thr Pro Ser
    450                 455                 460
Thr Thr Thr Phe Ser Asp Gln Leu Thr Pro Ser Ser Ala Thr Phe Pro
465                 470                 475                 480
Asp Pro Leu Thr Ser Pro Leu Gln Ser Gln Leu Thr Glu Ala Ser Ala
                485                 490                 495
Arg Ile Tyr Glu Asp Gln Leu Thr Pro Cys Thr Ser Thr Phe Pro Glu
            500                 505                 510
Gln Leu Leu Pro Ser Thr Ala Thr Phe Pro Glu Ser Leu Ser Ser Pro
        515                 520                 525
Ser His Glu Gln Leu Thr Pro Pro Thr Thr Ala Phe Gln Ala His Leu
    530                 535                 540
Ser Ser Pro Ser Gln Thr Phe Pro Glu Gln Leu Ser Pro Asn Pro Thr

545                 550                 555                 560
Lys Thr Tyr Phe Ala Gln Glu Gly Cys Ser Phe Leu Tyr Glu Lys Leu
                565                 570                 575
Pro Pro Ser Pro Ser Ser Pro Gly Asn Gly Asp Cys Thr Leu Leu Ala
            580                 585                 590
Leu Ala Gln Leu Arg Gly Pro Leu Ser Val Asp Val Pro Leu Val Pro
        595                 600                 605
Asp Gly Leu Leu Thr Pro Glu Ala Ser Pro Val Lys Gln Ser Phe Phe
    610                 615                 620
His Tyr Ser Glu Lys Glu Gln Asn Glu Ile Asp Arg Leu Ile Gln Gln
625                 630                 635                 640
Ile Ser Gln Leu Ala Gln Gly Met Asp Arg Pro Phe Ser Ala Glu Ala
                645                 650                 655
Ala Thr Gly Gly Leu Glu Pro Leu Gly Gly Leu Glu Pro Leu Asp Ser
            660                 665                 670
Asn Leu Ser Leu Ser Gly Ala Gly Pro Pro Val Leu Ser Leu Asp Leu
        675                 680                 685
Lys Pro Trp Lys Cys Gln Glu Leu Asp Phe Leu Ala Asp Pro Asp Asn
    690                 695                 700
Ile Phe Leu Glu Glu Met Pro Val Glu Asp Ile Phe Met Asp Leu Ser
705                 710                 715                 720
Thr Pro Ala Pro Asn Gly Glu Trp Gly Ser Glu Asp Pro Glu Ala Val
                725                 730                 735
Gly Pro Gly Glu Ala Pro Ser Pro Cys Asn Asn Leu Ser Pro Glu Asp
            740                 745                 750
His Ser Phe Leu Glu Asp Leu Ala Thr Tyr Glu Thr Ala Phe Glu Thr
        755                 760                 765
Gly Val Ser Ala Phe Pro Tyr Asp Gly Phe Thr Asp Glu Leu His Gln
    770                 775                 780
Leu Gln Ser Gln Val Gln Asp Ser Phe His Glu Asp Gly Ser Gly Gly
785                 790                 795                 800
Glu Pro Thr Phe

<210> 18
<211> 804
<212> PRT
<213> Monodelphis domestica

<400> 18
Met Tyr Arg Ser Thr Lys Gly Ala Ser Lys Ala Arg Arg Asp Gln Ile
1               5                   10                  15
Asn Ala Glu Ile Arg Ser Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Ala Gly Ala Pro Leu Thr
    50                  55                  60
Gly Pro Ala Gly Leu Leu Ser Ala Gln Glu Leu Glu Asp Ile Val Ala
65                  70                  75                  80
Ala Leu Pro Gly Phe Leu Leu Met Phe Thr Ala Glu Gly Lys Leu Leu
                85                  90                  95
Tyr Leu Ser Asp Ser Val Ser Glu His Leu Gly His Ser Met Val Asp
            100                 105                 110
Leu Val Ala Gln Gly Asp Ser Ile Tyr Asp Ile Ile Asp Pro Ala Asp
        115                 120                 125
His Leu Thr Val Arg Gln Gln Leu Ala Leu Pro Ser Val Pro Asp Thr
    130                 135                 140
Asp Arg Leu Phe Arg Cys Arg Phe Asn Thr Ser Lys Ser Leu Arg Arg
145                 150                 155                 160
Gln Ser Ala Gly Asn Lys Leu Val Leu Ile Arg Gly Arg Phe His Pro
                165                 170                 175
His Pro Pro Gly Ala Tyr Trp Ala Gly Asn Pro Val Phe Thr Ala Phe

180                 185                 190
Cys Ala Pro Leu Asp Ala Arg Ala Arg Pro Gly Pro Ala Ser Gly Pro
        195                 200                 205
Gly Val Ala Ser Ile Phe Leu Ala Val Phe Gln Ser Arg His Ala Lys
    210                 215                 220
Asp Leu Ala Leu Leu Asp Val Ser Glu Ser Val Leu Val Tyr Leu Gly
225                 230                 235                 240
Phe Glu Arg Ser Glu Leu Val Cys Lys Ser Trp Tyr Gly Leu Leu His
                245                 250                 255
Pro Glu Asp Leu Ala His Ala Ser Ala Gln His Tyr Arg Leu Leu Ala
            260                 265                 270
Asp Ser Gly Asp Ala Gln Ala Glu Met Val Val Arg Leu Gln Ala Lys
        275                 280                 285
Ala Gly Gly Trp Val Trp Val Tyr Ser Leu Met His Pro Glu Gly Ser
    290                 295                 300
Asp Gly Pro Ile Thr Ala Asn Asn Tyr Pro Ile Ser Asp Ser Glu Ala
305                 310                 315                 320
Trp Cys Leu Arg Gln Gln Leu Ser Ser Glu Asp Ala His Leu Ala Tyr
                325                 330                 335
Val Leu Gly Thr Ser Thr Ala Pro Ser Thr Phe Ser Glu Ser Leu Leu
            340                 345                 350
Ser Pro Ala Gln Cys Thr Ser Pro Asp Pro Thr Phe Thr Ser Ala Val
        355                 360                 365
Gly Gly Pro Arg Ser Thr Asn Phe Pro Thr Gly Pro Glu Leu Ala Ser
    370                 375                 380
Ile Ser Ser Ala Gln Gln Leu Pro Gln Pro Pro Lys Glu Leu Asp Phe
385                 390                 395                 400
Asn Tyr Leu Ala Phe Pro Thr Gly Pro Glu Ser Thr Phe Gln Gly Asp
                405                 410                 415
Leu Cys Lys Asp Phe Val Cys Thr Pro Pro Tyr Thr Pro His Gln Pro
            420                 425                 430
Gly Gly Cys Ala Phe Leu Phe Ser Leu Gln Glu Pro Phe Gln Thr His
        435                 440                 445
Ile Ser Thr Ser Ser Gly Ser Ile His Glu Gln Leu Thr Pro Ser Thr
    450                 455                 460
Ala Ile Phe Pro Asp Gln Leu Thr Pro Ser Ser Ala Thr Phe Pro Glu
465                 470                 475                 480
Leu Thr Ser Pro Ile Gln Gly Gln Leu Thr Asp Thr Thr Ala Gln Pro
                485                 490                 495
Tyr Glu Asp Gln Leu Thr Pro Cys Ala Thr Thr Phe Gln Asp Gln Leu
            500                 505                 510
Met Ser Asn Thr Ala Thr Phe Gln Glu Gln Leu Ala Ser Pro Val Gln
        515                 520                 525
Glu Gln Leu Thr Pro Pro Ser Thr Ala Phe Gln Ala Gln Leu Ala Ser
    530                 535                 540
Pro Ser Arg Thr Phe Pro Asp Gln Leu Ser Pro Asn Pro Thr Lys Pro
545                 550                 555                 560
Tyr Phe Pro Gln Asp Gly Cys Ser Phe Leu Tyr Glu Lys Leu Pro Pro
                565                 570                 575
Ser Pro Asn Ser Pro Gly Asn Gly Asp Cys Thr Leu Leu Ala Leu Ala
            580                 585                 590
Gln Leu Arg Gly Pro Leu Ser Val Asp Val Pro Leu Val Pro Asp Gly
        595                 600                 605
Leu Leu Thr Pro Glu Ala Ser Pro Val Lys Gln Ser Phe Phe His Tyr
    610                 615                 620
Ser Glu Lys Glu Lys Ser Glu Ile Asp Arg Leu Ile Gln Gln Ile Ser
625                 630                 635                 640
Gln Leu Ala Gln Gly Met Asp Cys Pro Phe Pro Ala Glu Pro Gly Ala
                645                 650                 655
Glu Gly Leu Glu Pro Leu Gly Gln Ser Leu Ser Phe Pro Gly Ala Gly
            660                 665                 670
Pro Pro Ile Phe Ser Leu Asp Leu Lys Ser Trp Lys Ser Gln Glu Leu
        675                 680                 685

Ala Phe Leu Asp Ala Pro Glu Asp His Phe Leu Glu Glu Lys Ser Val
    690                 695                 700
Glu Asp Ile Phe Met Gly Leu Ser Thr Pro Thr Pro Gln Gly Glu Trp
705                 710                 715                 720
Gly Pro Gly Gly Pro Glu Thr Ser Gly Pro Gly Gly Ala Pro Ser Pro
                725                 730                 735
Cys Thr Asp Leu Ser Pro Glu Asp His Ser Phe Leu Glu Asp Leu Ala
            740                 745                 750
Ala Tyr Glu Thr Asp Phe Glu Thr Gly Val Ser Ala Phe Pro Tyr Asp
        755                 760                 765
Gly Ser Ser Asp Glu Leu Tyr Gln Leu Gln Ser His Val Gln Asp Ser
    770                 775                 780
Phe His Asp Gly Glu His Asp Ile Asn Asn Gly Ser Pro Ser Ile Gly
785                 790                 795                 800
Leu Arg Ser Thr


<210> 19
<211> 803
<212> PRT
<213> Monodelphis domestica

<220>
<223> Felis catus

<400> 19
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ser Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Val
    130                 135                 140
Tyr Leu Asp Arg Leu Phe Arg Cys Arg Phe Asn Thr Ser Lys Ser Leu
145                 150                 155                 160
Arg Arg Gln Ser Ala Gly Asn Lys Leu Val Leu Ile Arg Gly Arg Phe
                165                 170                 175
His Ala His Pro Pro Gly Ala Tyr Trp Ala Gly Asn Pro Val Phe Thr
            180                 185                 190
Ala Phe Cys Ala Pro Leu Glu Pro Arg Pro Arg Pro Gly Pro Gly Pro
        195                 200                 205
Gly Pro Ala Ser Leu Phe Leu Ala Met Phe Gln Ser Arg His Ala Lys
    210                 215                 220
Asp Leu Ala Leu Leu Asp Ile Ser Glu Ser Val Leu Ile Tyr Leu Gly
225                 230                 235                 240
Phe Glu Arg Ser Glu Leu Leu Cys Lys Ser Trp Tyr Gly Leu Leu His
                245                 250                 255
Pro Glu Asp Leu Ala His Ala Ser Ala Gln His Tyr Arg Leu Leu Ala
            260                 265                 270
Glu Ser Gly Asp Ile Gln Ala Glu Met Val Val Arg Leu Gln Ala Lys
        275                 280                 285
Pro Gly Gly Trp Ala Trp Ile Tyr Cys Leu Leu Tyr Ser Glu Gly Pro

        290                 295                 300
Glu Gly Pro Ile Thr Ala Asn Asn Tyr Pro Ile Ser Asp Thr Glu Ala
305                 310                 315                 320
Trp Ser Leu Arg Gln Gln Leu Asn Ser Glu Asn Thr Gln Ala Ala Tyr
                325                 330                 335
Val Leu Gly Thr Pro Ala Met Leu Pro Ser Phe Pro Glu Asn Ile Leu
            340                 345                 350
Ser Gln Glu Gln Cys Ser Ser Pro Asn Pro Leu Phe Pro Pro Ala Leu
        355                 360                 365
Gly Ala Pro Arg Ser Thr Ser Phe Pro Ser Ala Pro Asp Leu Gly Ala
    370                 375                 380
Ala Ser Thr Ser Glu Glu Leu Pro Gln Ser Gln Lys Glu Leu Gly Phe
385                 390                 395                 400
Ser Tyr Leu Ala Phe Pro Ser Gly Pro Glu Pro Ser Leu Gln Ala Asp
                405                 410                 415
Leu Ser Lys Asp Leu Val Cys Thr Pro Pro Tyr Thr Pro His Gln Pro
            420                 425                 430
Gly Gly Cys Ala Phe Leu Phe Ser Leu His Glu Pro Phe Gln Thr His
        435                 440                 445
Leu Ser Thr Pro Ser Ser Ser Leu Gln Glu Gln Leu Thr Pro Ser Thr
    450                 455                 460
Ala Thr Phe Ser Asp Gln Leu Thr Pro Ser Ser Ala Thr Phe Pro Asp
465                 470                 475                 480
Pro Leu Met Ser Pro Leu Gln Gly Gln Met Thr Glu Ser Ser Ala Arg
                485                 490                 495
Ser Tyr Glu Asp Gln Leu Thr Pro Cys Thr Ser Ala Phe Pro Asp Gln
            500                 505                 510
Leu Leu Pro Ser Thr Ala Thr Phe Pro Glu Pro Leu Gly Ser Pro Ala
        515                 520                 525
His Glu Gln Leu Thr Pro Pro Ser Thr Ala Phe Gln Ala His Leu Asn
    530                 535                 540
Ser Pro Ser Gln Thr Phe Pro Glu Gln Leu Ser Pro Asn Pro Thr Lys
545                 550                 555                 560
Thr Tyr Phe Thr Gln Glu Gly Cys Ser Phe Leu Tyr Glu Lys Leu Pro
                565                 570                 575
Pro Ser Pro Ser Ser Pro Gly Asn Gly Asp Cys Thr Leu Leu Ala Leu
            580                 585                 590
Ala Gln Leu Arg Gly Pro Leu Ser Val Asp Val Pro Leu Val Pro Glu
        595                 600                 605
Gly Leu Leu Thr Pro Glu Ala Ser Pro Val Lys Gln Ser Phe Phe His
    610                 615                 620
Tyr Ser Glu Lys Glu Gln Asn Glu Ile Asp Arg Leu Ile Gln Gln Ile
625                 630                 635                 640
Ser Gln Leu Ala Gln Gly Met Asp Arg Pro Phe Ser Ala Glu Ala Gly
                645                 650                 655
Thr Gly Gly Leu Glu Pro Leu Gly Gly Leu Glu Pro Leu Asp Ser Asn
            660                 665                 670
Leu Ser Leu Pro Gly Ala Gly Pro Pro Val Leu Ser Leu Asp Leu Lys
        675                 680                 685
Pro Trp Lys Cys Gln Glu Leu Asp Phe Leu Ala Asp Pro Asp Asn Ile
    690                 695                 700
Phe Leu Glu Glu Thr Pro Val Glu Asp Ile Phe Met Asp Leu Ser Thr
705                 710                 715                 720
Pro Asp Pro Asn Gly Glu Trp Ser Ser Gly Asp Pro Glu Ala Ala Val
                725                 730                 735
Pro Gly Gly Ala Ser Ser Pro Cys Asn Asn Leu Ser Pro Glu Asp His
            740                 745                 750
Ser Phe Leu Glu Asp Leu Ala Thr Tyr Glu Thr Ala Phe Glu Thr Gly
        755                 760                 765
Val Ser Ala Phe Pro Tyr Asp Gly Phe Thr Asp Glu Leu His Gln Leu
    770                 775                 780
Gln Ser Gln Val Gln Asp Ser Phe His Glu Asp Gly Ser Gly Gly Glu
785                 790                 795                 800

Pro Thr Phe

<210> 20
<211> 797
<212> PRT
<213> Bos taurus

<400> 20
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Ser Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Ala Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Thr Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Ala Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Gly His Ala Ser Ala Gln His Tyr Arg Leu Leu
            260                 265                 270
Ala Glu Ser Gly Asp Ile Gln Ala Glu Met Val Val Arg Leu Leu Ala
        275                 280                 285
Lys Pro Gly Gly Trp Ala Trp Ile Tyr Cys Leu Leu Tyr Ser Glu Gly
    290                 295                 300
Pro Glu Gly Pro Ile Thr Ala Asn Asn Tyr Pro Ile Ser Asp Met Glu
305                 310                 315                 320
Ala Trp Ser Leu Arg Gln Gln Leu Asn Ser Glu Glu Thr Gln Ala Ala
                325                 330                 335
Tyr Val Leu Gly Thr Pro Thr Met Leu Pro Ser Phe Pro Glu Asn Ile
            340                 345                 350
Leu Ser Gln Glu Gln Cys Ser Ser Pro Asn Pro Leu Phe Thr Met Gly
        355                 360                 365
Pro Pro Arg Ser Thr Ser Phe Pro Ser Ala Pro Glu Leu Gly Ala Val
    370                 375                 380
Pro Thr Ser Glu Glu Leu Pro Gln Ala Pro Lys Asp Leu Gly Phe Tyr
385                 390                 395                 400
Leu Thr Phe Pro Ser Gly Leu Gln Pro Ser Leu Gln Ala Asp Leu Ser
                405                 410                 415
Lys Asp Leu Val Cys Thr Pro Pro Tyr Thr Pro His Gln Pro Gly Gly
            420                 425                 430

Cys Ala Phe Leu Phe Ser Leu His Glu Pro Phe Gln Thr His Met Pro
        435                 440                 445
Ala Pro Ser Ser Ser Leu Gln Glu Gln Leu Thr Pro Ser Ala Val Thr
    450                 455                 460
Phe Ser Asp Gln Leu Thr Pro Asn Ser Ala Thr Phe Pro Asp Pro Leu
465                 470                 475                 480
Thr Ser Pro Leu Gln Gly Gln Leu Thr Glu Thr Ser Ala Arg Ser Tyr
                485                 490                 495
Glu Asp Pro Cys Thr Ser Thr Phe Pro Asp Gln Leu Leu Pro Ser Ala
            500                 505                 510
Ala Thr Phe Pro Glu Pro Leu Ser Ser Pro Ala Gln Glu Gln Leu Thr
        515                 520                 525
Ser Pro Ser Thr Ser Phe Gln Ala His Leu Asn Ser Pro Arg Gln Thr
    530                 535                 540
Phe Pro Glu Gln Leu Ser Pro Asn Ser Ser Lys Thr Tyr Phe Ala Gln
545                 550                 555                 560
Glu Gly Cys Ser Phe Leu Tyr Glu Lys Leu Pro Pro Ser Pro Ser Ser
                565                 570                 575
Pro Gly Asn Gly Asp Cys Thr Leu Leu Ala Leu Ala Gln Leu Arg Gly
            580                 585                 590
Pro Leu Ser Val Asp Val Pro Leu Val Pro Glu Gly Leu Leu Thr Pro
        595                 600                 605
Glu Ala Ser Pro Val Lys Gln Ser Phe Phe His Tyr Ser Glu Lys Glu
    610                 615                 620
Gln Ser Glu Ile Asp Arg Leu Ile Gln Gln Ile Ser Gln Leu Ala Gln
625                 630                 635                 640
Gly Met Asp Arg Pro Phe Ser Ala Glu Ala Gly Thr Gly Gly Leu Glu
                645                 650                 655
Pro Leu Gly Gly Leu Glu Pro Leu Asp Ser Asn Leu Ser Leu Ser Gly
            660                 665                 670
Ala Gly Pro Pro Val Leu Ser Leu Glu Leu Lys Pro Trp Lys Cys Gln
        675                 680                 685
Asp Leu Asp Phe Leu Thr Asp Pro Asp Leu Phe Leu Glu Glu Thr Pro
    690                 695                 700
Val Glu Asp Ile Phe Met Asp Leu Ser Thr Pro Asp Pro Asn Gly Glu
705                 710                 715                 720
Trp Glu Ser Glu Asp Pro Glu Ala Ala Val Pro Gly Gly Ala Pro Ser
                725                 730                 735
Pro Cys Asn Asn Leu Ser Pro Glu Asp Arg Ser Phe Leu Glu Asp Leu
            740                 745                 750
Ala Thr Tyr Glu Thr Ala Phe Glu Thr Gly Val Ser Ala Phe Pro Tyr
        755                 760                 765
Asp Gly Phe Thr Asp Glu Leu His Gln Leu Gln Ser Gln Val Gln Asp
    770                 775                 780
Ser Phe His Lys Asp Gly Ser Gly Gly Glu Pro Thr Phe
785                 790                 795

<210> 21
<211> 804
<212> PRT
<213> Loxodonta africana

<400> 21
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80

Leu Pro Gly Phe Leu Leu Val Phe Thr Ala Glu Gly Lys Leu Leu Tyr
                85                  90                  95
Leu Ser Asp Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Pro His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Thr Leu Leu Asp Ile Ser Glu Ser Val Leu Ile Tyr Leu
225                 230                 235                 240
Gly Phe Glu Arg Asn Glu Leu Leu Cys Lys Ser Trp Tyr Gly Leu Leu
                245                 250                 255
His Pro Glu Asp Leu Ala His Ala Ser Ala Gln His Tyr Arg Leu Leu
            260                 265                 270
Ala Glu Asn Gly Asp Ile Gln Ala Glu Met Val Val Arg Leu Gln Ala
        275                 280                 285
Lys Ala Gly Gly Trp Ala Trp Ile Tyr Cys Leu Leu Tyr Ser Glu Cys
    290                 295                 300
Leu Glu Gly Pro Ile Thr Ala Asn Asn Tyr Pro Ile Ser Asp Ser Glu
305                 310                 315                 320
Ala Trp Ser Leu Arg Gln Gln Leu Asn Ser Glu Asp Thr Gln Thr Ser
                325                 330                 335
Tyr Val Leu Gly Thr Pro Thr Met Leu Pro Pro Phe Pro Glu Ser Ile
            340                 345                 350
Leu Ser Gln Glu Gln Cys Ser Asn Pro Asn Pro Val Phe Thr Pro Arg
        355                 360                 365
Leu Gly Thr Pro Arg Ser Thr Thr Phe Pro Gly Ala Pro Glu Leu Gly
    370                 375                 380
Val Ala Ser Ala Ser Glu Glu Leu Ser Arg Pro Pro Lys Glu Leu Asp
385                 390                 395                 400
Phe Ser Tyr Leu Thr Phe Pro Ala Gly Pro Glu Pro Ser Leu Gln Ala
                405                 410                 415
Asp Leu Ser Lys Asp Phe Val Cys Thr Pro Pro Tyr Thr Pro Arg Gln
            420                 425                 430
Pro Gly Gly Cys Ala Phe Leu Phe Ser Leu His Glu Pro Phe Gln Thr
        435                 440                 445
His Leu Pro Thr Pro Ser Ser Ser Leu Gln Glu Gln Leu Thr Pro Ser
    450                 455                 460
Thr Ala Thr Phe Ser Asp Gln Leu Thr Pro Ser Ser Ala Thr Phe Pro
465                 470                 475                 480
Asp Pro Leu Thr Ser Pro Leu Arg Gly Gln Leu Thr Glu Ala Ser Ala
                485                 490                 495
Arg Ser Tyr Glu Asp Gln Leu Thr Pro Cys Thr Ser Thr Phe Pro Asp
            500                 505                 510
Gln Leu Leu Pro Ser Thr Ala Thr Phe Pro Glu Pro Leu Ser Ser Pro
        515                 520                 525
Ser Gln Glu Gln Leu Thr Pro Pro Ser Thr Ala Phe Gln Ala His Leu
    530                 535                 540
Ser Ser Pro Ser Gln Thr Phe Pro Glu Gln Leu Ser Pro Asn Ser Thr
545                 550                 555                 560
Lys Thr Tyr Phe Ala Gln Glu Gly Cys Ser Phe Leu Tyr Glu Lys Leu
                565                 570                 575
Pro Pro Ser Pro Ser Ser Pro Gly Asn Gly Asp Cys Thr Leu Leu Ala

                    580                 585                 590
Leu Ala Gln Leu Arg Gly Pro Leu Ser Val Asp Val Pro Leu Met Pro
            595                 600                 605
Glu Gly Leu Leu Thr Pro Glu Ala Ser Pro Val Lys Gln Ser Phe Phe
    610                 615                 620
His Tyr Ser Glu Lys Glu Gln Asn Glu Ile Asp Arg Leu Ile Gln Gln
625                 630                 635                 640
Ile Ser Gln Leu Ala Gln Gly Met Asp Arg Pro Phe Ser Ala Glu Ala
                645                 650                 655
Gly Thr Gly Gly Leu Glu Pro Leu Gly Gly Leu Glu Pro Leu Asp Ser
            660                 665                 670
Asn Leu Ser Leu Pro Gly Ala Gly Pro Pro Val Leu Ser Leu Asp Leu
        675                 680                 685
Lys Pro Trp Arg Cys Gln Glu Pro Asp Phe Leu Ala Asp Pro Asp Asp
    690                 695                 700
Ile Phe Leu Glu Glu Thr Pro Val Glu Asp Ile Phe Met Asp Leu Ser
705                 710                 715                 720
Thr Ser Ala Pro Asn Gly Glu Trp Gly Ser Glu Gly Pro Glu Ala Ala
                725                 730                 735
Gly Pro Gly Gly Ala Leu Ser Pro Cys Asn Asn Leu Ser Pro Glu Asp
            740                 745                 750
His Ser Phe Leu Glu Asp Leu Ala Thr Tyr Glu Thr Ala Phe Glu Thr
        755                 760                 765
Gly Val Ser Ala Phe Pro Tyr Asp Gly Phe Thr Asp Glu Leu His Gln
    770                 775                 780
Leu Gln Ser Gln Val Gln Asp Ser Phe His Glu Asp Gly Ser Gly Gly
785                 790                 795                 800
Glu Pro Thr Phe


<210> 22
<211> 796
<212> PRT
<213> Sarcophilus harrisii

<400> 22
Met Tyr Arg Ser Thr Lys Gly Ala Ser Lys Ala Arg Arg Asp Gln Ile
1               5                   10                  15
Asn Ala Glu Ile Arg Ser Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Ala Gly Ala Pro Leu Thr
    50                  55                  60
Gly Pro Ala Gly Leu Leu Ser Ala Gln Glu Leu Glu Asp Ile Val Ala
65                  70                  75                  80
Ala Leu Pro Gly Phe Leu Leu Met Phe Thr Ala Glu Gly Lys Leu Leu
                85                  90                  95
Tyr Leu Ser Asp Ser Val Ser Glu His Leu Gly His Ser Met Val Asp
            100                 105                 110
Leu Val Ala Gln Gly Asp Ser Ile Tyr Asp Ile Ile Asp Pro Ala Asp
        115                 120                 125
His Leu Thr Val Arg Gln Gln Leu Thr Leu Pro Ser Leu Pro Asp Thr
    130                 135                 140
Glu Arg Leu Phe Arg Cys Arg Phe Asn Thr Ser Lys Ser Leu Arg Arg
145                 150                 155                 160
Gln Ser Ala Gly Asn Lys Leu Val Leu Ile Arg Gly Arg Phe His Pro
                165                 170                 175
His Pro Pro Gly Ala Tyr Trp Ala Gly Asn Pro Val Phe Thr Ala Phe
            180                 185                 190
Cys Ala Pro Leu Asp Pro Arg Ala Arg Pro Gly Pro Gly Cys Gly Pro
        195                 200                 205
Gly Leu Ala Ser Ile Phe Leu Ala Val Phe Gln Ser Arg His Ala Lys

210                 215                 220
Asp Leu Ala Leu Leu Asp Val Ser Glu Ser Val Leu Val Tyr Leu Gly
225                 230                 235                 240
Phe Glu Arg Ser Glu Leu Val Cys Lys Ser Trp Tyr Gly Leu Leu His
                245                 250                 255
Pro Glu Asp Leu Ala His Ala Ser Ala Gln His Tyr Arg Leu Leu Ala
            260                 265                 270
Asp Gly Gly Asp Val Gln Ala Glu Met Val Val Arg Leu Gln Ala Lys
        275                 280                 285
Ala Gly Gly Trp Val Trp Ile Tyr Ser Leu Met His Pro Glu Gly Ser
    290                 295                 300
Asp Gly Pro Ile Thr Ala Asn Asn Tyr Pro Ile Ser Asp Ser Glu Ala
305                 310                 315                 320
Trp Ser Leu Arg Gln Gln Leu Ser Ser Glu Asp Ala His Leu Ala Tyr
                325                 330                 335
Val Leu Gly Thr Thr Ala Ala Pro Ala Ala Phe Pro Gly Ser Leu Leu
            340                 345                 350
Ser Pro Ala Gln Cys Ala Ser Pro Asp Pro Thr Phe Pro Pro Ala Phe
        355                 360                 365
Gly Gly Pro Arg Ser Thr Ile Phe Pro Thr Gly Pro Glu Leu Ala Pro
    370                 375                 380
Ile Pro Ser Gly Glu Gln Leu Leu Gln Pro Pro Lys Glu Met Asp Phe
385                 390                 395                 400
Ser Tyr Leu Ala Phe Pro Pro Arg Pro Glu Ser Thr Phe Gln Gly Asp
                405                 410                 415
Leu Cys Lys Asp Phe Val Cys Thr Pro Pro Tyr Thr Pro His Gln Pro
            420                 425                 430
Gly Gly Cys Ala Phe Leu Phe Gly Leu Gln Glu Pro Phe Gln Pro His
        435                 440                 445
Leu Pro Ala Pro Ser Gly Ser Gly Thr Glu Gln Leu Pro Pro Gly Ala
    450                 455                 460
Gly Ile Phe Pro Glu Gln Leu Thr Pro Ser Ser Ala Thr Phe Pro Glu
465                 470                 475                 480
Leu Ala Gly Pro Val Pro Gly Gln Leu Thr Asp Thr Pro Ala Gln Thr
                485                 490                 495
Tyr Glu Asp Gln Leu Pro Pro Cys Gly Pro Ala Phe Pro Glu Gln Leu
            500                 505                 510
Leu Pro Gly Ala Ala Thr Phe Gln Glu Gln Leu Ala Ser Pro Gly Gln
        515                 520                 525
Ala Gln Leu Thr Pro Pro Ser Thr Ala Phe Pro Thr His Thr Ala Ser
    530                 535                 540
Pro Gly Arg Thr Phe Pro Glu Gln Gln Ser Pro Thr Pro Ser Lys Pro
545                 550                 555                 560
Tyr Phe Pro Gln Glu Gly Cys Ser Phe Leu Tyr Glu Lys Leu Pro Pro
                565                 570                 575
Ser Pro Asn Ser Pro Gly Asn Gly Asp Cys Thr Leu Leu Ala Leu Ala
            580                 585                 590
Gln Leu Arg Gly Pro Leu Ser Val Asp Val Pro Leu Val Pro Asp Gly
        595                 600                 605
Leu Leu Thr Pro Glu Ala Ser Pro Val Lys Gln Ser Phe Phe His Tyr
    610                 615                 620
Ser Glu Lys Glu Arg Asn Glu Leu Asp Arg Leu Ile Arg Gln Ile Ser
625                 630                 635                 640
Gln Leu Ala Arg Gly Leu Asp Cys Pro Phe Pro Ala Glu Pro Gly Ala
                645                 650                 655
Glu Gly Leu Glu Pro Leu Gly Gln Ala Leu Ser Phe Pro Gly Ala Gly
            660                 665                 670
Pro Pro Leu Phe Gly Leu Asp Pro Lys Ser Trp Lys Ser Gln Asp Leu
        675                 680                 685
Ala Phe Leu Asp Ala Pro Glu Ala Pro Phe Leu Glu Glu Lys Ser Pro
    690                 695                 700
Glu Asp Ile Phe Met Gly Leu Ser Ala Pro Ala Pro Gln Gly Asp Trp
705                 710                 715                 720

Gly Pro Gly Gly Pro Glu Thr Ser Gly Pro Gly Gly Val Pro Ser Pro
                725                 730                 735
Cys Thr Asp Leu Ser Pro Glu Asp His Ser Phe Leu Glu Asp Leu Ala
            740                 745                 750
Thr Tyr Glu Pro Asp Phe Glu Thr Gly Val Ser Ala Phe Pro Cys Asp
        755                 760                 765
Gly Cys Thr Asp Glu Leu Tyr Gln Leu Gln Ser Gln Val Gln Asp Ser
    770                 775                 780
Phe His Asp Asp Gly Ser Gly Gly Glu Pro Thr Phe
785                 790                 795

<210> 23
<211> 802
<212> PRT
<213> Equus caballus

<400> 23
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Pro Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Thr Leu Pro Ser Ala Leu Asp Ser Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Pro Arg Pro Arg Pro Gly Pro Gly Pro Gly Leu Gly
        195                 200                 205
Pro Ala Ser Leu Phe Leu Ala Met Phe Gln Ser Arg His Ala Lys Asp
    210                 215                 220
Leu Ala Leu Leu Asp Ile Ser Glu Ser Val Leu Ile Tyr Leu Gly Phe
225                 230                 235                 240
Glu Arg Ser Glu Leu Leu Cys Lys Ser Trp Tyr Gly Leu Leu His Pro
                245                 250                 255
Glu Asp Leu Gly His Ala Ser Ala Gln His Tyr Arg Leu Leu Ala Glu
            260                 265                 270
Ser Gly Asp Ile Gln Ala Glu Met Val Val Arg Leu Gln Ala Lys Pro
        275                 280                 285
Gly Gly Trp Ala Trp Ile Tyr Cys Leu Leu Tyr Ser Glu Gly Pro Glu
    290                 295                 300
Ser Pro Ile Thr Ala Asn Asn Tyr Pro Ile Ser Asp Thr Glu Ala Trp
305                 310                 315                 320
Ser Leu Arg Gln Gln Leu Asn Ser Glu Asp Thr Gln Ala Ala Tyr Ile
                325                 330                 335
Leu Gly Thr Pro Ala Val Leu Pro Ser Phe Pro Glu Asn Ile Leu Ser
            340                 345                 350
Gln Glu Gln Cys Ser Ser Ser Asn Pro Leu Phe Asn Pro Ala Leu Gly
        355                 360                 365

Ala Pro Arg Ser Thr Ser Phe Pro Ser Ala Pro Glu Leu Gly Ala Val
    370                 375                 380
Ser Thr Ser Glu Glu Leu Pro Arg Pro Pro Lys Glu Leu Gly Phe Ser
385                 390                 395                 400
Tyr Met Thr Phe Ser Ser Gly Pro Glu Pro Ser Leu Gln Ala Asp Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Leu
        435                 440                 445
Pro Thr Pro Ser Ser Ser Leu Gln Glu Gln Met Thr Pro Ser Thr Ala
    450                 455                 460
Thr Phe Ser Asp Gln Leu Thr Pro Ser Ser Ala Thr Phe Pro Asp Pro
465                 470                 475                 480
Leu Thr Ser Pro Leu Gln Gly Gln Leu Thr Glu Thr Ser Thr Arg Ser
                485                 490                 495
Tyr Glu Asp Gln Leu Asn Pro Cys Thr Ser Thr Phe Pro Asp Gln Leu
            500                 505                 510
Leu Pro Ser Thr Ala Thr Phe Pro Glu Pro Leu Gly Ser Pro Ala Gln
        515                 520                 525
Glu Gln Leu Thr Pro Pro Ser Thr Ala Phe Gln Ala His Leu Asn Ser
    530                 535                 540
Pro Ser Gln Thr Phe Pro Glu Gln Leu Ser Pro Ser His Thr Lys Thr
545                 550                 555                 560
Tyr Phe Ala Gln Glu Gly Cys Ser Phe Leu Tyr Glu Lys Leu Pro Pro
                565                 570                 575
Ser Pro Ser Ser Pro Gly Asn Gly Asp Cys Thr Leu Leu Ala Leu Ala
            580                 585                 590
Gln Leu Arg Gly Pro Leu Ser Val Asp Val Pro Leu Met Pro Glu Gly
        595                 600                 605
Leu Leu Thr Pro Glu Ala Ser Pro Val Lys Gln Ser Phe Phe His Tyr
    610                 615                 620
Ser Glu Lys Glu Gln Asn Glu Ile Asp Arg Leu Ile Gln Gln Ile Ser
625                 630                 635                 640
Gln Leu Ala Gln Gly Met Asp Arg Pro Phe Ser Ala Glu Ala Ser Thr
                645                 650                 655
Gly Gly Leu Glu Pro Leu Gly Gly Leu Glu Pro Leu Asp Ser Asn Leu
            660                 665                 670
Ser Leu Ser Gly Ala Gly Pro Pro Val Leu Ser Leu Asp Leu Lys Pro
        675                 680                 685
Trp Lys Cys Gln Glu Leu Asp Phe Leu Ala Asp Pro Asp Thr Ile Phe
    690                 695                 700
Leu Glu Glu Thr Pro Val Glu Asp Ile Phe Met Asp Leu Ser Thr Pro
705                 710                 715                 720
Asp Pro Ser Gly Glu Trp Ser Ser Gly Asp Pro Glu Ala Ala Val Pro
                725                 730                 735
Gly Gly Ala Pro Ser Pro Cys Asn Asn Leu Ser Pro Glu Asp His Ser
            740                 745                 750
Phe Leu Glu Asp Leu Ala Thr Tyr Glu Thr Ala Phe Glu Thr Gly Val
        755                 760                 765
Ser Ala Phe Pro Tyr Asp Gly Phe Pro Asp Glu Leu Tyr Gln Leu Gln
    770                 775                 780
Ser Gln Val Gln Asp Ser Phe His Asp Asp Gly Ser Gly Gly Glu Pro
785                 790                 795                 800
Thr Phe

<210> 24
<211> 798
<212> PRT
<213> Ovis aries

<400> 24

Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Leu Ala Glu Ala
            20                  25                  30
Asp Lys Val Arg Leu Ser Tyr Leu His Ile Met Ser Leu Ala Cys Ile
        35                  40                  45
Tyr Thr Arg Lys Gly Val Phe Phe Ala Gly Gly Thr Pro Leu Ala Gly
    50                  55                  60
Ser Thr Gly Leu Leu Ser Ala Gln Glu Leu Glu Asp Ile Val Ala Ala
65                  70                  75                  80
Leu Pro Gly Phe Leu Leu Val Phe Thr Ala Glu Gly Lys Leu Leu Tyr
                85                  90                  95
Leu Ser Glu Ser Val Ser Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Leu Thr Val Arg Gln Gln Leu Ala Leu Pro Ser Ala Leu Asp Thr Asp
    130                 135                 140
Arg Leu Phe Arg Cys Arg Phe Asn Thr Ser Lys Ser Leu Arg Arg Gln
145                 150                 155                 160
Ser Ala Gly Asn Lys Leu Val Leu Ile Arg Gly Arg Phe His Ala His
                165                 170                 175
Pro Pro Gly Ala Tyr Trp Ala Gly Asn Pro Val Phe Thr Ala Phe Cys
            180                 185                 190
Ala Pro Leu Glu Thr Arg Pro Arg Pro Gly Pro Gly Pro Gly Pro Gly
        195                 200                 205
Pro Gly Pro Ala Ser Leu Phe Leu Ala Met Phe Gln Ser Arg His Ala
    210                 215                 220
Lys Asp Leu Ala Leu Leu Asp Ile Ser Glu Ser Val Leu Ile Tyr Leu
225                 230                 235                 240
Gly Phe Glu Arg Ser Glu Leu Leu Cys Lys Ser Trp Tyr Gly Leu Leu
                245                 250                 255
His Pro Glu Asp Leu Gly His Ala Ser Ala Gln His Tyr Arg Leu Leu
            260                 265                 270
Ala Glu Ser Gly Asp Ile Gln Ala Glu Met Val Val Arg Leu Leu Ala
        275                 280                 285
Lys Pro Gly Gly Trp Ala Trp Ile Tyr Cys Leu Leu Tyr Ser Glu Gly
    290                 295                 300
Pro Glu Gly Pro Ile Thr Ala Asn Asn Tyr Pro Ile Ser Asp Met Glu
305                 310                 315                 320
Ala Trp Ser Leu Arg Gln Gln Leu Asn Ser Glu Glu Thr Gln Ala Ala
                325                 330                 335
Tyr Val Leu Gly Thr Pro Thr Met Leu Pro Ser Phe Pro Glu Asn Ile
            340                 345                 350
Leu Ser Gln Glu Gln Cys Ser Ser Pro Asn Pro Leu Phe Thr Met Gly
        355                 360                 365
Pro Pro Arg Ser Thr Ser Phe Pro Ser Ala Pro Glu Leu Gly Ala Val
    370                 375                 380
Ser Thr Ser Glu Glu Leu Pro Arg Ala Pro Lys Asp Leu Gly Phe Ser
385                 390                 395                 400
Tyr Leu Thr Phe Pro Ser Gly Pro Gln Pro Ser Leu Gln Ala Asp Leu
                405                 410                 415
Ser Lys Asp Leu Val Cys Thr Pro Pro Tyr Thr Pro His Gln Pro Gly
            420                 425                 430
Gly Cys Ala Phe Leu Phe Ser Leu His Glu Pro Phe Gln Thr His Met
        435                 440                 445
Pro Ala Pro Ser Ser Ser Leu Gln Glu Gln Leu Thr Pro Ser Thr Val
    450                 455                 460
Thr Phe Ser Asp Gln Leu Thr Pro Asn Ser Ala Thr Phe Pro Asp Pro
465                 470                 475                 480
Leu Thr Ser Pro Leu Gln Gly Gln Leu Thr Glu Thr Ser Ala Arg Ser
                485                 490                 495
Tyr Glu Asp Pro Cys Thr Ser Thr Phe Pro Asp Gln Leu Leu Pro Ser

500                 505                 510
Thr Ala Thr Phe Pro Glu Pro Leu Ser Ser Pro Ala Gln Glu Gln Leu
        515                 520                 525
Thr Ser Pro Ser Thr Ser Phe Gln Ala His Leu Asn Ser Pro Arg Gln
    530                 535                 540
Thr Phe Pro Glu Gln Leu Ser Pro Asn Ser Ser Lys Thr Tyr Phe Ala
545                 550                 555                 560
Gln Glu Gly Cys Ser Phe Leu Tyr Glu Lys Leu Pro Pro Ser Pro Ser
                565                 570                 575
Ser Pro Gly Asn Gly Asp Cys Thr Leu Leu Ala Leu Ala Gln Leu Arg
            580                 585                 590
Gly Pro Leu Ser Val Asp Val Pro Leu Val Pro Glu Gly Leu Leu Thr
        595                 600                 605
Pro Glu Ala Ser Pro Val Lys Gln Ser Phe Phe His Tyr Ser Glu Lys
    610                 615                 620
Glu Gln Ser Glu Ile Asp Arg Leu Ile Gln Gln Ile Ser Gln Leu Ala
625                 630                 635                 640
Gln Gly Met Asp Arg Pro Phe Ser Ala Glu Ala Gly Thr Gly Gly Leu
                645                 650                 655
Glu Pro Leu Gly Gly Leu Glu Pro Leu Asp Ser Asn Leu Ser Leu Ser
            660                 665                 670
Gly Ala Gly Pro Pro Val Leu Ser Leu Glu Leu Lys Pro Trp Lys Cys
        675                 680                 685
Gln Asp Leu Asp Phe Leu Thr Asp Pro Asp Leu Phe Leu Glu Glu Thr
    690                 695                 700
Pro Val Glu Asp Ile Phe Met Asp Leu Ser Thr Pro Asp Pro Asn Gly
705                 710                 715                 720
Glu Trp Glu Ser Glu Asp Pro Glu Ala Ala Val Pro Gly Gly Ala Ser
                725                 730                 735
Ser Pro Cys Asn Asn Leu Ser Pro Glu Asp Arg Ser Phe Leu Glu Asp
            740                 745                 750
Leu Ala Thr Tyr Glu Thr Ala Phe Glu Thr Gly Val Ser Ala Phe Pro
        755                 760                 765
Tyr Asp Gly Phe Thr Asp Glu Leu His Gln Leu Gln Ser Gln Val Gln
    770                 775                 780
Asp Ser Phe His Lys Asp Gly Ser Gly Gly Glu Pro Thr Phe
785                 790                 795

<210> 25
<211> 647
<212> PRT
<213> Danio rerio

<400> 25
Met Ser Cys Gly Asp Ser Gly Ile Arg Arg Ile Leu Arg Ala Ser Lys
1               5                   10                  15
Arg Phe Arg Ser Thr Lys Gly Ala Ser Lys Ala Arg Arg Asp Gln Met
            20                  25                  30
Asn Ser Glu Ile Arg Asn Leu Arg Ala Leu Leu Pro Ile Ser Pro Glu
        35                  40                  45
His Arg Leu Ser Tyr Leu His Ser Met Ser Ile Thr Cys Thr Tyr Ile
    50                  55                  60
Arg Lys Ser Val Glu Leu Arg Gly Val Cys Glu Glu Ser Thr Val Phe
65                  70                  75                  80
Ser Ala Val Asn Gly Cys Val Pro Gln Asp Cys Ala Leu Gln Asp Cys
                85                  90                  95
Ala Leu Gln Glu Cys Val Leu Gln Glu Cys Val Leu Gln Glu Cys Val
            100                 105                 110
Leu Gln Glu Cys Val Leu Gln Ala Leu Pro Gly Phe Ile Val Ala Phe
        115                 120                 125
Thr Thr Asp Gly Lys Leu Leu Tyr Val Ser Glu Asn Val His Glu Tyr
    130                 135                 140
Leu Gly Leu Ser Met Val Asp Val Leu Gln Ser Asp Ser Phe Phe Asp

145                 150                 155                 160
Met Leu Asp Arg Ser Asp Val Glu Ala Val Arg Ser Val Leu Ala Asp
                165                 170                 175
Ala Ser Pro Ser Gly Glu Arg Trp Val Val Cys Arg Met Leu Val Ser
            180                 185                 190
Lys Ala Met Arg Leu Arg Ser Ser Cys Cys Pro Leu Leu Val Arg Ile
        195                 200                 205
Arg Leu Arg Asp Gly Val Cys Val Ser Leu Cys Arg Pro Thr Ala Asp
    210                 215                 220
Arg Leu Pro Ala Arg Asn Ala Asp Phe His Thr His His Ser Ala Asp
225                 230                 235                 240
Met Arg Leu Ala Ser Ala Ser Ser Ser Val Leu Phe His Leu Gly Phe
                245                 250                 255
Ser Ala Asp Glu Leu Ile Gly Arg Ser Trp Tyr Glu Leu Leu His Pro
            260                 265                 270
Asp Asp Leu Arg His Ala Ala Asp Arg His Ala Ala Ile Leu Ala Ala
        275                 280                 285
Ala Thr Ala Asp Ala Glu Met Leu Ile Arg Val Gln Cys Lys Asp Leu
    290                 295                 300
Ser Trp Val Trp Met Tyr Thr His Ala Ser Ala Thr Ala Glu Arg Asp
305                 310                 315                 320
Ala Ile Ser Cys Ser Asn Tyr Met Ile Ser Glu Ala Glu Ala Val Tyr
                325                 330                 335
Leu Gln Gln Arg Leu Ser Ser Ser Ser Ser Ser Ser Thr Ser Ser Ser
            340                 345                 350
Ser Ser Ser Ala Ser Pro Gln Cys Ser Ser Val Ser Asp Ser Ser Asp
        355                 360                 365
Ala His Ser Ala His Thr Leu Phe Cys Thr Pro Pro Tyr Ser Pro Thr
    370                 375                 380
Ser Ser Gln Cys Ser Asp Phe Leu Ser Glu Gly Tyr Gly Ser Leu Glu
385                 390                 395                 400
Ala Leu Val Asp Ser Ala Phe Cys Ser Pro Pro Tyr Pro Pro Leu Phe
                405                 410                 415
Pro Asn Pro Cys Cys Thr Pro Thr Val Cys Pro Pro Asp Thr Ala Leu
            420                 425                 430
Asp Pro Ala Arg Leu Cys Pro Pro Asp Ser Ala Leu Val Pro Ala Ala
        435                 440                 445
Leu Cys Pro Pro Asp Ala Ala Leu Asp Pro Thr Gln Leu Cys Pro Pro
    450                 455                 460
Gly Ala Ala Leu Asp Pro Thr Leu Leu Cys Pro Pro Gly Ala Ala Leu
465                 470                 475                 480
Asp Pro Thr Gln Leu Cys Leu Pro Asp Gly Ala Leu Val Pro Ala Val
                485                 490                 495
Phe Cys Pro Pro Asn Gly Ala Leu Val Pro Ala Arg Leu Cys Pro Ser
            500                 505                 510
Asp Ala Ala Leu Val Pro Ala Ala Leu Gly Arg Pro Asp Ala Ala Leu
        515                 520                 525
Val Pro Val Cys Arg Pro Leu Gln Val Cys Glu Cys Pro Leu Asp Gly
    530                 535                 540
Ala Val Pro Leu Glu Asp Leu Ser Met Phe Pro Leu Pro Gln Gly Gly
545                 550                 555                 560
Gly Ser Arg Leu Met Pro Pro Glu Ala Ser Pro Thr Thr His Thr His
                565                 570                 575
Phe Ser Tyr Asp Ala Ala Gln Gln Ala Gly Ile Gly Thr Leu Ala Met
            580                 585                 590
Gln Ile His Thr Leu Ile Cys Ser Phe Asp Ala Tyr Ser Ala Ala Arg
        595                 600                 605
His Thr His Ser His His Thr Ser Cys Trp Ala Pro Glu Pro Leu Leu
    610                 615                 620
Asp Glu Gly Ile Ile Asp Ser Ile Leu Arg Glu Leu Asp Ser Thr His
625                 630                 635                 640
Thr Asp Ala His Thr Leu Ile
                645

<210> 26
<211> 688
<212> PRT
<213> Astyanax mexicanus

<400> 26
   Met Val Ser Cys Arg Ser Thr Lys Gly Ala Ser Lys Ala Arg Arg Asp
   1               5                   10                  15
   His Ile Asn Ala Glu Ile Arg Lys Met Arg Glu Leu Leu Pro Ile Pro
               20                  25                  30
   Ala Glu Glu Gln Glu Arg Leu Ser Tyr Leu His Thr Met Ser Ile Ile
           35                  40                  45
   Cys Thr Phe Ile Arg Lys Thr Ile Leu Leu Ser Gly Asp Gly Gly Arg
       50                  55                  60
   Gly Asp Glu Ala Pro Leu Pro Leu Tyr Glu Asp Trp Leu Gln Ala Leu
   65                  70                  75                  80
   Pro Gly Phe Ile Val Ala Val Asp Lys Glu Gly Lys Leu Val Tyr Val
                   85                  90                  95
   Ser Glu Asn Val Ser Gln Tyr Leu Gly Phe Ser Met Val Asp Val Leu
               100                 105                 110
   Gln Ser Asp Ser Leu Tyr Glu Leu Met Asp Pro Asp Asp Val Asp Ser
           115                 120                 125
   Val Lys Tyr Ser Leu Glu Glu Glu Ser Gly Ala Ala Glu Arg Ser Phe
       130                 135                 140
   Val Cys Arg Met Asn Thr Ser Lys Ala Phe Arg Leu Gln Tyr Gly Gly
   145                 150                 155                 160
   Ser Cys Pro Leu Leu Val Gln Gly Trp Phe Gln Asp Pro Gln His Cys
                   165                 170                 175
   Leu Phe Val Ala Leu Cys Ser Pro Thr Val Asp Arg Leu Thr Asp Ser
               180                 185                 190
   Glu Arg Leu Ser Phe Arg Ala His Phe Gln Ser Val His Arg Ala Asp
           195                 200                 205
   Met Ser Tyr Thr His Thr Asp His Ser Val Ala Leu Phe Leu Gly Tyr
       210                 215                 220
   Glu Ala Glu Glu Leu Ile Gly Arg Ser Trp Tyr Ser Met Leu His Pro
   225                 230                 235                 240
   Asp Asp Leu Ser Leu Ser Ala Ser Ala His Thr His Leu Ile Gln Gln
                   245                 250                 255
   Ser Gly Asp Ala Glu Val Glu Leu Val Phe Arg Val Gln Thr Lys Gly
               260                 265                 270
   His Ser Trp Val Trp Leu Tyr Thr Cys Ala Ser Lys Ser Leu Gln Lys
           275                 280                 285
   Gln Glu Ile Thr Cys Thr Asn Tyr Ile Ile Ser Lys Pro Glu Ala Glu
       290                 295                 300
   Tyr Ile Arg Gln Lys Leu Cys Ser Arg Ser Ser Ser Leu Leu Pro Leu
   305                 310                 315                 320
   Ser Thr Pro Ile Ser Gln Met Pro Trp Gly Pro Gly Pro Tyr Thr Ser
                   325                 330                 335
   Pro Lys Arg Gln Ala Glu Asn Ser Glu Gln Arg Ala Ala Pro Arg Lys
               340                 345                 350
   Lys Thr Arg Arg Leu Ser Glu Ser Pro Val Ser Ser Tyr Cys Val Thr
           355                 360                 365
   Thr Ser Thr Ser Thr Asn Gly His Ser Arg Asp Val Ser Ala Asp Gln
       370                 375                 380
   Ala Leu Phe Ser Thr Pro Pro Tyr Ser Pro Ala Ser Ser Leu Ser Asp
   385                 390                 395                 400
   Asp Cys Ser Leu Asp Ala Ser Gln Asp Met Met Tyr Ser Leu Thr Lys
                   405                 410                 415
   Thr Thr Ala Ser Pro Ser Ser Ser Tyr Ser Pro Ser Thr Ser Ser Ser
               420                 425                 430
   Ser Thr Leu Ser Phe Gln Leu Gln Pro Phe Leu Gln Ser Ser Phe Arg
           435                 440                 445
   Val Glu Pro Cys Pro Leu Thr Pro Pro Pro Tyr Ser Tyr Pro Asp Ala
       450                 455                 460
   Gln Ala Ala Ala Ser Leu Ser Pro Asn Tyr Gln Pro Val Glu Asp Cys
   465                 470                 475                 480
   Gly Gly Ser Thr Asp Ser Val Leu His Phe Glu Asp Phe Gly Leu Leu
                   485                 490                 495
   Ser Glu Leu Asn Ser Ser Glu Gly Glu Val Phe Gln Thr Pro Glu Asp
               500                 505                 510
   Ser Gly Ser Ile Ser Pro Ala Glu Leu Leu Thr Ile Asp Pro Ser Pro
           515                 520                 525
   Ile Ser Asn Ser Ser Thr Leu Ser Pro Gln Glu Gln Ala Glu Ile Ser
       530                 535                 540
   Val Leu Ala His Gln Ile Ser Thr Leu Ala Ser Ser Phe Asp Leu Tyr
   545                 550                 555                 560
   Arg Thr Lys Ser Ser Ala Pro Cys Trp Pro Ser Thr Ser Thr Gln Thr
                   565                 570                 575
   Ala Glu Pro Leu Leu Asp Glu Asp Val Ile Asp Cys Ile Leu Lys Asp
               580                 585                 590
   Trp Asp Gly Pro Thr Leu Arg Lys Glu Ser Gly Thr Gly Trp Gly Gln
           595                 600                 605
   Leu Pro Arg Thr Thr Met Glu Gln Gly Ser Ala Met Gln Gly Ser Ala
       610                 615                 620
   Val Gln Gly Leu Ser Leu Asp Ala Leu Val Asp Phe Ile Pro Ser Glu
   625                 630                 635                 640
   Leu Pro Ala Glu Asp Pro His Pro Trp Pro Cys Val Phe Arg Gln Asp
                   645                 650                 655
   Cys His Glu Asp Asp Ile Glu Leu His Gln Leu Gly Leu Tyr Leu Pro
               660                 665                 670
   Ser Asn Phe Gln Gln Gly Lys Lys Leu His Leu Pro Ala Pro Pro Leu
           675                 680                 685

<210> 27
<211> 721
<212> PRT
<213> Xenopus (Silurana) tropicalis

<400> 27
Met Tyr Arg Ser Thr Lys Gly Ala Ser Lys Ala Arg Arg Asp Gln Ile
1               5                   10                  15
Asn Ala Glu Ile Arg Asn Leu Lys Glu Leu Leu Pro Ile Ser Glu Gly
            20                  25                  30
Asp Lys Val Arg Leu Ser Tyr Leu His Ile Met Ser Leu Ala Cys Ile
        35                  40                  45
Tyr Thr Arg Lys Ser Val Phe Phe Ser Arg Gly Gln Gln Ala Glu Glu
    50                  55                  60
Leu Glu Gly Leu Leu Ser Asn Gln Asp Leu Met Asp Phe Val His Ser
65                  70                  75                  80
Leu Pro Gly Phe Leu Leu Thr Phe Thr Ser Glu Gly Lys Leu Ile Tyr
                85                  90                  95
Val Ser Glu Asn Val Ala Asp His Leu Gly His Ser Met Val Asp Leu
            100                 105                 110
Val Ala Gln Gly Asp Ser Ile Tyr Asp Ile Ile Asp Pro Ser Asp His
        115                 120                 125
Phe Val Met Arg Asn Gln Leu Ala Met Pro Ser Ser Pro Asp Ser Glu
    130                 135                 140
Arg Met Phe Arg Cys Arg Phe Asn Thr Ser Lys Thr Ala Arg Arg Gln
145                 150                 155                 160
Ser Ala Gly Asn Lys Leu Val Leu Ile Arg Gly Arg Phe Gln Gln Pro
                165                 170                 175
Pro Ala Gly Thr Tyr Trp Ser Ser Asn Pro Val Phe Thr Ala Phe Cys
            180                 185                 190

Ile Pro Leu Asp Pro Lys Pro Arg Val Ile Gln Ser His Leu Leu Val
        195                 200                 205
Asp Phe Phe Glu Ser Gln His Ala Lys Asp Leu Thr Val Leu Asp Val
    210                 215                 220
Ser Asp Ser Ile Leu Gln Tyr Leu Trp Tyr Glu Lys Ser Asp Leu Leu
225                 230                 235                 240
Ser Lys Ser Trp Tyr Asn Leu Ile His Pro Glu Asp Leu Thr His Leu
                245                 250                 255
Ser Lys Gln His Leu Lys Leu Leu Asn Glu Ser Cys Glu Gly Arg Ala
            260                 265                 270
Glu Val Val Ile Arg Leu Gln Arg Lys Asp Gln Leu Trp Val Trp Val
        275                 280                 285
Tyr Ser Ile Leu Ile Leu Glu Ala Ser Glu Ala Ser Ile Thr Ser Tyr
    290                 295                 300
Asn Tyr Ile Thr Ser Glu Glu Glu Ala Trp Cys Leu Arg Gln Gln Leu
305                 310                 315                 320
Thr Ser Glu Glu Thr Pro Val Ser Phe Ser Ala Gly Ala Ser Ser Tyr
                325                 330                 335
Gln Asp Thr Leu Leu Ser Pro Gly Asp Leu Ser Ser Pro Asp Gln Val
            340                 345                 350
Phe Thr Pro Leu Ala Asn Thr Pro Thr Ser Ala Gly Gln Ser Phe Asp
        355                 360                 365
Phe Ser Glu Pro Met Thr Ile Gly Ser Glu Asp Pro Ile Leu Cys Gln
    370                 375                 380
Thr Thr Ile Pro Leu Val Glu Asp Pro Val Ala Pro Glu Gly Gln Ser
385                 390                 395                 400
Glu Asn Gln Ser Ile Phe Thr Leu Phe Gln Pro Thr Ser His Glu His
                405                 410                 415
Thr Phe Arg Arg Asp Gln Leu Gly Thr Pro Lys Glu Phe Thr Cys Thr
            420                 425                 430
Pro Pro Tyr Thr Pro His Gln Asn Cys Ser Phe Leu Phe Gly Thr Leu
        435                 440                 445
Glu Asn Pro Pro Gln Thr Thr Ser Glu Gln Glu Thr Thr Ile Leu Asn
    450                 455                 460
Pro Glu Met Tyr Tyr Ser Tyr Cys Ser Ser Leu Tyr Glu Lys Leu Pro
465                 470                 475                 480
Pro Thr Pro Asp Ser Pro Gly Asp Gly Gly Asp Cys Thr Val Met Arg
                485                 490                 495
Val Pro Glu Ile Pro Gly Pro Leu Phe Val Asp Leu Pro Met Leu Pro
            500                 505                 510
Glu Gly Leu Val Thr Pro Glu Thr Ser Pro Ile Thr Gln Ala Met Phe
        515                 520                 525
Lys Tyr Ser Glu Gln Glu Lys Ser Glu Ile Asp Leu Leu Val Lys Gln
    530                 535                 540
Ile Gly Ser Leu Ala Asn Val Phe Asn Asn Val Ala Thr Lys Glu Phe
545                 550                 555                 560
Ser Pro Ser Asp Thr Met Leu Leu Asn Gly His Gly Leu Ala Tyr Arg
                565                 570                 575
Ser Val Ser Leu Pro Ala Cys Leu Pro Glu Thr Asp Thr Thr Ala Pro
            580                 585                 590
Phe Pro Arg Ser Trp Lys Ser Ile Asp Leu Ser Ile Phe Leu Thr Cys
        595                 600                 605
Gln Asp Pro Ser Leu Pro Ser Ser Gly His Pro Val Cys Ser Leu Phe
    610                 615                 620
Lys Asp Leu Pro Asp Ser Pro Pro Phe Ala Glu Thr Gly Ser Pro Cys
625                 630                 635                 640
Ser Ala Leu Glu Glu Asp Glu Leu Gly Gly Asp Leu Ser Ala Ser Pro
                645                 650                 655
Ser Met Thr Ser Asn Val Thr Thr Asp Leu Ser Pro Glu Glu Cys Asn
            660                 665                 670
Phe Leu Glu Glu Leu Met Ser Tyr Lys Thr Asp Leu Glu Ala Gly Ala
        675                 680                 685
Ser Glu Ile Gln Cys Asp Arg Phe Asn Asp Glu Leu Tyr Gln Leu Gln

690                 695                 700
Ile Gln Leu Gln Asp Gly Phe Gln Gln Asp Gly Ser Gly Lys Pro Ser
705                 710                 715                 720
Phe

<210> 28
<211> 537
<212> PRT
<213> Pelodiscus sinensis

<400> 28
Met Gln Thr Arg Thr Met Thr Val Phe Cys Asp His Cys Ser Arg Pro
1               5                   10                  15
Leu Arg Lys Glu Ile Ser His Pro Arg Gly Arg Ser Gly Met Glu Pro
            20                  25                  30
Ser Ala Pro Lys Pro Phe Arg Ser Thr Lys Gly Ala Ser Lys Ala Arg
        35                  40                  45
Arg Asp Gln Ile Asn Ala Glu Leu Gln Ala Leu Arg Ser Leu Leu Pro
    50                  55                  60
Ile Ser Ala Gln Glu Lys Glu Gln Leu Ser Tyr Leu Gln Thr Met Ala
65                  70                  75                  80
Leu Val Cys Leu His Ile Arg Gly Ala Gln Leu Phe Pro Ala Asp Ser
                85                  90                  95
Ser Glu Cys Ala Ala Ala Thr Val Pro His Leu Glu Leu Leu Ser Ser
            100                 105                 110
Leu Pro Gly Phe Ile Met Ala Leu Ser Ala Glu Gly Lys Leu Ala Tyr
        115                 120                 125
Ile Ser Glu Asn Val Ala His Leu Leu Gly Phe Ser Val Leu Glu Leu
    130                 135                 140
Leu Ala His Gly Asp Ser Ile Phe Asp Leu Leu Asp Gly Ser Ala His
145                 150                 155                 160
Lys Ala Val Gln Glu Lys Leu Arg Ser Ala Gln Glu His Pro Gly Thr
                165                 170                 175
Glu Ile Val Phe Val Ser Glu Met Arg Thr Ser Arg Ser Phe Arg Val
            180                 185                 190
Arg Tyr Gly Gly Asn Arg Ala Val Ala Val Arg Gly Arg Phe Val Thr
        195                 200                 205
Leu Asn Gly Met Thr Ser Ser Ser Ile Leu Thr Phe Ile Gly Phe Cys
    210                 215                 220
Thr Pro Ile Met Gln Leu Ser Asn Tyr Gly Asp Gly Ile Ser Tyr Gly
225                 230                 235                 240
Ala Pro Phe Gln Ser Gln His Thr Leu Asp Met Lys Val Thr Asp Val
                245                 250                 255
Thr Glu Ser Val Ile Tyr His Leu Gly Tyr Gln Arg Glu Glu Leu Ile
            260                 265                 270
Gly Gln Ser Trp Tyr Ser Leu Leu His Pro Glu Asp Ala Ala Ala Ala
        275                 280                 285
Ala Ala Gln His Glu Ser Leu Ile Cys Asp Thr Gly Lys Cys Asn Gln
    290                 295                 300
Lys Leu Val Val Arg Leu Leu Cys Lys Asp Val Ser Trp Thr Trp Val
305                 310                 315                 320
Gln Ile Ile Ala Ser Lys Glu Tyr Glu Arg Gly Arg Glu Ile Ile Thr
                325                 330                 335
Cys Asn Tyr Phe Cys Leu Ser Glu Glu Glu Ala Gln Tyr Leu Gln Gly
            340                 345                 350
Gln Asn Pro Trp His Gly Ala Ile Ser Ala Ala Ala Pro Ser Pro Arg
        355                 360                 365
His Tyr Ala Arg Glu Lys Gln Gln Lys Thr Cys Ser Arg Thr Leu Asn
    370                 375                 380
Ser Phe Pro Gln Glu Thr Val Met Pro Asn Gln Gly Ala Leu Asn Tyr
385                 390                 395                 400
Ser Pro Cys Gln Leu Pro Asn Ala Tyr Gln Glu Glu Leu Leu Pro Ala

                    405                     410                     415
Ser Val Gln Leu Ser Ser Thr Pro Cys Phe Pro Ala Pro Thr Val Gly
            420                     425                     430
Ala Ala Leu Pro Ser Gln Lys Thr Gly Tyr Ser Ile Leu Pro Ala Gly
        435                     440                     445
Gln Arg Asn Leu Ser Cys Pro Phe Ser Val Tyr Lys Ser Pro Cys Ser
    450                     455                     460
Gln Pro Cys Ala Leu Trp Ser Pro Glu Val Pro Leu Ser Pro Ala Ser
465                     470                     475                     480
Ser Pro Ser Val Gln Thr Cys Pro Val Pro Ala Glu Glu Pro Gly Ala
                485                     490                     495
Gly Leu Gln Cys Ala Glu Ala Glu Arg Arg Ala Ile Ser Val Leu Ala
            500                     505                     510
Glu Gln Ile Gln Ala Leu Ala Glu Arg Leu Ser Arg His Thr Lys Arg
        515                     520                     525
Ala Gly Ala Glu Pro Pro Gly Ala Pro
    530                     535

<210> 29
<211> 760
<212> PRT
<213> Python bivittatus

<400> 29
Met Tyr Arg Ser Thr Lys Gly Ala Ser Lys Ala Arg Arg Asp Gln Ile
1               5                   10                  15
Asn Ala Glu Ile Arg His Leu Lys Asp Leu Leu Pro Ile Ala Glu Gly
            20                  25                  30
Asp Lys Leu Arg Leu Ser Tyr Leu His Ile Met Ser Leu Ala Cys Ile
        35                  40                  45
Tyr Thr Arg Lys Ser Ile Phe Phe Ser Lys Gly Ala Thr Ser Glu Gly
    50                  55                  60
Ser Glu Ser Leu Leu Ser Ser Gln Asp Leu Glu Asp Phe Met Gln Thr
65                  70                  75                  80
Leu Pro Gly Phe Leu Leu Ala Phe Thr Gly Glu Gly Lys Leu Ile Tyr
                85                  90                  95
Val Ser Glu Asn Val Ala Glu His Leu Gly His Ser Val Val Asp Leu
            100                 105                 110
Val Ala Gln Gly Asp Ser Ile Tyr Asp Ile Ile Asp Pro Ala Asp His
        115                 120                 125
Phe Val Met Arg Asn Gln Leu Val Leu Pro Ser Pro Ile Asn Thr Asp
    130                 135                 140
Arg Leu Phe Arg Cys His Phe Asn Thr Ser Lys Thr Ile Arg Arg Gln
145                 150                 155                 160
Ser Ala Gly Asn Lys Leu Val Leu Ile Arg Gly Arg Phe His Gln Pro
                165                 170                 175
Pro Pro Gly Ser Tyr Trp Ser Thr Asn Pro Val Phe Thr Ala Phe Cys
            180                 185                 190
Thr Pro Leu Asp Pro Lys Pro Arg Met Asp Gln Asn Ser Phe Phe Leu
        195                 200                 205
Ser Ala Phe Glu Ser Arg His Thr Lys Asp Leu Ala Ile Val Asp Ile
    210                 215                 220
Ser Glu Ser Val Ile Phe His Leu Gly Phe Glu Lys Ser Glu Leu Phe
225                 230                 235                 240
Cys Lys Ser Trp Tyr Ser Leu Ile His Pro Glu Asp Leu Ser His Ala
                245                 250                 255
Ser Ala Gln His Tyr Arg Leu Leu Gly Asp Gln Ser Ser Met Gln Thr
            260                 265                 270
Glu Met Val Ile Arg Leu Gln Thr Lys Asp Gly Val Ser Trp Ile Trp
        275                 280                 285
Ile Tyr Ser Leu Leu Arg Ile Glu Asn Ala Glu Val Pro Ile Val Ser
    290                 295                 300
His Asn Tyr Val Ile Ser Asp Ser Glu Ala Trp Cys Leu Lys Gln Gln

305                 310                 315                 320
Val Ser Ala Glu Gly Ser Gln Val Ala Tyr Ala Leu Glu Ser Thr Thr
                325                 330                 335
Thr Thr Thr Phe Leu Glu Gly Leu Leu Ser Pro Gly Gln Leu Ser Ser
            340                 345                 350
Pro Asp Gln Val Phe Thr Pro Val Ala Gly Thr Pro Thr Ser Thr Leu
        355                 360                 365
Val Ala Pro Thr Phe Asp Phe Ser Ser Ala Gly Gly Thr Glu Gly Pro
    370                 375                 380
Ser Ala Phe Ser Glu Thr Ser Glu Ser Leu Pro Gln Glu Val Met Asp
385                 390                 395                 400
Ser Gly Asn Ile Ser Ser Ile Glu Asp Ala Pro Pro Gly Ser Thr Leu
                405                 410                 415
Ser Leu Leu Ser Lys Glu Ala Gly Phe Gly Tyr Leu Ile Leu Pro Gln
            420                 425                 430
Gly Tyr Glu Gln Thr Phe Arg Ser Glu Asn Pro Gly Ser Ser Ser Ser
        435                 440                 445
Ser Lys Asp Phe Val Cys Thr Pro Pro Tyr Thr Pro His Gln Ser Ser
    450                 455                 460
Ala Phe Leu Phe Gly Ala Gln Glu Leu Tyr Gly Pro Pro Ser Ala Pro
465                 470                 475                 480
Ala Ala Met Pro Ala Gly Thr Ala Ser Pro Gly Ala Thr Ser Ala Thr
                485                 490                 495
Pro Pro Thr Thr Ile Thr Ala Met Thr Thr Ser Glu Leu Phe Phe Pro
            500                 505                 510
Gln Glu Gln Cys Ser Val Leu Tyr Glu Lys Leu Pro Pro Thr Pro Asp
        515                 520                 525
Ser Pro Gly Asn Gly Asp Cys Thr Ile Met Thr Leu Pro Glu Ile Arg
    530                 535                 540
Gly Pro Leu Tyr Val Asp Val Pro Met Val Pro Glu Gly Ile Leu Thr
545                 550                 555                 560
Pro Glu Ala Ser Pro Ile Lys Gln Thr Phe Phe Arg Tyr Ser Glu Lys
                565                 570                 575
Glu Lys Asn Glu Ile Glu Leu Leu Ala Gln Gln Ile Cys Ser Leu Ala
            580                 585                 590
Glu Thr Phe Gly Ser Thr Ala Ser Arg Glu Leu Ala Lys Asn Gly Gln
        595                 600                 605
Val Ala Leu Arg Glu Ala Ala Pro Gly Pro Val Pro Glu Pro Ser Pro
    610                 615                 620
Val Pro Glu Pro Cys Leu Asp Phe Gln Leu Pro Lys Asn Trp Arg Ser
625                 630                 635                 640
Val Asp Phe Ser Val Leu Thr Cys Leu Glu Asp Asp Leu Leu Glu Glu
                645                 650                 655
Lys Ala Leu Glu Thr Leu Phe Gln Asp Leu Pro Ala Phe Leu Phe Glu
            660                 665                 670
Lys Gly Gly Ala Gly Met Arg Cys Pro His His Gln Phe Cys Gly Gly
        675                 680                 685
Asn Val Ser Ser Pro Ile Gly Leu Asp Thr Glu Asp Ser Ser Gln Gly
    690                 695                 700
Ala Phe Ala Pro Ser Pro Ser Thr Asp Ile Pro Pro Glu Gln Gln Cys
705                 710                 715                 720
Phe Leu Glu Glu Leu Ala Ser Phe Glu Thr Ala Phe Glu Thr Arg Ala
                725                 730                 735
Ser Asn Ser Pro Cys Asp Gly Leu Asp Glu Leu Tyr Gln Leu Gln Ser
            740                 745                 750
His Arg Gln Glu Gly Phe His Glu
        755                 760

<210> 30
<211> 817
<212> PRT
<213> Thamnophis sirtalis

<400> 30
Met Tyr Arg Ser Thr Lys Gly Ala Ser Lys Ala Arg Arg Asp Gln Ile
1               5                   10                  15
Asn Ala Glu Ile Arg His Leu Lys Asp Leu Leu Pro Ile Ala Glu Gly
            20                  25                  30
Asp Lys Leu Arg Leu Ser Tyr Leu His Ile Met Ser Leu Ala Cys Ile
        35                  40                  45
Tyr Thr Arg Lys Ser Ile Phe Phe Ser Lys Gly Ala Thr Leu Glu Gly
    50                  55                  60
Leu Glu Ser Met Leu Ser Ser Gln Asp Leu Asp Asp Phe Met Gln Thr
65                  70                  75                  80
Leu Pro Gly Phe Leu Leu Ala Phe Thr Gly Glu Gly Lys Leu Ile Tyr
                85                  90                  95
Val Ser Glu Ser Val Ala Glu His Leu Gly His Ser Met Val Asp Leu
            100                 105                 110
Val Ala Gln Gly Asp Ser Ile Tyr Asp Ile Ile Asp Pro Ala Asp His
        115                 120                 125
Ile Val Met Lys Thr Gln Leu Ala Leu Pro Ser Pro Leu Ser Thr Glu
    130                 135                 140
Arg Leu Phe Arg Cys His Phe Asn Thr Ser Lys Thr Ile Arg Arg Gln
145                 150                 155                 160
Ser Ala Gly Asn Lys Leu Ile Leu Ile Arg Gly His Phe His Gln Pro
                165                 170                 175
Pro Pro Gly Ser Tyr Trp Ser Thr Asn Pro Ile Phe Thr Ala Phe Cys
            180                 185                 190
Thr Pro Leu Asp Pro Lys Pro Arg Met Gly Gln Asn Ala Phe Phe Leu
        195                 200                 205
Ser Ala Phe Glu Ser Arg His Thr Lys Asp Leu Ala Ile Met Asp Ile
    210                 215                 220
Ser Glu Cys Val Ile Phe His Leu Gly Phe Glu Lys Thr Glu Leu Phe
225                 230                 235                 240
Cys Lys Ser Trp Tyr Gly Leu Ile His Pro Glu Asp Leu Ser His Ala
                245                 250                 255
Ser Ala Gln His Tyr Arg Leu Leu Gly Asp Gln Ser Asn Thr Gln Ala
            260                 265                 270
Glu Met Val Ile Arg Leu Gln Ala Lys Asp Gly Val Ser Trp Ile Trp
        275                 280                 285
Val Tyr Ser Leu Leu Arg Leu Glu Asn Ala Glu Ile Pro Ile Val Ser
    290                 295                 300
His Asn Tyr Val Ile Ser Asp Ser Glu Ala Trp Cys Leu Lys Gln Gln
305                 310                 315                 320
Leu Ser Ala Glu Gly Ser Gln Gly Pro Tyr Gly Leu Glu Ser Thr Thr
                325                 330                 335
Thr Phe Leu Glu Ser Leu Leu Ser Pro Gly Gln Leu Ser Ser Pro Asp
            340                 345                 350
Gln Val Phe Thr Pro Ala Ala Gly Thr Pro Thr Thr Thr Leu Leu Ala
        355                 360                 365
Ser Thr Phe Asp Phe Ser Thr Ala Gly Val Pro Glu Gly Pro Ser Thr
    370                 375                 380
Phe Ser Lys Pro Pro Glu Gly Leu Pro Glu Glu Thr Met Asp Pro Gly
385                 390                 395                 400
Asn Ile Ser Ser Ile Glu Asp Ala Pro Pro Gly Ser Ser Leu Ser Leu
                405                 410                 415
Leu Val Lys Glu Pro Gly Phe Gly Tyr Leu Ile Leu Pro Gln Gly Gly
            420                 425                 430
Tyr Gly Gln Ala Phe Ala Ser Glu Lys Pro Gly Gly Lys Pro Lys Ser
        435                 440                 445
Lys Asp Leu Val Cys Thr Pro Pro Tyr Thr Pro His Gln Ser Ser Pro
    450                 455                 460
Phe Leu Phe Gly Thr Pro Glu Leu Tyr Gly Ala Gln Gly Ala Gly Gly
465                 470                 475                 480
Pro Ala Ala Leu Pro Leu Ala Thr Ala Ser Ser Thr Asn Ser Pro Ala
                485                 490                 495

Ala Leu Ala Ala Val Thr Glu Leu Phe Tyr Ala Gln Glu Pro Cys Asn
                500                 505                 510
Val Leu Tyr Glu Lys Leu Pro Pro Thr Pro Asp Ser Pro Gly Lys Gly
        515                 520                 525
Gly Cys Ala Val Met Ser Leu Pro Glu Ile Arg Gly Pro Leu Tyr Val
    530                 535                 540
Asp Val Pro Met Val Pro Glu Gly Ile Leu Thr Pro Glu Ala Ser Pro
545                 550                 555                 560
Ile Lys Gln Thr Phe Phe Gln Tyr Ser Glu Lys Glu Lys Asn Glu Ile
                565                 570                 575
Glu Leu Leu Ala Gln Gln Ile Cys Ser Leu Ala Glu Thr Phe Gly Ser
            580                 585                 590
Ala Ala Ser Gln Glu Leu Ala Glu Asn Asp Leu Glu Gly Phe Arg Glu
        595                 600                 605
Asp Pro Pro Cys Ser Ser Pro Glu Leu Ser Pro Ala Pro Gln Pro Cys
    610                 615                 620
Pro Glu Phe Gln Leu Pro Lys Thr Trp Arg Ser Val Asp Phe Ser Leu
625                 630                 635                 640
Leu Thr Cys Leu Glu Asp Asp Leu Leu Asp Asp Ser Ala Leu Glu Ala
                645                 650                 655
Leu Leu Gln Asp Leu Ser Ser Phe Leu Phe Glu Lys Asp Gly Ala Gly
            660                 665                 670
Thr Arg Cys Pro Arg His Gln Phe Cys Gly Gly Asp Val Ser Ser Ser
        675                 680                 685
Pro Leu Gly Leu Asp Thr Glu Asp Ser Ser Ser Pro Gly Cys Phe Ala
    690                 695                 700
Pro Ser Pro Arg Met Asp Leu Ala Pro Glu Gln Pro Pro Cys Phe Leu
705                 710                 715                 720
Glu Asp Leu Thr Ser Phe Glu Thr Ala Phe Glu Thr Arg Ala Ser Asn
                725                 730                 735
Ser Pro Cys Glu Gly Leu Asp Glu Leu Tyr Gln Leu Gln Ser Gln Arg
            740                 745                 750
Pro Lys Gly Phe Gln Glu Gly Cys Arg Gly Ile Lys Arg Asp Pro Lys
        755                 760                 765
Ile Phe Lys Asp Phe Asp Leu Val Asp Ala Asn Ala Thr Phe His Asp
    770                 775                 780
Asp Ala Leu Lys Asp Asp Tyr Phe Cys Phe Leu Leu Ile Lys Val Arg
785                 790                 795                 800
Pro Ile Phe His Thr Glu Lys Asp Lys Thr Pro Lys Thr Phe Phe Glu
                805                 810                 815
Thr


<210> 31
<211> 579
<212> PRT
<213> Haliaeetus leucocephalus

<400> 31
Met Leu Arg Ser Thr Lys Gly Ala Ser Lys Ala Arg Arg Asp Gln Ile
1               5                   10                  15
Asn Ala Glu Ile Arg Ala Leu Arg Asp Leu Leu Pro Leu Pro Glu Gly
            20                  25                  30
Ala Arg Pro Arg Leu Ser Tyr Leu His Val Met Ala Leu Ala Cys Ile
        35                  40                  45
Tyr Thr Arg Lys Gly Ala Cys Leu Arg Pro Gly Pro Ala Arg Gly Ala
    50                  55                  60
Pro Met Glu Leu Leu Gly Gly Pro Glu Leu Ala Asp Leu Val Ala Ser
65                  70                  75                  80
Leu Pro Gly Phe Leu Leu Ala Val Thr Arg Glu Gly Lys Leu Val Gly
                85                  90                  95
Val Thr Asp Asn Val Ala Gln His Leu Gly His Ser Met Val Asp Leu
            100                 105                 110

Val Ala Gln Gly Asp Ser Ile Tyr Asp Leu Leu Asp Pro Ala Asp His
            115                 120                 125
Pro Leu Val Arg His Gln Leu Thr Leu Pro Gly Pro Pro Gln Ala Glu
    130                 135                 140
Arg Leu Phe Arg Cys Arg Phe Thr Thr Ser Arg Ala Ser Arg Arg Pro
145                 150                 155                 160
Ser Ala Gly Arg Lys Leu Val Leu Leu Arg Gly Arg Phe Gln Ala Pro
                165                 170                 175
Pro Gly Pro Pro Gly Ala Ser Pro Gly Leu Phe Val Ala Phe Cys Ala
            180                 185                 190
Pro Leu Asp Pro Pro Pro Trp Pro Cys Pro Asp Cys Leu Leu Leu Pro
        195                 200                 205
Ala Phe Gln Ser Arg His Ala Arg Asp Leu Ala Leu Leu Asp Ile Ser
    210                 215                 220
Asp Ser Val Leu Ile His Leu Gly Tyr Gly Arg Gly Glu Leu Leu Gly
225                 230                 235                 240
Arg Ser Trp Tyr Arg Leu Leu His Pro Glu Asp Leu Gly His Val Ala
                245                 250                 255
Arg Gln His Leu Arg Leu Ala Gly Ala Gly Ala Glu Ala Arg Gly Glu
            260                 265                 270
Leu Val Thr Arg Leu Gln Arg Lys Asp Gly Leu Gly Trp Thr Trp Val
        275                 280                 285
Tyr Ala Arg Leu Arg Pro Glu Gly Pro Ala Leu Leu Ala His Asn Phe
    290                 295                 300
Val Ile Ser Glu Ala Glu Ala Trp Cys Leu Arg Gln Gln Leu Ala Ala
305                 310                 315                 320
Glu Ala Pro Pro Gly Pro Pro Glu Pro Phe Gly Pro Ser Leu Asp Phe
                325                 330                 335
Pro Gly Ala Ala Ile Gly Ala Gly Gly Asp Leu Gly Val Gly Ala Asp
            340                 345                 350
Pro Ser Ile Gly Leu Ser Val Gly Pro Gly Ile Gly Ser Ser Val Asp
        355                 360                 365
Leu Gly Val Gly Ser Gly Leu Gly Asp Asp Ile Gly Ala Asn Val Gly
    370                 375                 380
Val Asn Val Gly Leu Gly Leu Gly Val Asn Val Gly Cys Gly Leu Gly
385                 390                 395                 400
Ala Asp Val Gly His Gly Leu Gly Ala Gly Leu Gly Ala Thr Val Asp
                405                 410                 415
Pro Ala Val Gly Ala Gly Val Gly Pro Gly Leu Gly Ala Asn Met Gly
            420                 425                 430
Pro Gly Leu Asp Ala Asn Met Gly Pro Ala Leu Gly Ala Ala Ile Gly
        435                 440                 445
His Gly Leu Gly Gly Asn Val Gly Ala Asn Ile Asp Pro Ala Leu Glu
    450                 455                 460
Leu Ala Leu Ala Trp Glu Leu Ala Ser Glu Ser Thr Leu Ala Ser Val
465                 470                 475                 480
Trp Gln Leu Ala Pro Ala Pro Met Leu Ala Pro Thr Leu Gly Leu Ile
                485                 490                 495
Leu Glu Leu Leu Leu Glu Leu Ala Pro Glu Met Ile Leu Glu Leu Val
            500                 505                 510
Ser Lys Pro Met Val Glu Arg Thr Leu Ala Pro Val Ser Glu Pro Ala
        515                 520                 525
Leu Arg Ser Val Ser Gly Pro Thr Leu Gly Leu Val Leu Val Ala Val
    530                 535                 540
Ser Glu Pro Val Leu Gly Ser Ala Leu Gly Leu Leu Val Leu Leu Phe
545                 550                 555                 560
His Leu Met Ser Ala Leu Ala Trp Asp Ser Pro Ser Val Pro Ala Leu
                565                 570                 575
Glu Pro Ile

<210> 32
<211> 1938

<212> DNA
<213> Danio rerio

<220>
<223> npas4l

<400> 32
agctgcggag actcgggcat caggaggatc ctccgcgcgt ccaagcgctt caggtccacc 60

aaaggagcat ctaaggcgcg gcgtgatcag atgaacagtg agatacggaa cctgcgagcg 120

ctgctgccca tcagcccaga gcacagactc tcatacctgc acagcatgag catcacctgc 180

acctacatca ggaagagtgt ggagctgaga ggtgtgtgtg aggaatccac agttttttct 240

gccgtgaatg gatgtgtccc gcaggactgt gctctacagg actgtgctct acaggagtgt 300

gttctgcagg agtgtgttct gcaggagtgt gttctgcagg agtgtgttct gcaggctctg 360

ccggggttta tcgttgcctt taccacagat ggaaaactgc tgtatgtgtc tgagaacgta 420

cacgagtatc tgggtctgtc catggtggac gttctccaga gcgactcttt cttcgatatg 480

ctggaccgca gtgatgtgga ggcggtgagg agtgtgttag ctgacgccag tccttcagga 540

gagcggtggg ttgtgtgtcg tatgctggtg tctaaagcga tgcgtctgcg gagctcctgc 600

tgccccctgc tggtgcggat ccgtctgcgg gacggagtgt gtgtgtcgct ctgcagaccc 660

accgctgacc ggctgccggc gcgcaacgct gacttccaca cacaccacag cgcagacatg 720

aggctcgcca gcgcatcctc cagtgtgttg ttccacctgg gcttctcagc ggacgagctg 780

attggtcgat cctggtacga gctcctgcac cccgacgacc tccgacacgc tgctgaccgc 840

cacgccgcaa tccttgcagc cgccacagca gacgcagaga tgctgatccg ggttcagtgt 900

aaggatctgt cctgggtgtg gatgtacaca cacgcatcag caacagcaga gcgggacgcg 960

atcagctgct ccaactacat gatcagtgaa gcagaggcag tgtatctgca gcagagactc 1020

tcctcctcct cctcctcctc cacctcctcc tcctcctcct ctgcctctcc tcagtgtagc 1080

tccgtctcgg acagcagtga tgcccacagc gcacacacac tcttctgcac gccgccctac 1140

agccccactt cctctcagtg ctctgacttc ctgtctgaag gttatggctc tctggaggcg 1200

ctggtggatt ctgccttctg ctctccgccg tacccgccgc tgttccccaa cccatgctgc 1260

acccccactg tctgcccacc ggacactgcc ctggaccccg ctcggctctg cccgccagat 1320

tctgccctgg tccccgctgc gctctgtccg ccggacgctg ccctggaccc cactcagctc 1380

tgcccgccgg gagctgccct ggaccccact ctgctctgcc cgccgggagc tgccctggac 1440

cccactcagc tctgcctgcc ggacggtgcc ttggtccctg ctgtgttctg cccgccgaac 1500

ggtgccctgg tccctgcccg gctctgcccg tcagatgctg ccctggtccc cgctgcactc 1560

ggtcggccgg acgctgccct ggtgcctgtg tgtcggccac tgcaggtgtg tgagtgccct 1620

ctggatggcg ctgtgccgct ggaggacctc agcatgttcc cgctgccgca gggcggaggc 1680

tctcgtctga tgccgcctga agcatcaccc actacccaca cacacttctc gtatgatgca 1740

gcacagcagg cggggatcgg cacgttggca atgcagatac acacactcat ctgcagcttc 1800

gatgcgtaca gcgccgcacg acacacacac tctcaccaca cctcctgctg ggcaccagag 1860

ccactgctgg acgagggcat catcgacagc atcctcagag agctggacag cacacacact 1920

gatgcacaca cactgatc 1938

<210> 33
<211> 4317
<212> DNA
<213> Danio rerio

<220>
<223> npas4l complete cDNA

<400> 33
acacactctc tctcccagac acacaaacac acacacaccg cagagataag agctccagcg 60

ccgctgactg accgccgata gccgaccctg acccgctgcg cccggctgat gctgcgcccg 120

gagaagttcc tgatcatctg tgtgtgcgtg aggagtgtgt tcatcagcag cgtcgagtgt 180

gttctcctca tcctccgtgt ctaaggagtg tgtgtgtgtg tgagatgagc tgcggagact 240

cgggcatcag gaggatcctc cgcgcgtcca agcgcttcag gtccaccaaa ggagcatcta 300

aggcgcggcg tgatcagatg aacagtgaga tacggaacct gcgagcgctg ctgcccatca 360

gcccagagca cagactctca tacctgcaca gcatgagcat cacctgcacc tacatcagga 420

agagtgtgga gctgagaggt gtgtgtgagg aatccacagt tttttctgcc gtgaatggat 480

gtgtcccgca ggactgtgct ctacaggact gtgctctaca ggagtgtgtt ctgcaggagt 540

gtgttctgca ggagtgtgtt ctgcaggagt gtgttctgca ggctctgccg gggtttatcg 600

ttgcctttac cacagatgga aaactgctgt atgtgtctga gaacgtacac gagtatctgg 660

gtctgtccat ggtggacgtt ctccagagcg actctttctt cgatatgctg gaccgcagtg 720

atgtggaggc ggtgaggagt gtgttagctg acgccagtcc ttcaggagag cggtgggttg 780

tgtgtcgtat gctggtgtct aaagcgatgc gtctgcggag ctcctgctgc cccctgctgg 840

tgcggatccg tctgcgggac ggagtgtgtg tgtcgctctg cagacccacc gctgaccggc 900

tgccggcgcg caacgctgac ttccacacac accacagcgc agacatgagg ctcgccagcg 960

catcctccag tgtgttgttc cacctgggct tctcagcgga cgagctgatt ggtcgatcct 1020

ggtacgagct cctgcacccc gacgacctcc gacacgctgc tgaccgccac gccgcaatcc 1080

ttgcagccgc cacagcagac gcagagatgc tgatccgggt tcagtgtaag gatctgtcct 1140

gggtgtggat gtacacacac gcatcagcaa cagcagagcg ggacgcgatc agctgctcca 1200

actacatgat cagtgaagca gaggcagtgt atctgcagca gagactctcc tcctcctcct 1260

cctcctccac ctcctcctcc tcctcctctg cctctcctca gtgtagctcc gtctcggaca 1320

gcagtgatgc ccacagcgca cacacactct tctgcacgcc gccctacagc cccacttcct 1380

ctcagtgctc tgacttcctg tctgaaggtt atggctctct ggaggcgctg gtggattctg 1440

ccttctgctc tccgccgtac ccgccgctgt tccccaaccc atgctgcacc cccactgtct 1500

gcccaccgga cactgccctg gaccccgctc ggctctgccc gccagattct gccctggtcc 1560

ccgctgcgct ctgtccgccg gacgctgccc tggaccccac tcagctctgc ccgccgggag 1620

ctgccctgga ccccactctg ctctgcccgc cgggagctgc cctggacccc actcagctct 1680

gcctgccgga cggtgccttg gtccctgctg tgttctgccc gccgaacggt gccctggtcc 1740

ctgcccggct ctgcccgtca gatgctgccc tggtccccgc tgcactcggt cggccggacg 1800

ctgccctggt gcctgtgtgt cggccactgc aggtgtgtga gtgccctctg gatggcgctg 1860

tgccgctgga ggacctcagc atgttcccgc tgccgcaggg cggaggctct cgtctgatgc 1920

cgcctgaagc atcacccact acccacacac acttctcgta tgatgcagca cagcaggcgg 1980

ggatcggcac gttggcaatg cagatacaca cactcatctg cagcttcgat gcgtacagcg 2040

ccgcacgaca cacacactct caccacacct cctgctgggc accagagcca ctgctggacg 2100

agggcatcat cgacagcatc ctcagagagc tggacagcac acacactgat gcacacacac 2160

tgatctagtg cacacacact gatgcacaca cactgatgca cacacactga cacacacaca 2220

cacacacaca aacacacact ggaggaactt tgactgatgt gtgcttatga acatgaatgt 2280

tgatttctgc agtcgtgtgt gtgtgtgtgt gtgtgtgtgt gtgtgtgtaa actagggatg 2340

cactgatcct attacttgga tcggatatcg gttcaataca gcatagtttt gctggaacgg 2400

gtatcagcca gctggtactg atccatatct ggtcgtgcgt gtgcgctata ttctgtgttt 2460

gtgagccagc agtagtcagg aagcagccga tggtaaggct ctagccagtt gtcctgtagg 2520

ctgctgtatc ccacatgtgc tgaagccgtt gtacagtgta atgtgcagca caggtgaagg 2580

ttcacctggt catctcatgt tcagttcagc gctttcctcc gctgctcctg tcaatcattc 2640

tccactcatt cacaactcaa actgcgtgtg gcgttcatga ccgcacgaac aactctccaa 2700

cacgattagt tctgttaata tcagtaatgg gtggagagac gcattcagtt ctgcattcac 2760

tggcttcatt ttgtcctgca gcgtggagat tgcgtcgctc actgcagaag tttttcaagt 2820

gtcgacgaaa gcgtcagcca ataagatcgc tgtatgcaaa tacaccagct cagacagtgg 2880

ctgattgctg actaatttca ttggctgatg ctgctatgac aatcgcgtca gcctcaactt 2940

cagacacgcc cactgtcaag cgctgaagct ccgtgtgaat gtggcacgcc cccttgtggc 3000

agtcgcacag aattttcagt tctgacgtgc accatgctaa tctcacagtg tctgaaataa 3060

aggagtgcaa agctagagcc atcattctct gcgttcacag acagattcag aatgaatgaa 3120

tactagaaac tcatagatcg ctgtaaacat tggagggaat gtggagacaa taatacaaat 3180

acatattatt attactttat cgcttgcatt tatgaacaga aatgttgtta tgtttgataa 3240

tttagacttt ttcagattta aaaaataacc aaaaactcat atttctcatc ccgtgcgcac 3300

gaacctgctg gtacaacact ggaatacgtc acctccggtc ggccggtcgc atatggccta 3360

gtcgcaggag ttcaaattat tcaacggatc cgcagctcag ttctgatcca aattttcctc 3420

atacggagat gatcagaaca ttcgcagcac tgggtcatca cctgctcttc acacacactg 3480

taggcctatt cctgaaagtt taaataagaa acggctcaac agcacgctgg acgggtcctc 3540

cacacactga tctctcccct ttctgctgct gctggagcgt ctgctgagag cggagagctg 3600

cgcgccgtct cagtgatcat caagcgcaga cgtgacgcgc ggcgctctgt aagagtattc 3660

tgatgaagag tttctgttct ctcatccttc tgcctgagtt tattgtgtgg aggtgaacat 3720

gctcagcgct gatgatagtg tgtaaagccc ggctcactgt gctcaaagta ctgactacat 3780

taataaaaga gaagcgtcat gttttagtca gacgctgtcg tctgtcttac acagtaggcc 3840

catacgcctg ttattttgat gaaggaggat ctgttattcc agcaccagaa ccatagaaac 3900

tgcattaggt ttaaaaaaca aacagcacag tatcgggatc agatctgtat tgatcgagac 3960

acagaattgt agtatcagga tcggttccaa aaaatggtct cagtgcatcc ccagtgtacc 4020

gggtcaataa tgagcatata tatgcactta ctgtactgat gctcctctgc agagcgggag 4080

ccggctcctc ctgatctggt gtgtgtgtgt gtgtgtgtgt gtgtgtgtgt gccctgtggt 4140

tcctctggat ctctcagcac agttgtctgt atctcctctg tcctcctcct cagtgttggt 4200

cagcgatgct cgctgcagtg tgtatagttc atatattcct gatgcttctg tttatttttg 4260

tatgaactct taataaagtt atgatcaaaa aaaaaaaaaa aaaaaaaaaa aaaaaaa 4317

<210> 34
<211> 23
<212> DNA
<213> Artificial Sequence

<220>
<223> npas4l ATG MO

<400> 34
gagtctccgc agctcatctc aca 23

<210> 35
<211> 25
<212> DNA
<213> Artificial Sequence

<220>
<223> npas4l I2E3 MO

<400> 35
cacctggaac acacagtgga ggatt 25

<210> 36
<211> 25
<212> DNA
<213> Artificial Sequence

<220>
<223> tal1 MO

<400> 36
aatgctctta ccatcgttga tttca                                          25


<210> 37
<211> 25
<212> DNA
<213> Artificial Sequence

<220>
<223> etv2 MO1

<400> 37
ttggtacatt tccatatctt aaagt                                          25


<210> 38
<211> 25
<212> DNA
<213> Artificial Sequence

<220>
<223> etv2 MO2

<400> 38
cactgagtcc ttatttcact atatc                                          25


<210> 39
<211> 20
<212> DNA
<213> Artificial Sequence

<220>
<223> z709 Forward 5'-3'

<400> 39
cccgtctaca acactgtgct                                                20


<210> 40
<211> 20
<212> DNA
<213> Artificial Sequence

<220>
<223> z709 Reverse 5'-3'

<400> 40
atctctgcgt tctgattggc                                                20


<210> 41
<211> 20
<212> DNA

<213> Artificial Sequence

<220>
<223> z1496 Forward 5'-3'

<400> 41
acgacaggcg gagaaagata                                                    20


<210> 42
<211> 20
<212> DNA
<213> Artificial Sequence

<220>
<223> z1496 Reverse 5'-3'

<400> 42
ggcgctcctt cagtttacag                                                    20


<210> 43
<211> 20
<212> DNA
<213> Artificial Sequence

<220>
<223> z8617 Forward 5'-3'

<400> 43
gagcctgcgg tcatctacag                                                    20


<210> 44
<211> 20
<212> DNA
<213> Artificial Sequence

<220>
<223> z8617 Reverse 5'-3'

<400> 44
ccgcttctgt tttattccca                                                    20


<210> 45
<211> 20
<212> DNA
<213> Artificial Sequence

<220>
<223> z60320 Forward 5'-3'

<400> 45
gacccagcat gaacacactg                                                    20


<210> 46
<211> 20
<212> DNA
<213> Artificial Sequence

<220>

<223> z60320 Reverse 5′-3′

<400> 46
cgagagcttt ccagccatat                                                    20


<210> 47
<211> 23
<212> DNA
<213> Artificial Sequence

<220>
<223> klhdc3 sslp Forward 5′-3′

<400> 47
acatcacact gctccacatt cac                                                23


<210> 48
<211> 23
<212> DNA
<213> Artificial Sequence

<220>
<223> klhdc3 sslp Reverse 5′-3′

<400> 48
atgaagagat gggaatgctc aaa                                                23


<210> 49
<211> 22
<212> DNA
<213> Artificial Sequence

<220>
<223> z1412 Forward 5′-3′

<400> 49
tcaaactgcg gctctgttta ca                                                 22


<210> 50
<211> 22
<212> DNA
<213> Artificial Sequence

<220>
<223> z1412 Reverse 5′-3′

<400> 50
aagtgcttta gctggacgag gc                                                 22


<210> 51
<211> 22
<212> DNA
<213> Artificial Sequence

<220>
<223> z1826 Forward 5′-3′

<400> 51

cgccactgta acgccactat aa                                                22

<210> 52
<211> 22
<212> DNA
<213> Artificial Sequence

<220>
<223> z1826 Reverse 5'-3'

<400> 52
ttgaagactc cagagccaca aa                                                22

<210> 53
<211> 20
<212> DNA
<213> Artificial Sequence

<220>
<223> z4252 Forward 5'-3'

<400> 53
tgtgcgtttg cagtgctctg                                                   20

<210> 54
<211> 20
<212> DNA
<213> Artificial Sequence

<220>
<223> z4252 Reverse 5'-3'

<400> 54
ggtactgctg ccgctggact                                                   20

<210> 55
<211> 20
<212> DNA
<213> Artificial Sequence

<220>
<223> z25644 Forward 5'-3'

<400> 55
atgtgtgtgt gcacggattt                                                   20

<210> 56
<211> 20
<212> DNA
<213> Artificial Sequence

<220>
<223> z25644 Reverse 5'-3'

<400> 56
gctgctcaga gcgtctgaat                                                   20

<210> 57
<211> 20
<212> DNA
<213> Artificial Sequence

<220>
<223> z26667 Forward 5'-3'

<400> 57
gcttcacatg cttgtgtcgt                                                    20


<210> 58
<211> 20
<212> DNA
<213> Artificial Sequence

<220>
<223> z26667 Reverse 5'-3'

<400> 58
tggagcattt acaaacgctg                                                    20


<210> 59
<211> 20
<212> DNA
<213> Artificial Sequence

<220>
<223> z99040 Forward 5'-3'

<400> 59
aaggcaggtg acgagtccta                                                    20


<210> 60
<211> 20
<212> DNA
<213> Artificial Sequence

<220>
<223> z99040 Reverse 5'-3'

<400> 60
ctggagctca gtcctctgct                                                    20


<210> 61
<211> 22
<212> DNA
<213> Artificial Sequence

<220>
<223> tmem121 sslp Forward 5'-3'

<400> 61
ctcagatctg caggtgttct cc                                                 22


<210> 62
<211> 22
<212> DNA

<213> Artificial Sequence

<220>
<223> tmem121 sslp Reverse 5'-3'

<400> 62
agcgttcctc atattcctga tg                                                22


<210> 63
<211> 22
<212> DNA
<213> Artificial Sequence

<220>
<223> z1496 F

<400> 63
cctgagcttc atccagagct cc                                                22


<210> 64
<211> 22
<212> DNA
<213> Artificial Sequence

<220>
<223> z1496 R

<400> 64
cttcagttta cagcagcgct cg                                                22


<210> 65
<211> 20
<212> DNA
<213> Artificial Sequence

<220>
<223> Z1452 F

<400> 65
cagattagct ttggcttcgg                                                   20


<210> 66
<211> 22
<212> DNA
<213> Artificial Sequence

<220>
<223> Z1452 R

<400> 66
   gttttaattc ccctccaact cc                                                 22


<210> 67
<211> 43
<212> DNA
<213> Artificial Sequence

<220>

<223> OAS1323

<400> 67
gattacgcca agctttgtga gtgccctctg gatggcgctg tgc                43


<210> 68
<211> 41
<212> DNA
<213> Artificial Sequence

<220>
<223> OAS1324

<400> 68
gattacgcca agcttggacc tcagcatgtt cccgctgccg c                  41


<210> 69
<211> 43
<212> DNA
<213> Artificial Sequence

<220>
<223> OAS1325

<400> 69
gattacgcca agcttgcggc agcgggaaca tgctgaggtc ctc                43


<210> 70
<211> 42
<212> DNA
<213> Artificial Sequence

<220>
<223> OAS1326

<400> 70
gattacgcca agcttgcaca gcgccatcca gagggcactc ac                 42


<210> 71
<211> 52
<212> DNA
<213> Artificial Sequence

<220>
<223> OAS1343

<400> 71
acttgttctt tttgcaggat ccaccatgag ctgcggagac tcgggcatca gg      52


<210> 72
<211> 42
<212> DNA
<213> Artificial Sequence

<220>
<223> OAS1344

<400> 72

tacgactcac tatagttcta gatcagtgtg tgtgcatcag tg                42

<210> 73
<211> 27
<212> DNA
<213> Artificial Sequence

<220>
<223> OAS1438

<400> 73
ctttaccaca gatggaaaac tgctgta                                    27

<210> 74
<211> 22
<212> DNA
<213> Artificial Sequence

<220>
<223> OAS1439

<400> 74
actcgtgtac gttctcagac ac                                         22

<210> 75
<211> 21
<212> DNA
<213> Artificial Sequence

<220>
<223> SR1470

<400> 75
gcacaaaggt catgttctca c                                          21

<210> 76
<211> 21
<212> DNA
<213> Artificial Sequence

<220>
<223> SR1471

<400> 76
cgaggttctg gtaggtttga g                                          21

<210> 77
<211> 21
<212> DNA
<213> Artificial Sequence

<220>
<223> SR1472

<400> 77
gaaatcaacg atggttcgca g                                          21

<210> 78
<211> 20
<212> DNA
<213> Artificial Sequence

<220>
<223> SR1473

<400> 78
gagtttcttg tcaggagggt                                                    20


<210> 79
<211> 23
<212> DNA
<213> Artificial Sequence

<220>
<223> SR1468

<400> 79
agaaacctgc caagtatgat gag                                                23


<210> 80
<211> 20
<212> DNA
<213> Artificial Sequence

<220>
<223> SR1469

<400> 80
taccatgtga ccagcttgac                                                    20


<210> 81
<211> 22
<212> DNA
<213> Artificial Sequence

<220>
<223> SR1474

<400> 81
aaactacaca atgtcaacag gc                                                 22


<210> 82
<211> 21
<212> DNA
<213> Artificial Sequence

<220>
<223> SR1475

<400> 82
agagaatacg ctcctacatg c                                                  21


<210> 83
<211> 21
<212> DNA

<213> Artificial Sequence

<220>
<223> SR1447

<400> 83
ctacaggatt gtgctctaca g                                                21


<210> 84
<211> 22
<212> DNA
<213> Artificial Sequence

<220>
<223> SR1448

<400> 84
gtacgttctc agacacatac ag                                               22


<210> 85
<211> 21
<212> DNA
<213> Artificial Sequence

<220>
<223> SR1494

<400> 85
cagatgaaca gtgagatacg g                                                21


<210> 86
<211> 20
<212> DNA
<213> Artificial Sequence

<220>
<223> SR1495

<400> 86
gtaaaggcaa cgataaaccc                                                  20


<210> 87
<211> 20
<212> DNA
<213> Artificial Sequence

<220>
<223> SR1496

<400> 87
cctagcaatc gagtcaagcg                                                  20


<210> 88
<211> 18
<212> DNA
<213> Artificial Sequence

<220>

<223> SR1497

<400> 88
aagctctctg ggctggcg                                                    18

## Description

The present invention relates to a method for producing endothelial cells from progenitor cells, said method comprising inducing NPAS4 function in the progenitor cells. The present invention also encompasses a method for producing hematopoietic cells from progenitor cells, said method comprising inducing NPAS4 function in the progenitor cells. The present invention furthermore pertains to a method for producing cardiomyocytes from progenitor cells, said method comprising inhibiting NPAS4 function in the progenitor cells.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

In the course of cellular differentiation within organisms pluripotent stem cells undergo further specialization into multipotent progenitor cells that then give rise to specialized cell types. Each specialized cell type is generated from the pluripotent stem cells/ multipotent progenitor cells by the expression of a specific subset of genes of the genome of these organisms. Cell differentiation is thus a transition of a cell from one cell type to another cell type which transition involves a switch from one pattern of gene expression to another pattern of gene expression. The genes involved in cellular differentiation are the subject of scientific investigations since decades, because knowing these genes provides *inter alia* the basis for artificially establishing differentiated cell types from progenitor cells.

Thereby generated differentiated cell types are in particular useful for cell replacement therapy. In contrast to specialized cells pluripotent stem cells/ multipotent progenitor cells can be maintained and expanded as pure populations of undifferentiated cells for extended periods of time, possibly indefinitely, in culture. An unlimited source of cells and tissues for transplantation for the treatment of a broad spectrum of diseases becomes thereby available.

Studies during the past decades have led to the development of culture conditions and protocols for the generation of a broad spectrum of specialized cell types from pluripotent stem cells/ multipotent progenitor cells. For example, culture conditions and protocols for the generation of mesoderm-derived cells, including hematopoietic, vascular, and cardiac cells are available. However, still many genes being involved *in vivo* in the generation of differentiated cell types from progenitor cells are unknown. Because the identification of each further gene being involved in cellular differentiation provides new or better possibilities of generating differentiated cell types from progenitor cells there is an ongoing need for the identification of further genes that may be used or targeted in culture conditions and protocols for the generation of specialized cell types. This need is addressed by the present invention.

Accordingly the present invention relates in a first aspect to a method for producing endothelial cells from progenitor cells, said method comprising inducing NPAS4 function in the progenitor cells.

In a second aspect the present invention relates to a method for producing hematopoietic cells from progenitor cells, said method comprising inducing NPAS4 function in the progenitor cells.

The term "progenitor (or precursor) cell" as used herein refers to any cell having the capacity of differentiating into the desired specific type of cell. Thus, a progenitor cell specifies a cell which is partially or fully undifferentiated. In connection with the first aspect of the invention the progenitor cell has the capacity of differentiating into an endothelial cell. In connection with the second aspect of the invention the progenitor cell has the capacity of differentiating into a hematopoietic cell. A particular example of a progenitor cell is a stem cell. In general, stem cells can inter alia be distinguished from other progenitor cells in that stem cells can replicate indefinitely, whereas other progenitor cells can divide only a limited number of times.

The progenitor cell is preferably a vertebrate cell, more preferably an amniote cell, and most preferably a mammalian cell. Non-limiting examples of mammals are cat, dog, horse, cattle, swine, goat, sheep, mouse, rat, monkey, ape and human. Within the order of mammals human is most preferred.

Endothelial cells line the interior surface of blood vessels and lymphatic vessels. They thereby form an interface between circulating blood or lymph in the lumen and the rest of the vessel wall. Accordingly, endothelial cells have a barrier function. They are moreover involved in blood clotting, blood vessel formation, inflammation, blood pressure control and tissue repair. Endothelial cells in direct contact with blood are called vascular endothelial cells, whereas those in direct contact with lymph are known as lymphatic endothelial cells. Endothelial cells are of mesodermal origin. It is to be understood that in connection with the present invention the term "endothelial cells" refers to cells having essentially a mature endothelial cell phenotype. As is well-known in the art, cell differentiation methods in general do not result in cells which are identical to the corresponding *in vivo* differentiated cell-type. For instance, certain differences as regards the gene expression profile or even slight phenotypic differences may be observed as long as the artificially differentiated cell-type can *in vitro* and/or *in vivo* substitute the functions of *in vivo* differentiated cell-type. Cell differentiation methods generally aim at generating cells resembling the corresponding *in vivo* cell-type as closely as possible.

Hematopoietic cells (also called blood cells or hematocytes) commonly designate the cell types, which can be found in the blood of vertebrates. These cell types fall into the three general categories of erythrocytes, leukocytes and thrombocytes. Hematopoietic cells can also be divided into the myeloid and lymphoid lineage. Myeloid cells include monocytes, macrophages, neutrophils, basophils, eosinophils, erythrocytes, dendritic cells, megakaryocytes and platelets. The hematopoietic cells of the invention are preferably myeloid cells. Lymphoid cells include T cells, B cells, and natural killer cells. Also hematopoietic cells are of mesodermal origin. More recent studies have revealed that a subset of endothelial cells, otherwise known as hemogenic endothelium, can give rise to hematopoietic stem cells (HSCs) that eventually generates the complete range of adult blood cells (Hirschi (2012), Blood 119, 4823-7 and Ciau-Uitz et al (2014), Exp Hematol. 42, 669-83). For the reasons discussed herein above, in connection with the present invention the term "hematopoietic cells" refers to cells having essentially a mature hematopoietic cell phenotype, which can *in vitro* and/or *in vivo* substitute the functions of *in vivo* differentiated hematopoietic cells.

Neuronal PAS domain protein 4 (NPAS4 and also known as NXF; Le-PAS; PASD10 or bHLHe79) is a member of the basic helix-loop-helix-PER (MIM 602260)-ARNT (MIM 126110)-SIM (see SIM2; MIM 600892) (bHLH-PAS) class of transcriptional regulators. This class of transcriptional regulators is involved in a wide range of physiologic and developmental events. NPAS4 contains in its N-terminal part a bHLH domain, followed by two PAS domains (PAS1 and PAS2) and a C-terminal activation domain. The amino acid sequence of human NPAS4 is shown in SEQ ID NO: 1 whereas SEQ ID NOs 2 to 31 are orthologs of human NPAS4. In more detail, SEQ ID NOs 2 to 9 are orthologs of human NPAS4 from other primates, including apes, old word monkeys, new world monkeys, and a prosimian. SEQ ID NOs 10 to 14 are orthologs of human NPAS4 from rodentia, including mouse and rat. SEQ ID NOs 15 to 24 are orthologs of human NPAS4 from mammals other than primates and rodentia, including evolutionary distinct mammalian species, such as Opossum or Tasmanian devil. SEQ ID NOs 25 to 31 are orthologs of human NPAS4 from vertebrates other than mammals, including a bird, reptiles and fish. It is of note that the ortholog of human NPAS4 from zebrafish (SEQ ID NO: 25) was not known from the prior art but has been isolated and sequenced in connection with the present invention; see examples herein below.

As used herein the term "inducing NPAS4 function" designates the induction of the expression of the gene tal1 and/or the gene etv2. Species orthologs of zebrafish tal1 and etv2 can be found throughout vertebrates and the corresponding nucleotide sequences and encoded amino acid sequences are available from genome databases, such as ensemble or NCBI. The induction of the expression of the gene tal1 and/or the gene etv2 may either mean that before the induction of NPAS4 function no expression of these genes is observed or that upon the induction of NPAS4 function the expression of the gene tal1 and/or the gene etv2 is enhanced. Enhanced expression means with increasing preference that gene expression is enhanced at least 2-fold, at least 3-fold, at least 4-fold and at least 5-fold as compared to corresponding conditions in the absence of the induction of NPAS4 function. As is evident from the examples herein below, in the course of the differentiation of endothelial cells and hematopoietic cells in the embryogenesis of zebrafish the npas4-like (or napas4I) gene is expressed upstream of the expression of tal1 and etv2. In the absence of npas4-like no tal1 and etv2 expression is observed. Moreover, in the zebrafish cloche mutant (Stainier et al., 1995, Development 121, 3141-3150), the expression of tal1 and etv2 can be restored by the human npas4 gene or the zebrafish ortholog npas4-like. Accordingly, the examples also provide means and methods for determining whether the gene tal1 and/or the gene etv2 is/are expressed or not, which means and method may be employed to check whether an NPAS4 function has been induced.

The gene etv2 encodes the transcription factor ets variant 2 (ETV2 and is also know as Etsrp71). Mouse knockouts of etv2 are embryonic lethal, said lethal embryos lacking both hematopoietic and endothelial lineages (Rasmussen (2013), Genesis, 51(7):471-80). ETV2 is furthermore known to govern endothelial development by regulating downstream transcriptional networks (Behrens et al. (2014), Stem Cell Dev., 23(17):2004-13). For this reason, the term "inducing NPAS4 function" in connection with the first aspect of the invention more preferably designates the induction of the expression of the gene etv2 and most preferably of both genes etv2 and tal1.

The gene tal1 encodes the T-cell acute lymphocytic leukemia protein 1 (TAL1 and is also know as SCL, TCL5 or bHLHa17). Tal1 is a transcriptionally complex gene that is expressed throughout development. The encoded helix-loop-helix (HLH) transcription factor TAL1 is expressed in hematopoietic and endothelial progenitor cells (Hosur et al. (2013), PLoS One 8: e53426). The disruption of the tal1 gene in mice demonstrates that tal1 is essential for embryonic blood formation *in vivo.* Further loss-of-function studies show that tal1 is inter alia essential for the formation of hematopoietic stem cells, subsequent erythroid development, and yolk sac angiogenesis. For this reason, the term "inducing NPAS4 function" in connection with the second aspect of the invention more preferably designates the induction of the expression of the gene tal1 and most preferably of both genes tal1 and etv2.

It is to be understood that in the above-described methods in general one or more differentiation factors are used. The term "differentiation factor" in accordance with the invention generally specifies any chemicals and compounds, such as proteins, nucleic acid molecules or small organic compounds, which when contacted with the progenitor cells leads to the desired more differentiated cellular stage, either when used alone or in combination with other differentiation factor(s). A preferred example of such a differentiation factor is a transcription factor or a gene encoding the same. The term "transcription factor" as used herein specifies a specific subclass of the umbrella term "differentiation factors". To explain further, a transcription factor specifies a protein that binds to specific DNA sequences and thereby controls the transcription of genetic information from DNA to mRNA. In turn, the protein encoded by the mRNA (directly or via the induction of further gene products) leads to a more differentiated cellular stage. Thus, a transcription factor falls within the definition of a differentiation factor in accordance with the invention.

In accordance with the first and second aspect of the present invention at least one differentiation factor is capable of inducing NPAS4 function and is thus capable of inducing the expression of the gene tal1 and/or the gene etv2. However, also one or more additional differentiation factors are preferably used which additional differentiation factor(s) when contacted with the progenitor cells lead(s) to the desired more differentiated cellular stage. In accordance with the first aspect of the invention the additional differentiation factor(s) contribute to producing endothelial cells from progenitor cells and in accordance with the second aspect of the invention the additional differentiation factor(s) contribute to producing hematopoietic cells from progenitor cells.

Various differentiation factors for obtaining endothelial cells are known in the art and non-limiting examples are Shh, VEGF, PLCγ, MEK, ERK, VEGFR-1-NRP-1 receptor complex, components of PI3K/AKT pathway, COUP-TFII, Foxc1/2, Notch signaling (e.g. Notch1, Notch4, Jag1, Jag2, and DII4), Sox7, Sox18, Prox1, ephrinB2, EphB4 and Nrp-1 (Atkins et al. (2011), Arteriosclerosis, Thrombosis, and Vascular Biology, 31: 1476-1484). It has been recently demonstrated that combined TGFβ and canonical Wnt agonists are essential and sufficient for iPS/ES cell-to-mesoderm transition and that VEGF-KDR signalling alone is required for endothelial cell formation while further supplementation with FGF allows for colonial endothelial differentiation (Wu et al. (2015), Scientific Reports, 5:9718:DOI: 10.1038/srep09718). It is therefore preferred that a differentiation factor being capable of inducing NPAS4 function is used in combination with VEGF-KDR signalling, more preferably in combination with one or more of TGFβ, a canonical Wnt agonist and VEGF-KDR signalling.

Likewise various differentiation factors for obtaining hematopoietic cells are known in the art and non-limiting examples are (members of the) activins, BMPs (Bone Morphogenetic Proteins), common beta chain receptor family, common gamma chain receptor family, EGF family, FGF family, growth/differentiation factors (GDFs), hedgehog family, IGF family, IL-3, IL-6 family, PDGF family, SCF, Flt-3 ligand and M-CSF, VEGF family and Wnt family. Several transcription factors have been found to play critical roles in HSC (hematopoietic stem cells) physiology, including SCL (stem cell leukemia hematopoietic transcription factor), GATA-2, and Lmo-2, which are essential for primitive and definitive hematopoiesis, and AML-1 that is required for definitive hematopoiesis. Moreover, the transcription factors HoxB4 and Ikaros activate the nuclear form of Notch1, the cell cycle inhibitor P21, TGF/BMP-4 family members as well as TNFα receptor P55 signaling all being involved in the maintenance or promotion of the hematopoietic stem cell renewal (Zhu and Emerson (2002), Oncogen, 21(21):3295-3313). It is therefore preferred that a differentiation factor being capable of inducing NPAS4 function is used in combination with one or more selected from SCL (stem cell leukemia hematopoietic transcription factor), GATA-2, Lmo-2 and AML-1, and more preferably in addition with one or more selected from HoxB4, Ikaros, Notch1, P21, TGF/BMP-4 family members and factors of TNFα receptor P55 signaling.

In the methods of the present invention the differentiation factor(s) is/are generally introduced into the progenitor cells. The term "introducing" as used herein specifies the process of bringing a protein and/or nucleic acid sequence into a living cell, and in particular also into the cell nucleus, preferably by introducing means as defined herein below. The differentiation factor(s) may be introduced into the progenitor cells, for example, by microinjection, electroporation, lipofection and/or protein transduction All these introduction methods are well-established in the art. In this regard, the differentiation factor(s) to be introduced may either be isolated from their natural environment or recombinantly produced.

A liposome used for lipofection is a small vesicle, composed of the same material as a cell membrane (i.e., normally a lipid bilayer e.g. out of phospholipids), which can be filled with one more protein(s) (e.g. Torchilin VP. (2006), Adv Drug Deliv Rev., 58(14):1532-55). To deliver a protein into a cell, the lipid bilayer of the liposome can fuse with the lipid bilayer of the cell membrane, thereby delivering the contained protein into the cell. It is preferred that the liposomes used in accordance with invention are composed of cationic lipids. The cationic liposome strategy has been applied successfully to protein delivery (Zelphati et al. (2001), J. Biol. Chem. 276:35103-35110). As known in the art, the exact composition and/or mixture of cationic lipids used can be altered, depending upon the protein(s) of interest and the cell type used (Feigner et al. (1994), J. Biol. Chem., 269:2550-2561).

Protein transduction specifies the internalisation of proteins into the cell, from the external environment (Ford et al (2001), Gene Therapy, 8:1-4). This method relies on the inherent property of a small number of proteins and peptides (preferably 10 to 16 amino acids long) being able to penetrate the cell membrane. The transducing property of these molecules can be conferred upon proteins which are expressed as fusions with them and thus offer, for example, an alternative to gene therapy for the delivery of therapeutic proteins into target cells. Commonly used proteins or peptides being able to penetrate the cell membrane are, for example; the antennapedia peptide, the herpes simplex virus VP22 protein, HIV TAT protein transduction domain, peptides derived from neurotransmitters or hormones, or a 9xArg-tag.

Microinjection refers to the process of using a glass micropipette to introduce substances at a microscopic or borderline macroscopic level into a single living cell. Electroporation relies on a significant increase in the electrical conductivity and permeability of the cell plasma membrane caused by an externally applied electrical field. By increasing permeability, protein (or peptides or nucleic acid sequences) can be introduced into the living cell.

It is demonstrated in the examples herein below that Npas4-like is essential for the formation of endothelial cells and hematopoietic cells in zebrafish. Endothelial cells and hematopoietic cells are built from common progenitor cells called hemangioblasts. In more detail, the zebrafish cloche mutant is known to have an embryonic lethal phenotype, which is caused by the lack of the development of endothelial cells and hematopoietic cells from hemangioblasts. It was unexpectedly found in accordance with the present invention that the cloche zebrafish mutant can be rescued by zebrafish Npas4-like and even more unexpectedly that it could also be rescued by the human ortholog of Npas4-like NPAS4, despite the fact that zebrafish Npas4-like and human NPAS4 share less than 35% sequence identity. The cellular fate between endothelial cells and hematopoietic cells may be controlled, for example, by one or more of the above-discussed further differentiation factor(s). It therefore can be assumed that inducing NPAS4 function in progenitor cells promotes the differentiation into endothelial cells as well as hematopoietic cells. To the best knowledge of the inventors it was not known from the prior art that human NPAS4 or a species ortholog thereof plays a role in the differentiation of endothelial cells and hematopoietic cells from progenitor cells. As will be further discussed herein below, the identity and sequence of zebrafish Npas4-like was even not known from the prior art.

In accordance with a preferred embodiment, the method of the first and second aspect of the invention further comprises isolating the endothelial cells or the hematopoietic cells, respectively.

In the above-described methods of the invention not each progenitor cell may be transformed into an endothelial cell or hematopoietic cell, respectively. For example, some cells may undergo cell death (i.e. apoptosis) or cells may stop differentiation at an intermediate stage. Any such cells that were not transformed into an endothelial cell or a hematopoietic cell, respectively, may be removed and the endothelial cells or the hematopoietic cells, respectively, may be isolated.

Methods for isolating cells are well known in the state of the art and comprise tecniques such as FACS (fluorescence activated cell sorting), sucrose gradient centrifugation, (laser) microdissection, single cell dilution, and Magnetic Labeled Bead Cell Separation.

FACS is a method for sorting a heterogeneous mixture of biological cells into two or more containers, one cell at a time, based upon the specific light scattering and fluorescent characteristics of each cell. For this purpose a cell specific marker. e.g. a specific endothelial or hematopoietic cell-surface marker may be labeled with a fluorescent dye like FLAG, GFP, YFP, RFP, dTomato, cherry, Cy3, Cy5, Cy 5.5., Cy7, AMCA, Tamra, Texas Red, rhodamine, Alexa fluors, FITC or TRITC. Endothelial cell-surface markers include but are not limited to VE-Cadherin, CD31/PECAM-1, CD34, CD117/c-kit, CXCR4, MCAM/CD146 S1P1/EDG-1, S1P2/EDG-5, S1P3/EDG-3, S1P4/EDG-6, S1P5/EDG-8, E-Selectin/CD62E, E-Selectin (CD62E)/P-Selectin (CD62P), Tie-2, VCAM-1/CD106, ETSRP, VEGF R1/Flt-1 and VEGF R2/KDR/Flk-1. Also hematopoietic cell-surface markers, including cell-surface markers being specific for the different lineages and types of hematopoietic cells are known and, for example, summarized in Attar (2014), Global Journal of Hematology and Blood Transfusion, 1:20-28 and Takafumi Yokota et al. (2012), Markers for Hematopoietic Stem Cells: Histories and Recent Achievements, Advances in Hematopoietic Stem Cell Research, Dr. Rosana Pelayo (Ed.), ISBN: 978-953-307-930-1, InTech.

Sucrose gradient centrifugation is a centrifugation technique which separates compounds according to their density along a sucrose density gradient which is typically created overlaying lower concentrations of sucrose on higher concentrations in a centrifuge tube. Single cell dilution is a technique of diluting cells, preferably in culture medium until a concentration is reached that allows to separate a single cell in a separated vial. Magnetic Labeled Bead Cell Separation is a commercial system available from Miltenyi Biotec (MACS), Bergisch Gladbach, Germany.

In accordance with a preferred embodiment of the first aspect of the invention, the progenitor cells are embryonic stem cells (ESCs), induced pluripotent stem cells (iPS), mesodermal progenitor cells, hemangioblasts, endothelial progenitor cells (EPCs), fibroblasts or a mixture thereof.

Embryonic stem cells (ESCs) are pluripotent stem cells derived from the inner cell mass of a blastocyst, an early-stage preimplantation embryo. While the derivation of human ESCs cells initially required the destruction of *ex utero* embryos, it is now possible to generate ESCs by techniques which do not interfere with the embryo's developmental potential, such as by a single-cell biopsy (WO 2003/046141, Thomson et. al (1998), Science 282 (1145): 1145-1147, Thomson et al. (1998), Science 282 (5391): 1145-7, Klimanskaya et al., 2006, Nature 444:481-485). The human ESCs herein were/are accordingly preferably prepared by techniques which do not require the destruction of a human embryo and/or do not interfere with the human embryo's developmental potential. Specific examples are the cell lines CHB-1, CHB-2, or CHB-3.

Induced pluripotent stem cells (also known as iPS cells or iPSCs) are a type of pluripotent stem cell that can be generated directly from adult cells. The first protocol for the generation of iPSCs was established in 2006 (Takahashi et al. (2006), Cell, 126(4):663-676) and today various protocols are available in the art. Since iPSCs can be derived directly from adult tissues, they not only bypass the need for embryos, but also can be made in a patient-matched manner. This means that each individual could have their own pluripotent stem cell line.

Mesodermal progenitor cells may be derived, for example, from adult human bone marrow (Trombi et al. (2009), Stem Cells Dev, 18(8):1227-34). They can easily be induced to differentiate into mature mesenchymal stromal cells (MSCs) or into endothelial cells.

Hemangioblast are multipotent progenitor cells that can differentiate into both hematopoietic and endothelial cells (A. Cumano, I. Godin: Ontogeny of the hematopoietic system. In: Annual review of immunology. 25, 2007, p.745-785). Hemangioblasts have been first extracted from embryonic cultures and can be manipulated, for example, by cytokines to differentiate into the hematopoietic or endothelial cell lineage.

Endothelial progenitor cells (EPCs) are capable of generating phenotypically and functionally competent endothelial cells and can *inter alia* be found in the blood (Pelosi et al. (2014), Blood Cells, Molecules, and Diseases, 52(4):186-194).

Also fibroblasts can serve as progenitor cells as they can be induced to become endothelial cells capable of angiogenesis and reendothelialization in tissue-engineered vessels (Margarati el al. (2012), PNAS, 109(34):13793-8).

In accordance with a preferred embodiment of the second aspect of the invention, the progenitor cells are embryonic stem cells (ESCs), induced pluripotent stem cells (iPSs), mesodermal progenitor cells, hemangioblasts, angioblasts, hematopoietic stem cells (HSCs), or a mixture thereof.

Angioblasts may be isolated from embryos and are the earliest formative tissue from which blood cells and blood vessels arise in further embryogenesis (Kovacic et al. (2008), Trends Cardiovasc Med, 18(2):45-51).

Hematopoietic stem cells (HSCs) give rise to all the other more differentiated hematopoietic cells through the process of haematopoiesis. They are derived from mesoderm and are located in adults in the red bone marrow, which is contained in the core of most bones.

In accordance with a preferred embodiment of the first and second aspect of the invention the NPAS4 function is induced by (a) a polypeptide comprising or consisting of human NPAS4 or a species ortholog thereof; or (b) a nucleic acid molecule encoding the amino acid sequence of (a).

The term "polypeptide" as used herein describes linear molecular chains of amino acids, including single chain proteins or their fragments, containing generally more than 50 amino acids. Polypeptides may further form oligomers consisting of at least two identical or different molecules. The corresponding higher order structures of such multimers are, correspondingly, termed homo- or heterodimers, homo- or heterotrimers etc. Furthermore, peptidomimetics of such polypeptides where amino acid(s) and/or peptide bond(s) have been replaced by functional analogs are also encompassed by the invention. Such functional analogues include all known amino acids other than the 20 gene-encoded amino acids, such as selenocysteine. The term "polypeptide" also refers to naturally modified polypeptides where the modification is effected e.g. by glycosylation, acetylation, phosphorylation and similar modifications which are well known in the art.

As mentioned, the amino acid sequence of human NPAS4 is shown in SEQ ID NO: 1 and species orthologs thereof in SEQ ID NOs 2 to 31. Orthologs (or orthologues) are genes in different species that evolved from a common ancestral gene by speciation. In general, orthologs retain the same or essentially same function in the course of evolution. It is shown in the examples herein below that in the cloche mutant of zebrafish human NPAS4 (SEQ ID NO: 1) can substitute the mutated zebrafish Npas4-like (SEQ ID NO: 25). For this reason human NPAS4 may be replaced by any species ortholog thereof. Among sequenced and annotated genomic sequences orthologs can be identified by routine methods, such as those described in Valender (2010), Methods, 49(1): 50-55. The species ortholog of human NPAS4 is preferably a verterbrate ortholog of human NPAS4, more preferably a tetrapoda ortholog of human NPAS4, even more preferably a mammalian ortholog of human NPAS4, and most preferably a primate ortholog of human NPAS4.

NPAS4 was found to heterodimerize with ARNT (aryl hydrocarbon receptor nuclear translocator), ARNT2 or ARNTL (aryl hydrocarbon receptor nuclear translocator-like protein). For this reason it is preferred that NPAS4 function is induced by a polypeptide comprising or consisting of human NPAS4 or a species ortholog thereof in combination with one or more selected from human ARNT, ARNT2, ARNTL or a species ortholog of one of human ARNT, ARNT2 and ARNTL. In this regard it is preferred that all polypeptides are from the same order (such as primates) and more preferably from the same species (such as human). Instead of or in addition to the polypeptides corresponding nucleic acid molecules encoding the desired amino acid sequences may be used. The term "nucleic acid molecule" will be further defined herein below.

In accordance with a preferred embodiment of the first and second aspect of the invention the NPAS4 function is induced by (i) a polypeptide comprising or consisting of an amino acid sequence being selected from the group consisting of SEQ ID NOs 1 to 31; (ii) a polypeptide being at least 60% identical to an amino acid sequence being selected from the group consisting of SEQ ID NOs: 1 to 31; (iii) a polypeptide comprising at least 600 amino acids, wherein the N-terminal portion of said polypeptide is at least 80% identical to the N-terminal half of an amino acid sequence being selected from the group consisting of SEQ ID NOs: 1 to 31; (iv) a polypeptide being at least 20% identical to an amino acid sequence being selected from the group consisting of SEQ ID NOs 1 to 31 outside the bHLH domain, the PAS1 domain and the PAS2 domain, being at least 75% identical to an amino acid sequence being selected from the group consisting of SEQ ID NOs 1 to 31 inside the bHLH domain, and being at least 50% identical to an amino acid sequence being selected from the group consisting of SEQ ID NOs 1 to 31 inside the PAS1 domain and the PAS2 domain; (v) a nucleic acid molecule encoding the polypeptide of any one of (i) to (iv); and/or (vi) a vector comprising the nucleic acid molecule of (v).

It is to be understood that the means for NPAS4 function as defined in items (i) to (vi) are capable of inducing NPAS4 function. The meaning of "inducing NPAS4 function" has been defined herein above in greater detail and is in particular amenable for combination with the above preferred embodiment.

The polypeptide of item (ii) above is with increasing preference as at least 70%, at least 75%, at least 80%, at least 85%, at least 90%, at least 92.5%, at least 95%, at least 98% and at least 99% identical to an amino acid sequence being selected from the group consisting of SEQ ID NOs 1 to 31.

The polypeptide of item (iii) above is with increasing preference at least 85%, at least 90%, at least 92.5%, at least 95%, at least 98% and at least 99% identical to the N-terminal half of an amino acid sequence selected from the group consisting of SEQ ID NOs 1 to 31. The term "N-terminal half" means the first 50% of the entire amino acid sequence of an amino acid sequence selected from the group consisting of SEQ ID NOs 1 to 31. For example, SEQ ID NO: 1 comprises 802 amino acids and the "N-terminal half" consequently consists of amino acids 1 to 401 of SEQ ID NO: 1. SEQ ID NO: 31 comprises 647 amino acids and the "N-terminal half" consequently consists of amino acids 1 to 324 SEQ ID NO: 1 (noting that .5 values are rounded up). The polypeptide of item (iii) comprises, preferably at least 625 amino acids, more preferably at least 650 amino acids and most preferably at least at least 700 amino acids. Combinations of the preferred lengths and % identities are also comprised by the present invention, such as at least 90% and at least 625 amino acids, at least 92.5% and at least 650 amino acids, and at least 95% and at least 700 amino acids. The number of amino acids of the "N-terminal portion" is preferably up to ± 20% of, more preferably up to ± 10% of, even more preferably up to ± 5% and most preferably corresponds to the number of amino acids of the N-terminal half.

The polypeptide of item (iv) above is with increasing preference at least 30%, at least 40%, at least 50%, at least 60%, at least 70%, at least 80%, at least 90%, at least 95%, at least 98% and at least 99% identical to an amino acid sequence being selected from the group consisting of SEQ ID NOs 1 to 31 outside the bHLH domain, the PAS1 domain and the PAS2 domain. Moreover, the polypeptide of item (iv) above is with increasing preference at least 80%, at least 85%, at least 90%, at least 92.5%, at least 95%, at least 98% and at least 99% identical to an amino acid sequence being selected from the group consisting of SEQ ID NOs 1 to 31 inside the bHLH domain. Further, the polypeptide of item (iv) above is with increasing preference at least 60%, at least 70%, at least 80%, at least 90%, at least 95%, at least 98% and at least 99% identical to an amino acid sequence being selected from the group consisting of SEQ ID NOs 1 to 31 inside the PAS1 domain and the PAS2 domain. It is to be understood that the present invention also encompasses any combination of the listed sequence identities (i) outside the bHLH domain, the PAS1 domain and the PAS2 domain, (ii) inside the bHLH domain, and (iii) inside the PAS1 domain and the PAS2 domain. Non-limiting examples are (i) at least 50%, (ii) at least 90%, and (iii) at least 80%; (i) at least 60%, (ii) at least 92.5%, and (iii) at least 90%; and (i) at least 80%, (ii) at least 95%, and (iii) at least 90%; and (i) at least 90%, (ii) at least 98%, and (iii) at least 95%.

In accordance with the present invention, the term "at least % identical to" in connection with the amino acid sequences of the present invention describes the number of matches ("hits") of identical amino acids of two or more aligned amino acid sequences as compared to the number of amino acids making up the overall length of the amino acid sequences (or the overall compared part thereof). In other terms, using an alignment, for two or more sequences or subsequences the percentage of amino acids that are the same (e.g. at least 70% identity) may be determined, when the (sub)sequences are compared and aligned for maximum correspondence over a window of comparison, or over a designated region as measured using a sequence comparison algorithm as known in the art, or when manually aligned and visually inspected. Preferred amino acid sequences in accordance with the invention are those where the described identity exists over a region that is at least 100 to 150 amino acids in length, more preferably, over a region that is at least 200 to 400 amino acids in length. More preferred amino acid sequences in accordance with the present invention are those having the described sequence identity over the entire length of the amino acid sequences as described in the embodiments herein. Those having skill in the art will know how to determine percent sequence identity between/among sequences using, for example, algorithms such as those based on CLUSTALW computer programme (Thompson Nucl. Acids Res. 2 (1994), 4673-4680) or FASTA (Pearson and Lipman, Proc. Natl. Acad. Sci., 1988, 85; 2444), as known in the art. Although the FASTA algorithm typically does not consider internal non-matching deletions or additions in sequences, i.e., gaps, in its calculation, this can be corrected manually to avoid an overestimation of the % sequence identity. CLUSTALW, however, does take sequence gaps into account in its identity calculations. Also available to those having skill in this art are the BLAST and BLAST 2.0 algorithms (Altschul, Nucl. Acids Res., 1977, 25:3389). For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. Hence, the percentage sequence identity of polypeptide sequences can be determined with programmes as the above explained programs CLUSTLAW, FASTA and BLAST. Preferably the BLAST program is used.

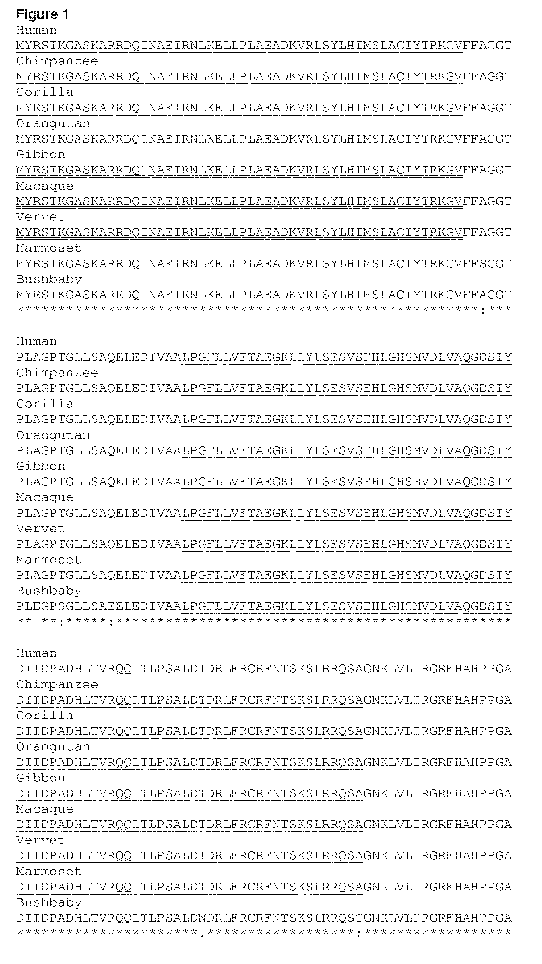

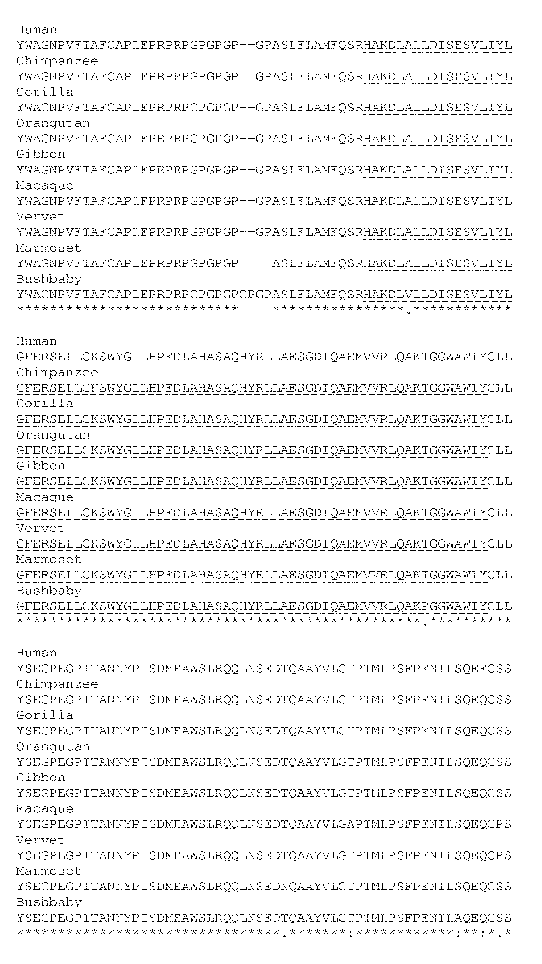

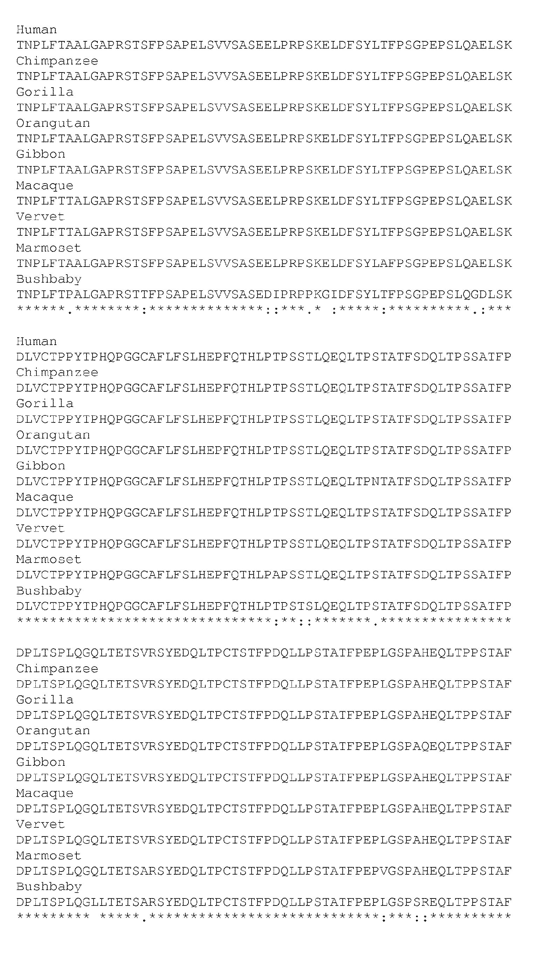

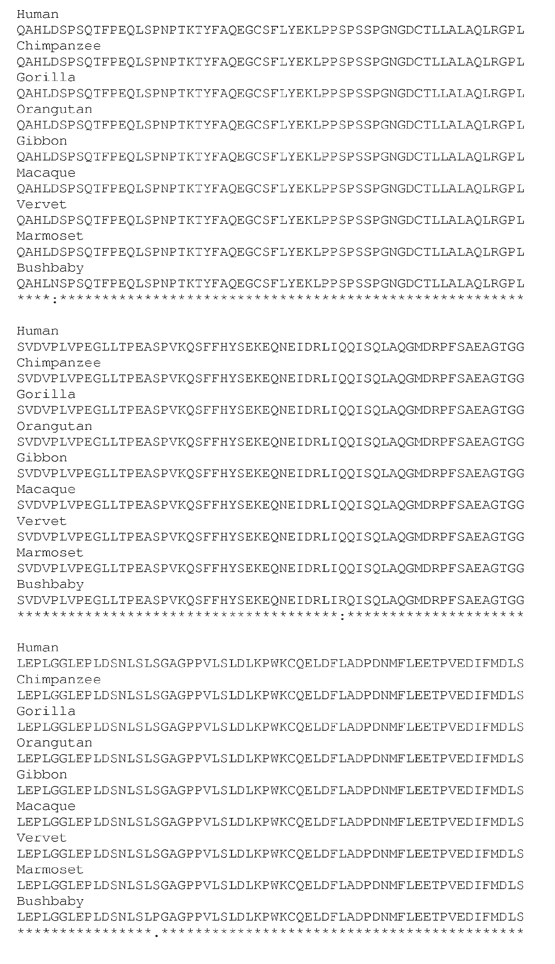

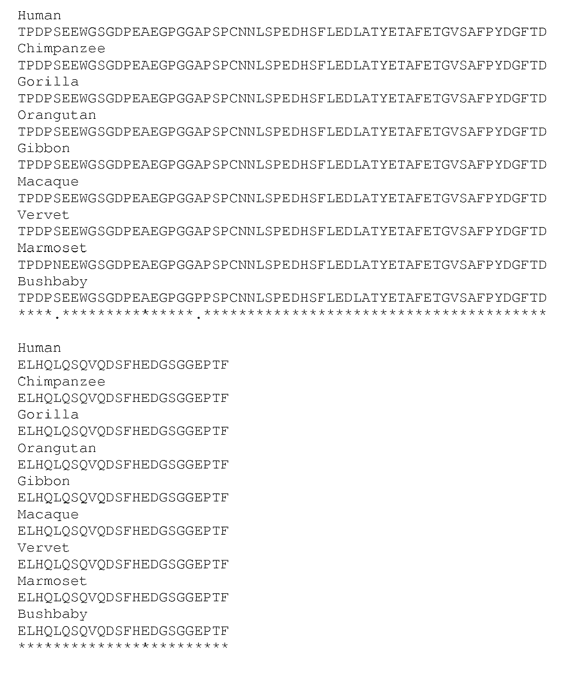

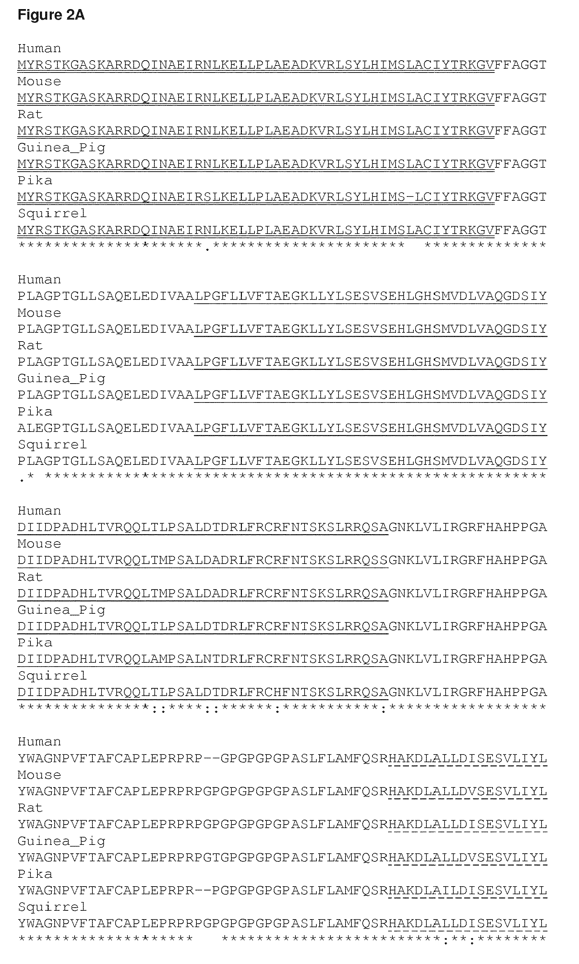

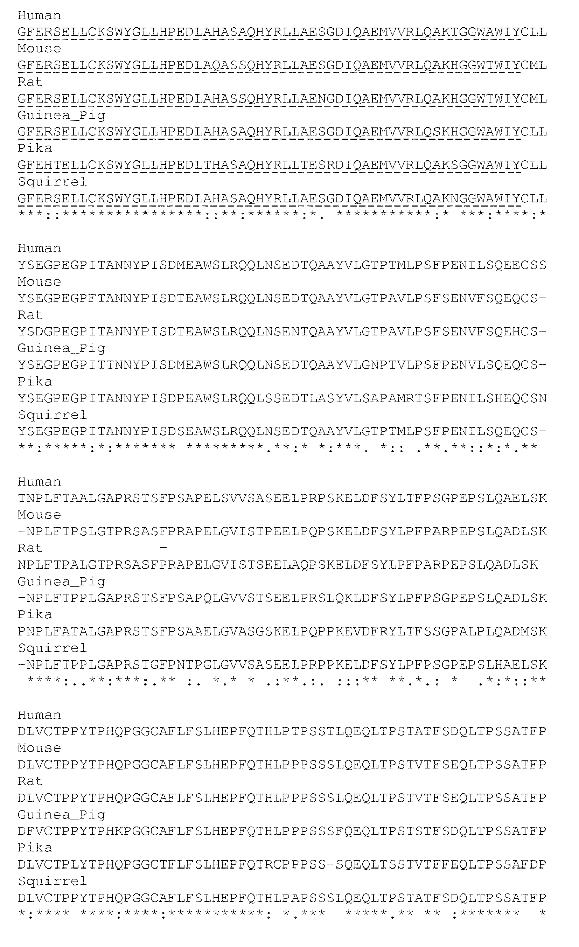

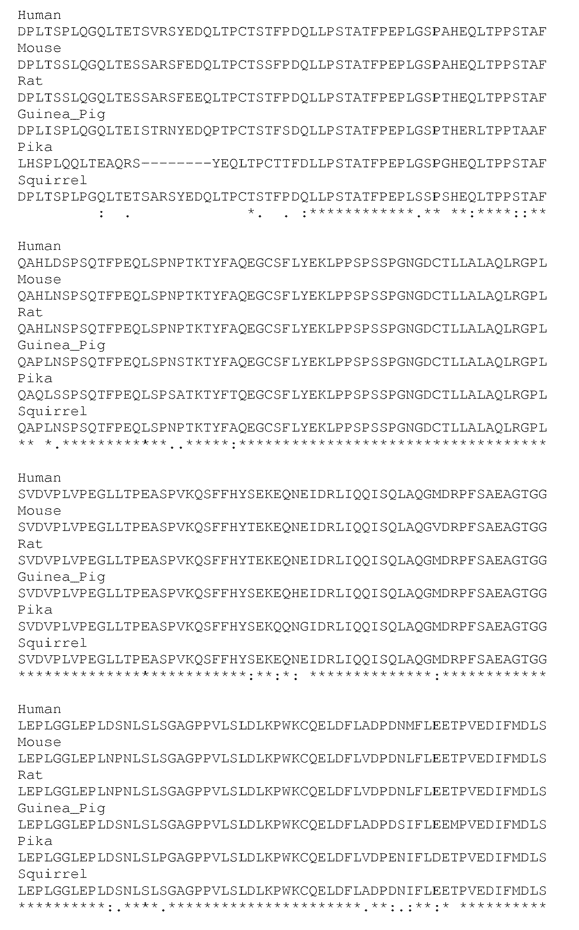

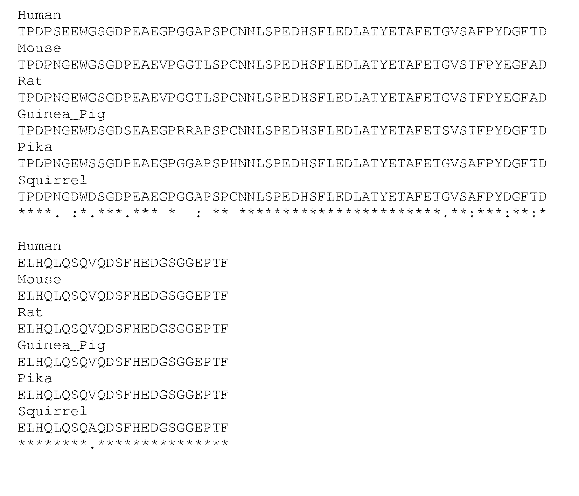

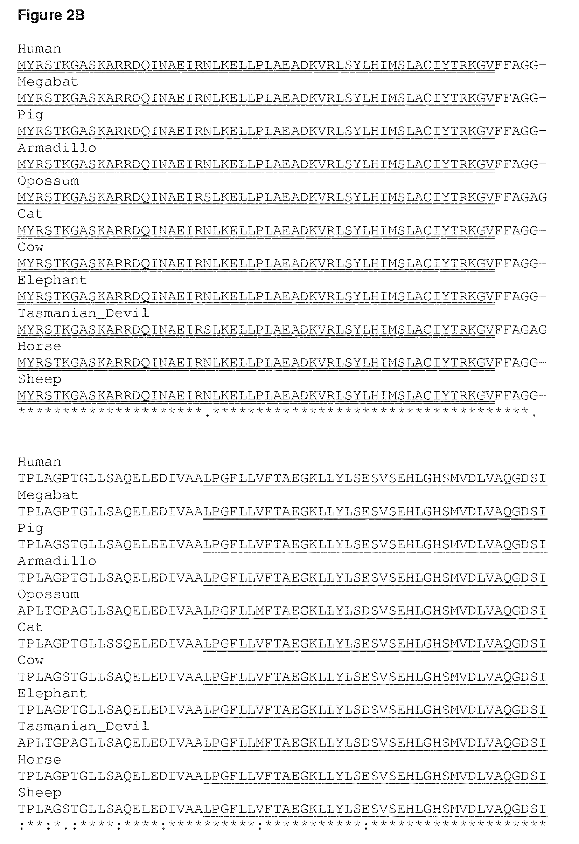

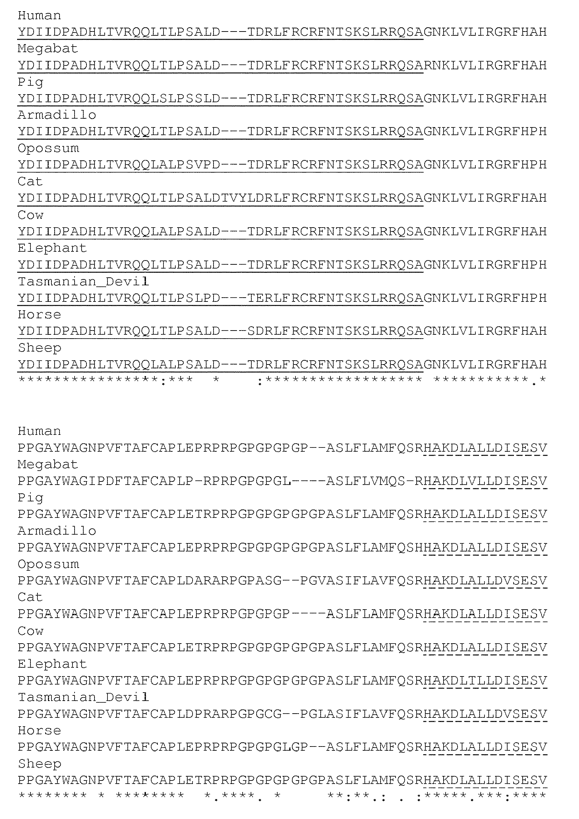

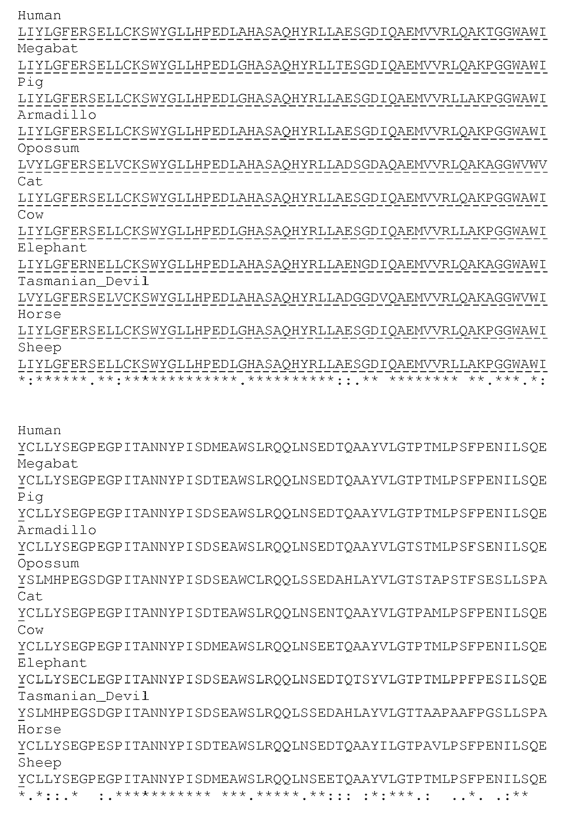

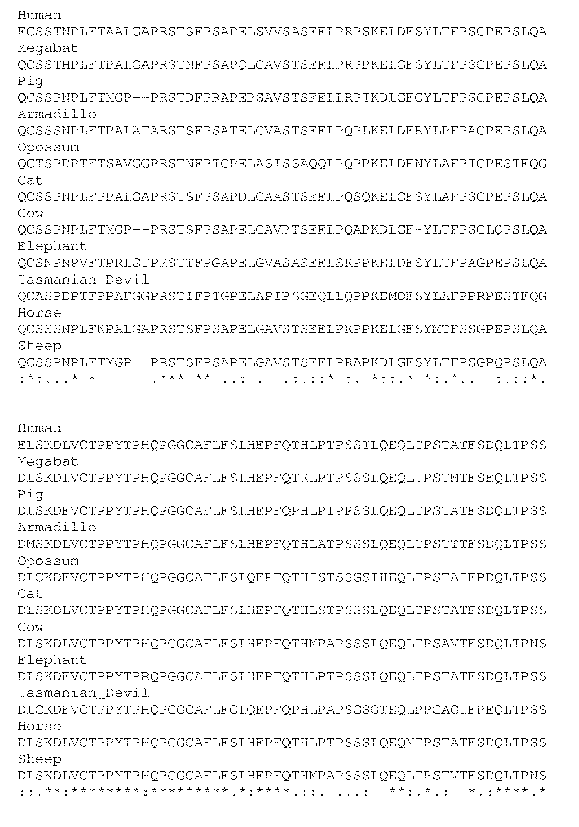

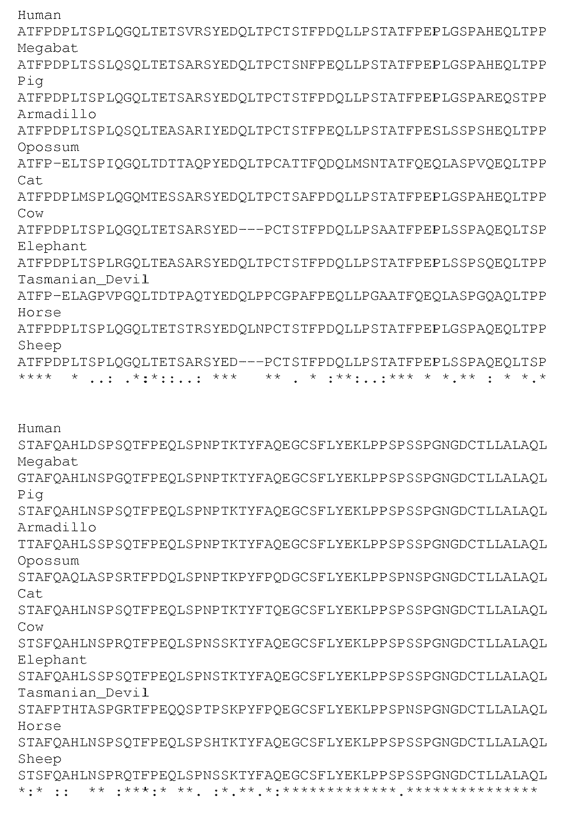

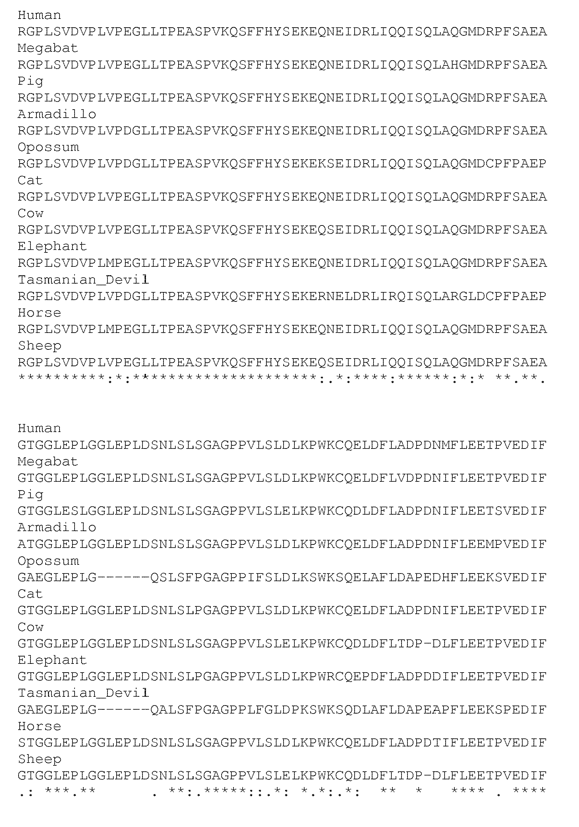

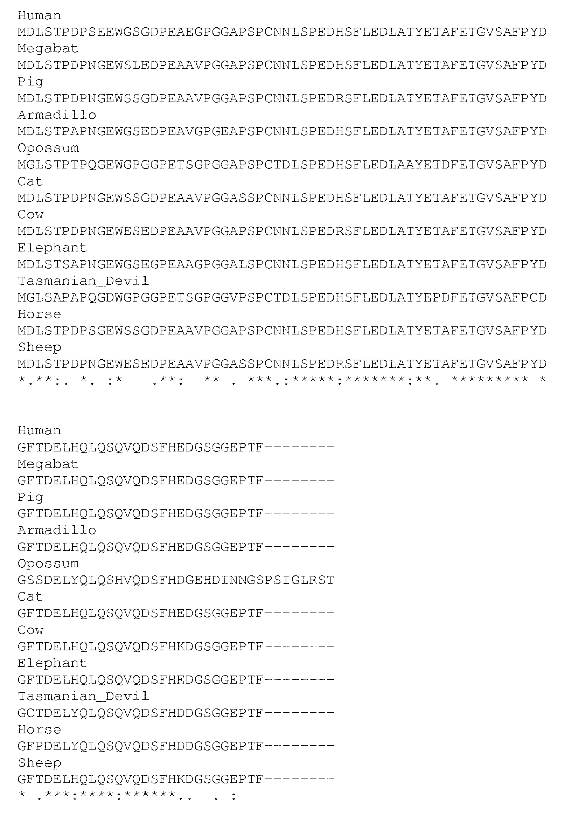

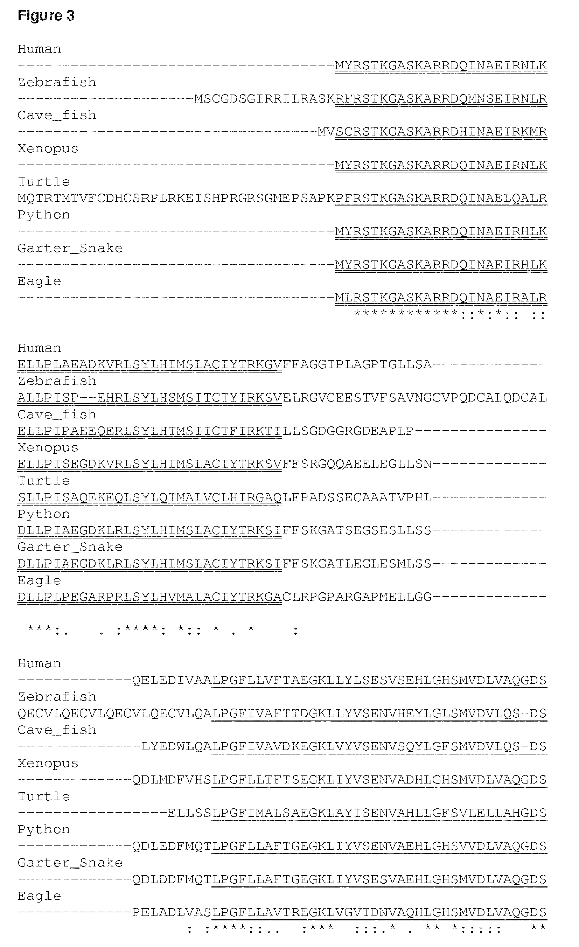

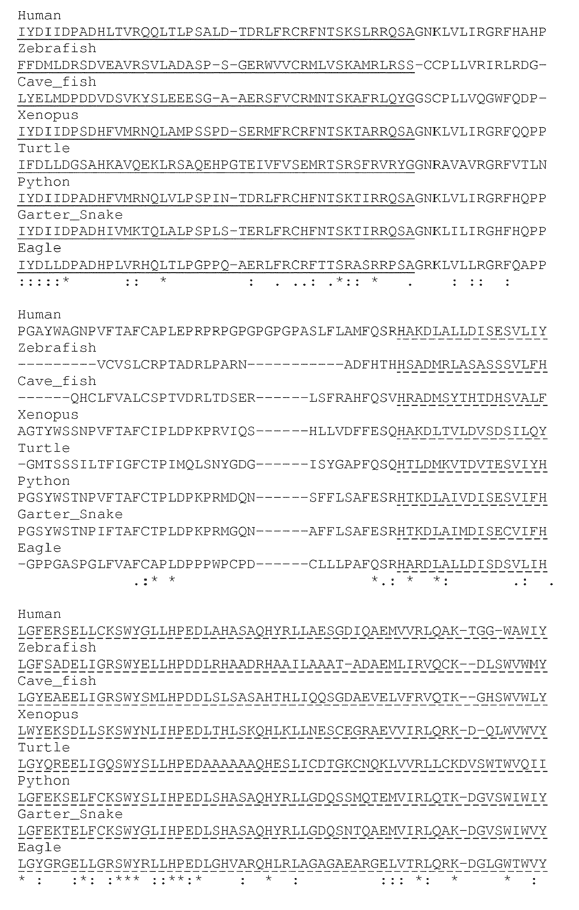

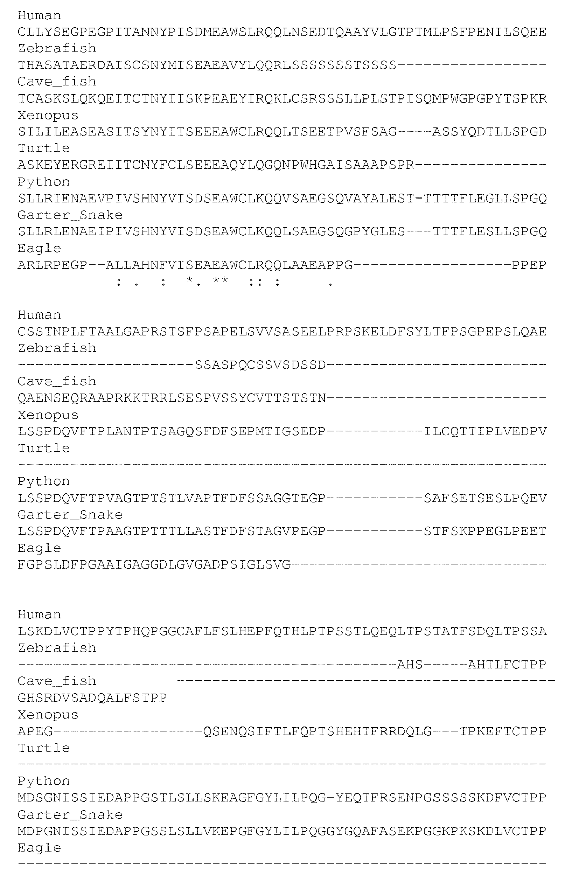

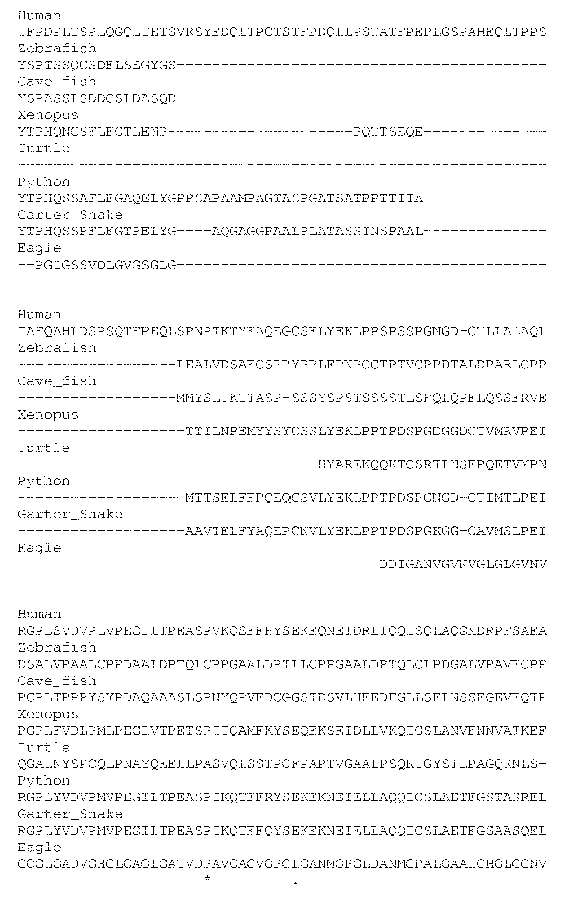

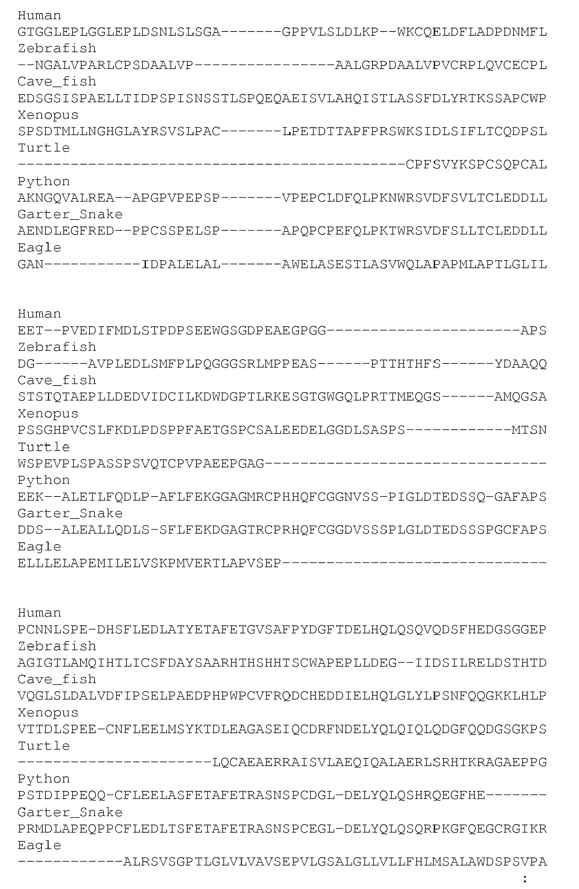

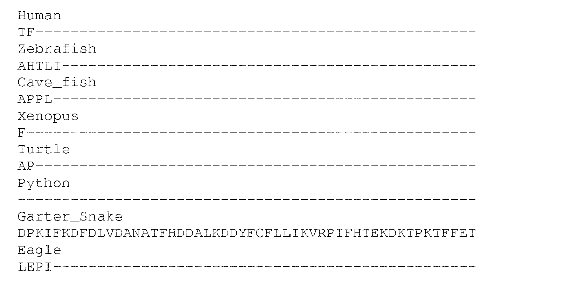

Figures 1 to 3 show multiple sequence alignments of human NPAS4 with various orthologs from other species. Figure 1 is an alignment of human NPAS4 with various orthologs from other primates, including apes, old word monkeys, new world monkeys and a prosimian. The alignment shows that NPAS4 is almost perfectly conserved among primates. Figure 2 is an alignment of human NPAS4 with various orthologs from mammals other than primates. The human NPAS4 still shares more than about 75% sequence identity with the NPAS4 from the marsupial Tasmanian devil. Figure 3 is an alignment of human NPAS4 with various orthologs from vertebrates other than mammals, including fish and sauropsida. Despite the overall low sequence identity between human NPAS4 and the NPAS4 orthologs from selected fish and sauropsida (i.e. in the range of about 30% to about 45%), it is surprisingly demonstrated in the examples herein below that in zebrafish development human NPAS4 can rescue the zebrafish mutant cloche which mutant lacks a functional species ortholog; i.e. a functional Npas4-like. This evidences that the species orthologs despite considerable sequence differences still display the same function or essentially same function *in vivo.*

As discussed above, human NPAS4 comprises in its N-terminal part a bHLH domain, followed by 2 PAS domains and in its C-terminal part an activation domain. This structure and these domains are evolutionary conserved in all species orthologs. Within the alignments shown in Figures 1 to 3 the location and sequences of the bHLH domain, the PAS1 domain and the PAS2 domain are indicated. The alignments also show that the N-terminal part including the functionally important bHLH domain and the two PAS domains are considerably more evolutionary conserved as compared to the C-terminal part harboring the activation domain. The exact sequence of the activation domain seems to be of no particular relevance and is believed to predominantly contribute to the folding of the NPAS4 protein. The bHLH domain, the PAS1 domain and the PAS2 domain show the highest evolutionary sequence conversation and among these domains the bHLH domain is most conserved. For example, the bHLH domain in NPAS4 of human and xenopus is 100% conserved.

In accordance with item (v) a nucleic acid molecule encoding the polypeptide of any one of the above items (i) to (iv) may be employed in the method of the first and second aspect of the invention. The term "nucleic acid molecule", in accordance with the present invention, includes DNA, such as cDNA or genomic DNA, and RNA. It is understood that the term "RNA" as used herein comprises all forms of RNA including mRNA. The term "nucleic acid molecule" is interchangeably used in accordance with the invention with the terms "polynucleotide" or "nucleic acid sequence".

The nucleic acid molecule(s) may also comprise regulatory regions or other untranslated regions. In a further embodiment, said nucleic acid molecule may comprise heterologous nucleic acid which may encode heterologous proteinaceous material thus giving rise, e.g., to fusion proteins.

The present invention thus relates to a nucleic acid molecule(s) of the invention encoding a fusion protein. For example, the nucleic acid molecule of the invention can be fused in frame to a detectable marker such as purification marker or a direct or indirect fluorescence marker. Purification marker comprise one or more of the following tags: His, lacZ, GST, maltose-binding protein, NusA, BCCP, c-myc, CaM, FLAG, GFP, YFP, cherry, thioredoxin, poly(NANP), V5, Snap, HA, chitin-binding protein, Softag 1, Softag 3, Strep, or S-protein. Suitable direct or indirect fluorescence markers comprise FLAG, GFP, YFP, RFP, dTomato, cherry, Cy3, Cy5, Cy5.5, Cy7, DNP, AMCA, Biotin, Digoxigenin, Tamra, Texas Red, rhodamine, Alexa fluors, FITC, TRITC or any other fluorescent dye or hapten. Other fusion partners may encode a protein, which may increase the solubility, correct protein folding, and/or a further differentiation factor enhancing differentiation and/or maintenance of the mature endothelial cell or hematopoietic cell phenotype.

In accordance with item (vi) the nucleic acid molecule of item (v) is comprised in a vector. Accordingly, the present invention also relates to a vector comprising the nucleic acid molecule(s) of the present invention. Preferably, the vector is a plasmid, cosmid, virus, bacteriophage or another vector used e.g. conventionally in genetic engineering.

The nucleic acid molecule may be inserted into several commercially available vectors. The nucleic acid molecule(s) referred to above may also be inserted into vectors such that a translational fusion with another polynucleotide is generated. Examples of such fusion proteins have been described herein above. For vector modification techniques see Sambrook and Russel (2001), Molecular Cloning, Cold Spring Harbor Laboratory; 3rd edition.

Generally, vectors can contain one or more origin of replication (ori) and inheritance systems for cloning or expression, one or more markers for selection in the host and one or more expression cassettes. Suitable origins of replication (ori) include, for example, the Col E1, the SV40 viral and the M 13 origins of replication. Suitable selectable markes are, e.g. dihydrofolate reductase, G418, neomycin, ampicillin, dhfr, gpt, hygromycin, or kanamycin.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e.g., promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Highly efficient transcription can be achieved with the early and late promoters from SV40, the long terminal repeats (LTRs) from retroviruses, e.g., RSV, HTLVI, HIVI, and the early promoter of the cytomegalovirus (CMV).

In accordance with a preferred embodiment of the method of the invention the nucleic acid sequences encode the nucleic acid molecule of the invention in an expressible form, such as an inducible expressible form. Accordingly, also the vector of the invention is preferably an expression vector. An expression vector according to this invention is capable of directing the replication, and the expression, of the polynucleotide and encoded differentiation factor(s) of this invention. Suitable expression vectors, which comprise the described regulatory elements are known in the art. The vector of the invention may be a mammalian expression vector. A typical mammalian expression vector contains the promoter element, which mediates the initiation of transcription of mRNA, the protein coding sequence, and signals required for the termination of transcription and polyadenylation of the transcript and moreover, elements such as origin of replication, drug resistance gene, regulators (as part of an inducible promoter) may also be included. The nucleic acid molecules as described herein above may be designed for direct introduction into a cell, or introduction via phage vectors or viral vectors (e.g. adenoviral, retroviral) into a cell. Systems for inducible expression are widely used in the state of the art and include but are not limited to the Tet-on system, Tet-off system, lac operator/expressor system and the commercial systems Q-mate™, Rheo Swith®, Cumate® or TREX™. As known in the art, inducible expression systems allow one to control the expression of a gene over time, the order of the genes expressed (provided that different expression systems, e.g. a Tet-on system and Tet-off system are used in parallel or subsequently for the expression of different genes) and also the amount of expressed mRNA from the vector.

In an even more preferred embodiment of the method of the invention the one or more vector(s) are one or more lentiviral vector(s).Examples of lentiviruses providing elements of a lentiviral vector are HIV, SIV, FIV, Puma lentivirus, EIA, ·Bovine immunodeficiency virus, Caprine arthritis encephalitis virus, or Visna/maedi virus. Preferably used in accordance with the invention is a 3^{rd} generation lentiviral expression plasmid containing a SFFV promoter driven IRES-Puro (Foxa2), IRES-dTomato (C/EBPα) or IRES-eGFP (HNF4α) expression cassette. Lentiviruses are particularly useful in the method for the invention because they integrate into the genome and thus allow for non-transient gene expression. Moreover, studies have shown that lentivirus vectors have a lower tendency to integrate in places that potentially cause cancer than gamma-retroviral vectors which also integrate into the genome (Cattoglio et al. (2007), Blood, 110(6):1770-1778). Thus they have a lower tendency of abnormal cell growth upon integration.

In a third aspect the present invention pertains to a method for producing cardiomyocytes from progenitor cells, said method comprising inhibiting NPAS4 function in the progenitor cells.

The definitions of terms, preferred embodiments and practical guidance provided herein above with regard to the first and second aspect of the invention apply *mutatis mutandis* to the third aspect of the invention.

Accordingly, in connection with the third aspect of the invention the progenitor cells have the capacity of differentiating into cardiomyocytes. Cardiomyocytes are the muscle cells of the heart. Cardiomyocytes make up the atria (i.e. the chambers in which blood enters the heart) and the ventricles (i.e. the chambers where blood is collected and pumped out of the heart). Just as endothelial cells and hematopoietic cells, cardiomyocytes are of mesodermal origin. For the reasons discussed herein above, in connection with the present invention the term "cardiomyocyte" refers to cells having essentially a mature cardiomyocyte phenotype which can *in vitro* and/or *in vivo* substitute the functions of *in vivo* differentiated cardiomyocytes.

The term "inhibiting NPAS4 function" designates the inhibition of the transcription factor activity of NPAS4 or a species ortholog thereof. The inhibition of the transcription factor activity of NPAS4 or a species ortholog thereof is preferably determined by measuring the inhibition of the expression of the gene encoding NPAS4 or a species ortholog thereof and/or of the downstream effector genes tal1 and/or etv2. In this respect, inhibiting NPAS4 function requires with increasing preference that the expression of the gene(s) is diminished by at least 70%, at least 80%, at least 90%, and at least 95% in the presence of the factor(s) inhibiting NPAS4 function as compared to corresponding conditions in the absence of the factor(s) inhibiting NPAS4 function. Most preferably the expression of the gene(s) is completely abolished (i.e. is no longer detectable).

It is to be understood that also in the above-described method of the third aspect of the invention in general one or more differentiation factors may be used. At least one differentiation factor is capable of inhibiting NPAS4 function. In accordance with the third aspect of the invention the additional transcription factor(s) contribute to producing cardiomyocytes from progenitor cells.

Various differentiation factors for obtaining cardiomyocytes are known in the art. For example, cardiomyocytes may be obtained by sequentially treating progenitor cells with activin A and BMP4 in a serum-free and insulin-free medium and then maintained in a serum-free medium with insulin (Jha et al. (2015), Methods Mol Biol;1299:115-31). As another example, a Gsk3 inhibitor may used in combination with a β-catenin inhibitor or a chemical Wnt inhibitor (Lia et. (2013), Nat Protoc.; 8(1):162-175). Also Fgf-signaling and in particular Fgf8 may be employed for obtaining cardiomyocytes. Accordingly the one or more additional differentiation factors are in connection with the third aspect of the invention preferably selected from a member of the Fgf-family (preferably Fgf8), activin A, BMP4, Gsk3 inhibitors, β-catenin inhibitors and a chemical Wnt inhibitor.

As demonstrated by the examples herein below Npas4-like was found by the present inventors to be essential for the formation of endothelial cells and hematopoietic cells in zebrafish. As mentioned, endothelial cells and hematopoietic cells are built from common progenitor cells called hemangioblasts. In contrast to mesodermal progenitor cells, hemangioblasts are no longer capable of differentiating into cardiomyocytes. The work of Schoenebeck et al. (2007), Dev Cell, 13:254-267 shows that expression of the putative cloche gene - which gene has only been identified as being Npas4-like in accordance with the present invention - and its downstream effectors tal1 and etv2 ensures that the mesoderm differentiates into endothelial cell and hematopoietic cells. On the other hand, the expression of hand2 ensures that the mesoderm differentiates into cardiomyocytes in zebrafish. It was found that the repression of myocardial specification by vascular and hematopoietic pathway coordinates the development of the cardiovascular system. It was furthermore found that the loss of cloche function causes an expansion of cardiogenic markers within the lateral plate mesoderm. For these reasons it can be assumed that inhibiting NPAS4 function in progenitor cells promotes their differentiation into cardiomyocytes.

In accordance with a preferred embodiment, also the method of the third aspect of the invention further comprises isolating the cardiomyocytes.

In the above-described methods of the third aspect of invention not each progenitor cell may be transformed into a cardiomyocyte for essentially the same reason discussed herein above in connection with endothelial cells and hematopoietic cells. Methods for isolating cells are well known in the state of the art and have been discussed in further detail herein above. Cardiomyocyte cell-surface markers include but are not limited to SIRPA, Cx43, and Desmin. Connexin 43 (Cx43) is the main constituent of cardiomyocyte gap junctions. Desmin is the major muscle-specific intermediate filament protein. SIRPA is a receptor-type transmembrane glycoprotein specifically expressed on cardiomyocytes.

In accordance with a further preferred embodiment of the third aspect of the invention the progenitor cells are embryonic stem cells (ESCs), induced pluripotent stem cells (iPSs), mesodermal progenitor cells, cardiomyocyte progenitor cells, or a mixture thereof.

Cardiomyocyte progenitor cells are capable of generating phenotypically and functionally competent cardiomyocytes. Cardiomyocyte progenitor cells can be isolated, for example, from cardiac surgical waste or alternatively from fetal heart tissue (Smits el al. (2009), Nat Protoc, 4(2):232-243).

In accordance with a different preferred embodiment of the third aspect of the invention the NPAS4 function is inhibited by (a) a dominant negative mutant of NPAS4 or a species ortholog thereof or a nucleic acid molecule encoding the same; (b) an aptamer, a ribozyme, an antibody, a protein drug, or a small molecule inhibitor against NPAS-4 or a species ortholog thereof; and/or (c) a siRNA, a shRNA, a morpholino, a microRNA or an antisense nucleic acid specifically interacting with a nucleic acid molecule encoding NPAS-4 or a species ortholog thereof.

Methods for introducing compounds into a cell have been discussed herein above and may also be used in connection with the third aspect of the invention.

A dominant negative mutant of NPAS4 or a species ortholog thereof is characterized by having one or more amino acid mutation(s) which mutation(s) result in a mutant NPAS4 or a species ortholog thereof that can interfere with the function of the normal (i.e. unmutated wild-type) NPAS4 or a species ortholog thereof.

Aptamers are DNA or RNA molecules that bind other molecules, such as nucleic acids, proteins, small organic compounds, and even entire organisms. A database of aptamers is maintained at http://aptamer.icmb.utexas.edu/. More specifically, aptamers can be classified as DNA or RNA aptamers or peptide aptamers. Whereas the former consist of (usually short) strands of oligonucleotides, the latter consist of a short variable peptide domain, attached at both ends to a protein scaffold. Nucleic acid aptamers are nucleic acid species that have been engineered through repeated rounds of *in vitro* selection or equivalently, SELEX (systematic evolution of ligands by exponential enrichment) to bind to various molecular targets such as small molecules, proteins, nucleic acids, and even cells, tissues and organisms. Peptide aptamers are proteins that are designed to interfere with other protein interactions inside cells. They consist of a variable peptide loop attached at both ends to a protein scaffold. This double structural constraint greatly increases the binding affinity of the peptide aptamer to levels comparable to an antibody's (nanomolar range). The variable loop length is typically comprised of 10 to 20 amino acids, and the scaffold may be any protein which has good solubility properties. Currently, the bacterial protein Thioredoxin-A is the most used scaffold protein, the variable loop being inserted within the reducing active site, which is a -Cys-Gly-Pro-Cys- loop in the wild-type protein, the two cysteine side chains being able to form a disulfide bridge. Peptide aptamer selection can be made using different systems, but the most used is currently the yeast two-hybrid system. Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications.

Non-modified aptamers are cleared rapidly from the bloodstream, with a half-life of minutes to hours, mainly due to nuclease degradation and clearance from the body by the kidneys, a result of the aptamer's inherently low molecular weight. Unmodified aptamer applications currently focus on treating transient conditions such as blood clotting, or treating organs such as the eye where local delivery is possible. This rapid clearance can be an advantage in applications such as *in vivo* diagnostic imaging. Several modifications, such as 2'-fluorine-substituted pyrimidines, polyethylene glycol (PEG) linkage, fusion to albumin or other half-life extending proteins etc. are available to scientists with which the half-life of aptamers may be extended. The aptamers of the present invention may also comprise or be composed of L-ribonucleic acids in which case they may be designated spiegelmers

The term "ribozymes" refers to RNA molecules that act as enzymes in the absence of proteins. These RNA molecules act catalytic or autocatalytic and are capable of cleaving e.g. other RNAs at specific target sites but they have also been found to catalyze the aminotransferase activity of the ribosome. Selection of appropriate target sites and corresponding ribozymes are known in the art. Examples of well-characterized small self-cleaving RNAs are the hammerhead, hairpin, hepatitis delta virus, and *in vitro*-selected lead-dependent ribozymes. The organization of these small catalysts is in contrast to that of larger ribozymes, such as the group I intron. The principle of catalytic self-cleavage has become well established in the last decades. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic antisense sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each with 6 to 8 nucleotides, are taken and the catalytic hammerhead sequence is inserted between them. Molecules of this type were synthesized for numerous target sequences. They showed catalytic activity *in vitro* and in some cases also *in vivo.* The best results are usually obtained with short ribozymes and target sequences.

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments, Fd, F(ab')₂, Fv or scFv fragments, single domain V_{H} or V-like domains, such as VhH or V-NAR-domains, as well as multimeric formats such as minibodies, diabodies, tribodies, tetrabodies or chemically conjugated Fab'-multimers. The term "antibody" also includes embodiments such as chimeric (human constant domain, non-human variable domain), single chain and humanized (human antibody with the exception of non-human CDRs) antibodies. Various techniques for the production of antibodies and fragments thereof are well known in the art and described, e.g. in Altshuler et al., 2010, ISSN 0006-2979, Biochemistry (Moscow), 2010, Vol. 75, No. 13, pp. 1584-1605. Thus, polyclonal antibodies can be obtained from the blood of an animal following immunisation with an antigen in mixture with additives and adjuvans and monoclonal antibodies can be produced by any technique, which provides antibodies produced by continuous cell line cultures. Furthermore, recombinant antibodies may be obtained from monoclonal antibodies or can be prepared *de novo* using various display methods such as phage, ribosomal, mRNA, or cell display. A suitable system for the expression of the recombinant (humanized) antibodies or fragments thereof may be selected from, for example, bacteria, yeast, insects, mammalian cell lines or transgenic animals or plants (see, e.g., US patent 6,080,560). Further, techniques described for the production of single chain antibodies (see, inter alia, US Patent 4,946,778) can be adapted to produce single chain antibodies specific for the target of this invention. Surface plasmon resonance as employed in the BIAcore system can be used to increase the efficiency of phage antibodies.

In the art a variety of protein drugs are available, which all have been designed by using wild-type proteins as scaffolds (for review, Tomlinson et al. (2004), Nature Biotechnology 22:521-522). Non-limiting examples of such protein drugs are small molecule inhibitors, which have been developed using transferrin, C-type lectins, trinectins, domain antibodies, kunitz domains, lipocalins, ankyrin repeats or the Fyn SH3 domain as protein scaffold. As detailed in Tomlinson et al. (2004), loc lit, once the suitability of a protein scaffold for the generation protein drugs is known, the protein scaffold can in principle be used to generate protein drugs to any desired target which target is in accordance with the present invention NPAS4 or a species ortholog thereof.

A small molecule inhibitor designates a low molecular weight (generally below 900 Daltons and preferably below 500 Daltons) organic compound that is in accordance with the present invention capable of inhibiting NPAS4 function. In the art several small molecule inhibitor libraries are available which can be screened by high-throughput techniques for a small molecule inhibitor having the desired function, being in accordance with the present invention the inhibition of NPAS4 or a species ortholog thereof. High-throughput screening (HTS) has become a routine method for identifying small molecule inhibitor (Inglese et al (2007), Nature Chemical Biology, 3:438-441).

The terms "antisense nucleic acid molecule", "small interfering RNAs (siRNAs)", "short hairpin RNAs (shRNAs)", "morpholinos" and "microRNAs (miRNAs)", as used herein, are known in the art and are defined in accordance with the pertinent art. Accordingly, an antisense nucleic acid molecule is a nucleic acid which is complementary to, and capable of hybridising with, the target nucleic acid. Due to the formation of the hybrid, transcription of the target gene(s) and/or translation of the target mRNA is reduced or blocked. Standard methods relating to antisense technology have been described (see, e.g., Melani et al., Cancer Res. (1991) 51:2897-2901). "Small interfering RNA (siRNA)", also known as short interfering RNA or silencing RNA, refers to a class of 18 to 30, preferably 19 to 25, most preferred 21 to 23 or even more preferably 21 nucleotide-long doublestranded RNA molecules capable of interfering with the expression of a target gene. Naturally occurring siRNAs have a well defined structure: a short double-strand of RNA (dsRNA) with 2-nt 3' overhangs on either end. Each strand has a 5' phosphate group and a 3' hydroxyl (-OH) group. siRNAs can also be exogenously (artificially) introduced into cells to bring about the specific knockdown of a target gene. Essentially any gene of which the sequence is known can be targeted based on sequence complementarity with an appropriately tailored siRNA. Exogenously introduced siRNAs may be devoid of overhangs at their 3' and 5' ends, however, it is preferred that at least one RNA strand has a 5'- and/or 3'-overhang. Preferably, one end of the double-strand has a 3'-overhang from 1-5 nucleotides, more preferably from 1-3 nucleotides and most preferably 2 nucleotides. The other end may be blunt-ended or has up to 6 nucleotides 3'-overhang. Most preferably, the siRNA is a duplexes composed of 21-nt sense and 21-nt antisense strands, paired in a manner to have 2-nt 3' overhangs on either end. The sequence of the 2-nt 3' overhang makes a small contribution to the specificity of target recognition restricted to the unpaired nucleotide adjacent to the first base pair. 2'-deoxynucleotides in the 3' overhangs are as efficient as ribonucleotides, but are often cheaper to synthesize and probably more nuclease resistant. A "short hairpin RNA (shRNA)" is a sequence of RNA that makes a tight hairpin turn that can be used to silence gene expression via RNA interference. shRNA can for example use a vector introduced into cells, in which case preferably the U6 promoter is utilized to ensure that the shRNA is always expressed. This vector is usually passed on to daughter cells, allowing the gene silencing to be inherited. The shRNA hairpin structure is cleaved by the cellular machinery into siRNA, which is then bound to the RNA-induced silencing complex (RISC). This complex binds to and cleaves mRNAs that match the siRNA that is bound to it. A "morpholino" (also known as a morpholino oligomer and as a phosphorodiamidate morpholino oligomer (PMO)), is a type of oligomer molecule (colloquially, an oligo) used in the art to modify gene expression. The molecular structure has a backbone of methylenemorpholine rings and phosphorodiamidate linkages. Morpholinos block access of other molecules to small (-25 base) specific sequences of the base-pairing surfaces of ribonucleic acid (RNA). Morpholinos are used in the art, for example, for reverse genetics by knocking down gene function. The examples herein below exemplify morpholinos against npas4-like (i.e. SEQ ID NOs 33 and 34). These morpholinos against npas4-like are preferred examples of morpholinos against npas4-like. A "microRNA (miRNA)" is a single-stranded RNA molecule that, as an endogenous RNA molecule, regulates gene expression. Binding to a complementary mRNA transcript triggers the degradation of said mRNA transcript through a process similar to RNA interference. Accordingly, miRNA may be employed as an inhibitor of NPAS4 function in accordance with the present invention.

The above-described aptamer, ribozyme, antibody, protein drug, small molecule inhibitor against NPAS-4 or a species ortholog thereof; and siRNA, shRNA, morpholino, or antisense nucleic acid against a nucleic acid molecule encoding NPAS-4 or a species ortholog thereof are all preferably specific for NPAS-4 and/or a respective species ortholog thereof. This means that all these compounds are capable of specifically inhibiting NPAS4 function and do not or essentially do not exert any off target inhibitory effects.

In accordance with a preferred embodiment of the first, second and third aspect of the invention, the method is an *ex vivo* or *in vitro* method.

The term *"ex vivo"* means that the method of the invention is performed outside an organism. "Ex *vivo"* thus refers to a method carried out on progenitor cells in an external environment, preferably with the minimum alterations of natural conditions. The term *"in vitro"* means that the method is performed with isolated progenitor cells that have obtained from an organism, preferably a mammal.

In a fourth aspect the present invention relates to a cell produced by the method of the first, second or third aspect of the invention.

As is evident from the above, the cell is in accordance with the first aspect of the invention an endothelial cell, in accordance with the second aspect of the invention a hematopoietic cell, and in accordance with the third aspect of the invention a cardiomyocyte. As also discussed herein above, the endothelial cell, hematopoietic cell, and cardiomyocyte of the invention on the one hand essentially display the cellular function and essentially have the mature phenotype of an endothelial cell, hematopoietic cell, and cardiomyocyte, respectively, but on the other hand are in general not 100% identical to the corresponding *in vivo* differentiated cell-type. For this reason it can be assumed that the cells established by the methods of the present invention are unique and not availabe from the prior art.

In a fifth aspect the present invention relates to a nucleic acid molecule encoding a transcription factor having Npas4-like function, wherein the nucleic acid molecule is selected from the group consisting of (i) a nucleic acid molecule encoding the amino acid sequence of SEQ ID NO: 25; (ii) a nucleic acid molecule comprising or consisting of SEQ ID NO: 32; (iii) a nucleic acid molecule encoding an amino acid sequence being at least 70% identical to SEQ ID NO: 25; (iv) a nucleic acid molecule encoding an amino acid sequence comprising at least 600 amino acids, wherein the N-terminal portion of said polypeptide is at least 80% identical to the N-terminal half of the amino acid sequence of SEQ ID NO: 32; (v) a nucleic acid molecule encoding an amino acid sequence being at least 60% identical to the amino acid sequence of SEQ ID NO: 25 outside the bHLH domain, the PAS1 domain and the PAS2 domain, being at least 90% identical to the amino acid sequence of SEQ ID NO: 25 inside the bHLH domain, and being at least 80% identical to the amino acid sequence of SEQ ID NO: 25 inside the PAS1 domain and the PAS2 domain; or (vi) a vector comprising the nucleic acid molecule of any one of (i) to (v).

The term "a transcription factor having Npas4-like function" means that the transcription factor is capable of the induction of the expression of the gene tal1 and/or the gene etv2, preferably of tal1 and/or etv2 from zebrafish. The examples herein below demonstrate that tal1 and etv2 are downstream of Npas4-like and that in the absence of Npas4-like no tal1 and etv2 expression is observed.

As mentioned, the amino acid sequence of SEQ ID NO: 25 as recited in item (i) is the amino acid sequence of the Npas4-like protein from zebrafish. The nucleotide sequence of SEQ ID NO: 32 as recited in item (ii) is the nucleotide sequence of the gene encoding the Npas4-like protein from zebrafish. The complete cDNA sequence of the Npas4-like gene including the start codon, stop codon and untranslated regions is shown in SEQ ID NO: 33.

The amino acid sequence of item (iii) above is with increasing preference at least 75%, at least 80%, at least 85%, at least 90%, at least 92.5%, at least 95%, at least 98% and at least 99% identical to the amino acid sequence of SEQ ID NO: 25.

The amino acid sequence of item (iv) above is with increasing preference at least 85%, at least 90%, at least 92.5%, at least 95%, at least 98% and at least 99% identical to the N-terminal half of amino acid sequence of SEQ ID NO: 25. Again, the term "N-terminal half" means the first 50% of the entire amino acid sequence of SEQ ID NO: 25. SEQ ID NO: 25 comprises 647 amino acids and the "N-terminal half" consequently consists of amino acids 1 to 324 SEQ ID NO: 1 (noting that .5 values get rounded up). The polypeptide of item (iii) comprises, preferably at least 625 amino acids, more preferably at least 640 amino acids and most preferably at least 645 amino acids. Combinations of the preferred lengths and % identities are also comprised by the present invention, such as at least 92.5% and at least 625 amino acids, at least 95% and at least 640 amino acids, and at least 98% ,and at least 645 amino acids. The number of amino acids of the "N-terminal portion" is preferably up to ± 20% of, more preferably up to ± 10% of, even more preferably up to ± 5% and most preferably corresponds to the number of amino acids of the N-terminal half.

The amino acid sequence of item (v) above is with increasing preference at least 70%, at least 80%, at least 90%, at least 95%, at least 98% and at least 99% identical to an amino acid sequence of SEQ ID NO: 25 outside the bHLH domain, the PAS1 domain and the PAS2 domain. Moreover, the polypeptide of item (iv) above is with increasing preference at least 92.5%, at least 95%, at least 98% and at least 99% identical to an amino acid sequence of SEQ ID NO: 25 inside the bHLH domain. Further, the polypeptide of item (iv) above is with increasing preference at least 85%, at least 90%, at least 95%, at least 98% and at least 99% identical to an amino acid sequence of SEQ ID NO: 25 inside the PAS1 domain and the PAS2 domain. It is to be understood that the present invention also encompasses any combination of the listed sequence identities (i) outside the bHLH domain, the PAS1 domain and the PAS2 domain, (ii) inside the bHLH domain, and (iii) inside the PAS1 domain and the PAS2 domain. Non-limiting examples are (i) at least 70%, (ii) at least 92.5%, and (iii) at least 85%; (i) at least 80%, (ii) at least 95%, and (iii) at least 90%; and (i) at least 85%, (ii) at least 98%, and (iii) at least 95%; and (i) at least 90%, (ii) at least 99%, and (iii) at least 98%.

Suitable vectors as recited in item (vi) which may comprise the nucleic acid molecule of any one of the above items (i) to (v) are discussed herein above in connection with the first and second aspect of the invention.

The examples of the present invention were conducted using the zebrafish cloche mutant. The zebrafish cloche mutant with effects in both the endothelial and hematopoietic lineages at a very early embryonic stage has already been known for over 20 years (Stainier et al., 1995, loc. lit.). Due to the unique phenotype displayed by *cloche* mutants, both the mutant and the genomic *cloche* locus have been the subject of intense investigation since then (Liao et al., 1997, Development 124, 381-389; Liao et al., 1998, Genes Dev 12, 621-626; Sumanas et al., 2005, Blood 106, 534-541; Schoenebeck et al., 2007, Dev Cell 13, 254-67; Nissim et al., 2014, Dev Cell 28, 423-437; Fortuna et al., 2015, Cell Rep 11, 1786-1796; Rasmussen et al., 2015, J Neurosci 35, 559-570). The *cloche* locus was mapped to one of the telomeres of chromosome 13 (Liao et al., 2000, Development 127, 4303-13). However, all of the numerous attempts of isolating the putative cloche gene have failed within the last 20 years. For example, the recent publication Leshchiner et al. (2012), Genome Res; 22(8):1541-1548 reports on the further resolution of the mutation region harbouring the putative cloche gene but evidently failed to isolate and sequence the gene.

The reason for the various failures to isolate the cloche gene is the above-discussed telomeric location of the cloche locus along with the lack of a reliable genome assembly at chromosome ends. Genome assembly at chromosome ends is particularly difficult due to the presence of telomeres. Telomeres have an unusual DNA sequence composition and organization that makes them particularly difficult to isolate and analyse. Telomers can be found in majority of organisms, as diverse as protozoan, fungi, fish, mammals and plants. All vertebrates have specific telomeric repeat sequence. Telomers are generally highly repetitive loci that show GT or AC repeats in intergenic or intronic sequences.

The inventors of the present invention finally and surprisingly succeeded in cloning and sequencing the putative cloche gene, being termed herein npas4-like (or npas4I) based on its human ortholog NPAS4. The approach taken by the inventors which finally resulted in the identification of the transcription factor Npas4-like required the design of a cascade of several inventive and laborious approaches.

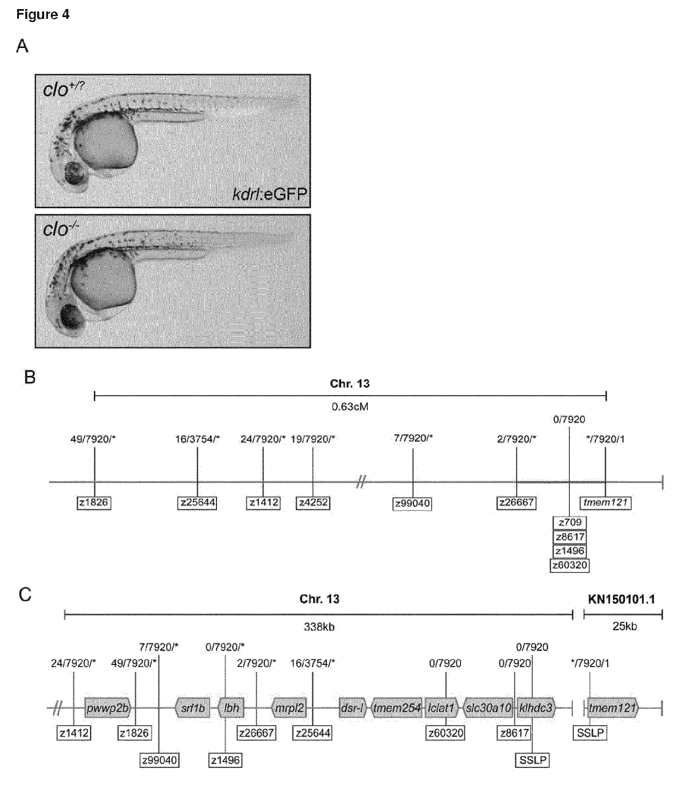

In order to provide higher genetic resolution to the *cloche* region and narrow down the critical interval that contains the *cloche* mutation, the inventors initially genotyped in addition to the 2359 known cloche mutant embryos 5561 further cloche mutant embryos for a total of 7920 which allowed them to identify more zero recombinant markers as well as very tightly linked flanking markers (see Fig. 4B). Searching the available genomic databases led to the identification of several genes in the *cloche* locus including *lclat* (aka *lycat*) (Xiong et al., 2008, Circulation Research; 102:1057-1064*), lbh, klhdc3,* and *inpp5a.* The inventors sequenced these genes looking for severe molecular lesions and also carried out functional studies including morpholino knockdowns and mRNA rescues, but again failed to isolate the putative *cloche* gene.

The inventors hypothesized on the basis of these initial failures that the physical genomic assembly of the *cloche* region may not match any available genetic map of the zebrafish genome and that consequently additional yet unknown genes may be located in the cloche region. In order to test this hypothesis and to identify these missing genes, (i) the deletion allele of *cloche m39* (clo^{m39}) and (ii) the prior art knowledge that the gene expression profile of endothelial and hematopoietic cells in early embryogenesis includes *etv2* and *tal1* were employed. Extensive bioinformatics analysis of about 40 million sequence reads then led to the identification of several new candidate genes as well as previously identified genes in the *cloche* region (Fig. 1F).

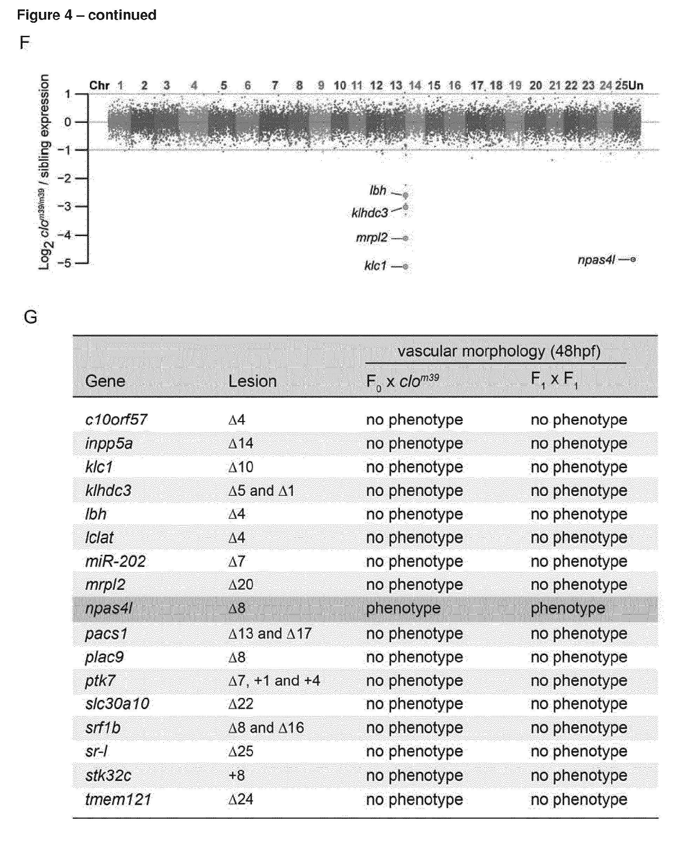

Each of these novel candidate genes was then mutated and a total of 17 individual zebrafish mutants were tested. Only one out of these 17 mutants, namely the npas4-like mutant was not able to complement the clo^{m39} allele, and showed a vascular phenotype (Fig. 4G). Confirming the hypothesis of the inventors, the npas4-like gene did not map to the physical assembly of the cloche region but to a small unassigned scaffold (KN150101.1, Fig. 4C).

Further, inventive efforts were required in order to obtain the full-length npas4-like sequence. Initially it was only possible to identify a sequence translating into a continuous reading frame of 293 amino acids that showed an identity of 33% over a length of 113 amino acids to the C-terminal region of Npas4 from *Cynoglossus semilaevis.* The inventors finally succeeded to assemble the full-length Npas4-like protein of 647 amino acids (SEQ ID NO: 25) by RACE of the 5' end of the cDNA and RACE to the 3' end of the cDNA.

In a sixth aspect the present invention relates to a host cell comprising the nucleic acid molecule of the fifth aspect of the invention.

In a preferred embodiment, the host cell is a progenitor cell as defined herein above, more preferably in connection with the first and second aspect of the invention. Such a host cell may be employed in the method of the first and second aspect of the invention.

In another preferred embodiment, the host cell is as an isolated cell which may be part of a cell culture. Such a host cell may be used for recombinantly producing the nucleic acid molecule of the fifth aspect of the invention, as will be further detailed herein below. Suitable prokaryotic host cells comprise e.g. bacteria of the species Escherichia, Bacillus, Streptomyces and Salmonella typhimurium. Suitable eukaryotic host cells are e.g. fungal cells, inter alia, yeasts such as Saccharomyces cerevisiae or Pichia pastoris or insect cells such as Drosophila S2 and Spodoptera Sf9 cells and plant cells as well as mammalian cells. Appropriate culture media and conditions for the above-described host cells are known in the art. Mammalian host cells that could be used include, human Hela, HEK293, H9 and Jurkat cells, mouse NIH3T3 and C127 cells, Cos 1, Cos 7 and CV1, quail QC1-3 cells, mouse L cells and Chinese hamster ovary (CHO) cells. Also within the scope of the present invention are primary mammalian cells such as mouse embryonic fibroblasts (MEF). Alternatively, the nucleic acid molecule of the fifth aspect of the invention can be expressed in stable cell lines that contain a gene construct with said nucleic acid molecule integrated into a chromosome.

In a more preferred embodiment, said cell is a primary cell or primary cell line. Primary cells are cells which are directly obtained from an organism. Suitable primary cells are in particular the progenitor cells discussed herein above, in connection with the first, second and third aspect of the invention.

In a seventh aspect the present invention relates to a method for producing a transcription factor having Npas4-like function comprising (a) culturing the host cell of the sixth aspect the invention, and (b) recovering the polypeptide encoded by the nucleic acid molecule of the fifth aspect of the invention.

As discussed, Npas4-like is the zebrafish ortholog of human NPAS4. For this reason a transcription factor having Npas4-like is a particular example of the above-discussed transcription factor having the function of an ortholog of human NPAS4. Therefore the transcription factor having Npas4-like function produced by the method of the seventh aspect of the invention is preferably employed in the methods of the first and second aspect of the invention as the transcription factor having the function of an ortholog of human NPAS4. It is furthermore to be understood that said polypeptide may comprise additional N- or C-terminal amino acid sequences. Such polypeptides are sometimes also referred to as fusion proteins. Particular examples of fusion partners have been discussed above.

A large number of suitable methods exist in the art to produce polypeptides (or fusion proteins) in appropriate hosts. If the host is a unicellular organism one can revert to a variety of available culture conditions. Conveniently, the produced protein is harvested from the culture medium, lysates of the cultured organisms or from isolated (biological) membranes by established techniques. In the case the host is a cell which is part of or derived from a part of the organism, for example said host cell may be the harvestable part of an organ or tissue. A preferred method involves the recombinant production of protein in hosts. For example, nucleic acid sequences comprising the polynucleotide according to the invention can be synthesized by PCR, inserted into an expression vector. Subsequently a suitable host may be transformed with the expression vector. Thereafter, the host is cultured to produce the desired polypeptide(s), which is/are isolated and purified. Protein isolation and purification for recovering the polypeptide can be achieved by any one of several known techniques; for example and without limitation, ion exchange chromatography, gel filtration chromatography and affinity chromatography, high pressure liquid chromatography (HPLC), reversed phase HPLC, and preparative disc gel electrophoresis. Protein isolation/purification techniques may require modification of the proteins of the present invention using conventional methods. For example, in case a histidine tag is added to the protein as purification tag a nickel column may be used. Further possible modifications for purification have been discussed herein above in connection with the vector of the invention. An alternative method for producing the polypeptide of the invention is *in vitro* translation of mRNA. Suitable cell-free expression systems for use in accordance with the present invention include rabbit reticulocyte lysate, wheat germ extract, canine pancreatic microsomal membranes, E. coli S30 extract, and coupled transcription/translation systems such as the TNT-system (Promega). These systems allow the expression of recombinant polypeptides upon the addition of cloning vectors, DNA fragments, or RNA sequences containing coding regions and appropriate promoter elements. In addition to recombinant production, the polypeptide of the invention may be produced synthetically, e.g. by direct peptide synthesis using solid-phase techniques (Stewart et al. (1969) Solid Phase Peptide Synthesis; Freeman Co, San Francisco; Merrifield, J. Am. Chem. Soc. 85 (1963), 2149-2154). Synthetic protein synthesis may be performed using manual techniques or by automation. Automated synthesis may be achieved, for example, using the Applied Biosystems 431A Peptide Synthesizer (Perkin Elmer, Foster City CA) in accordance with the instructions provided by the manufacturer. Various fragments may be chemically synthesized separately and combined using chemical methods to produce the full-length molecule. As indicated above, chemical synthesis, such as the solid phase procedure described by Houghton Proc. Natl. Acad. Sci. USA (82) (1985), 5131-5135, can be used. Furthermore, the polypeptide (protein), fragments of the protein or the fusion protein of the invention may be produced semi-synthetically, for example by a combination of recombinant and synthetic production.

In a eighth aspect the present invention relates to a polypeptide encoded by the nucleic acid molecule of the fifth aspect of the invention or produced by the method of the seventh aspect of the invention.

It is to be understood that said polypeptide of the eighth aspect of the invention comprises or is a transcription factor having Npas4-like function as defined herein above. As discussed, Npas4-like is the zebrafish ortholog of human NPAS4. For this reason the definition of the term "polypeptide" provided herein above in connection with the orthologs of human NPAS4 which may be used in the methods of the first and second aspect of the invention also applies to the polypeptide of the eighth aspect of the invention. It also has to be understood that the polypeptide of the eighth aspect of the invention is preferably employed in the methods of the first and second aspect of the invention.

In a ninth aspect the present invention relates to a pharmaceutical composition comprising (i) the polypeptide and/or the nucleic acid molecule as defined in connection with the first and second aspect of the invention; or the polypeptide, the nucleic acid molecule, and/or the vector as defined in connection with the first and second aspect of the invention; and/or (ii) the cell of the fourth aspect of the invention, the nucleic acid molecule of the fifth aspect of the invention, the host cell of the sixth aspect of the invention, the polypeptide of the eighth aspect of the invention, or a combination thereof.

In accordance with the present invention, the term "pharmaceutical composition" relates to a composition for administration to a patient, preferably a human patient. The pharmaceutical composition of the invention comprises the compounds recited above. It may, optionally, comprise further molecules capable of altering the characteristics of the compounds of the invention thereby, for example, stabilizing, modulating and/or activating their function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician.

Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.
Furthermore, if for example said compound is an polypeptide or nucleic acid molecule the total pharmaceutically effective amount of pharmaceutical composition administered will typically be less than about 75 mg per kg of body weight, such as for example less than about 70, 60, 50, 40, 30, 20, 10, 5, 2, 1, 0.5, 0.1, 0.05, 0.01, 0.005, 0.001, or 0.0005 mg per kg of body weight. More preferably, the amount will be less than 2000 nmol of polypeptide or nucleic acid molecule (per kg of body weight, such as for example less than 1500, 750, 300, 150, 75, 15, 7.5, 1.5, 0.75, 0.15, 0.075, 0.015, 0.0075, 0.0015, 0.00075 or 0.00015 nmol of polypeptide or nucleic acid molecule per kg of body weight.

The length of treatment needed to observe changes and the interval following treatment for responses to occur vary depending on the desired effect. The particular amounts may be determined by conventional tests which are well known to the person skilled in the art.

In a tenth aspect the present invention relates to the polypeptide and/or the nucleic acid molecule as defined in connection with the first and second aspect of the invention; or the polypeptide, the nucleic acid molecule, and/or the vector as defined in connection with the first and second aspect of the invention; or the cell of the fourth aspect of the invention, the nucleic acid molecule of the fifth aspect of the invention, the host cell of the sixth aspect of the invention, the polypeptide of the eighth aspect of the invention, or a combination thereof for use in (i) inducing angiogenesis, or (ii) treating a disease selected from atherosclerotic diseases, peripheral arterial disease, wound healing disorders, stroke, infertility, scleroderma, ulcers, diabetic vascular complications, erectile dysfunction and heart disease.

The present invention likewise pertains to a method of (i) inducing angiogenesis in patient in need thereof, or (ii) treating a patient suffering from a disease selected from atherosclerotic diseases, peripheral arterial disease, wound healing disorders, stroke, infertility, scleroderma, ulcers, diabetic vascular complications, erectile dysfunctionand heart disease by administering to said patient an therapeutically active amount of the polypeptide and/or the nucleic acid molecule as defined in connection with the first and second aspect of the invention; or the polypeptide, the nucleic acid molecule, and/or the vector as defined in connection with the first and second aspect of the invention; or the cell of the fourth aspect of the invention, the nucleic acid molecule of the fifth aspect of the invention, the host cell of the sixth aspect of the invention, the polypeptide of the eighth aspect of the invention, or a combination thereof.

As commonly known, "angiogenesis" is the physiological process through which new blood vessels form. Inducing angiogenesis is consequently a means for treating diseases characterized by either poor vascularisation or abnormal angiogenesis. Several diseases, such as ischemic chronic wounds, are the result of failure or insufficient blood vessel formation and can be treated by a local expansion of blood vessels. Moreover, pro-angiogenic therapies are currently explored as options to treat cardiovascular diseases. A large number of preclinical studies have been performed with protein-, gene- and cell-based therapies in animal models of cardiac ischemia, as well as models of peripheral artery disease. A non-limiting list of diseases which are expected to benefit from inducing angiogenesis is: Atherosclerotic diseases, peripheral arterial disease, wound healing disorders, stroke, infertility, scleroderma, ulcers, diabetic vascular complications, erectile dysfunctionand heart disease.

The figures show.
**Figure 1** Is an alignment of the amino acid sequence of human NPAS4 with the respective primate orthologs from chimpanzee, gorilla, orang-utan, gibbon, macaque, vervet, marmoset, and bushbaby. In the alignment the sequence of the bHLH domain is double underlined, the sequence of the PAS1 domain is underlined and the sequence of the PAS2 domain is dashed underlined.
**Figure 2 (A)** Is an alignment of the amino acid sequence of human NPAS4 with the respective rodentia orthologs from mouse, rat, guinea pig, pika and squirrel. **(B)** Is an alignment of the amino acid sequence of human NPAS4 with the respective mammalian orthologs from megabat, pig, armadillo, opossum, cat, cow, elephant, Tasmanian devil, horse and sheep. In Figure 2 and (A) and (B) the sequence of the bHLH domain is double underlined, the sequence of the PAS1 domain is underlined and the sequence of the PAS2 domain is dashed underlined.
**Figure 3** Is an alignment of the amino acid sequence of human NPAS4 with the respective mammalian orthologs from megabat, pig, armadillo, opossum, cat, cow, elephant, Tasmanian devil, horse and sheep. In the alignment the sequence of the bHLH domain is double underlined, the sequence of the PAS1 domain is underlined and the sequence of the PAS2 domain is dashed underlined.

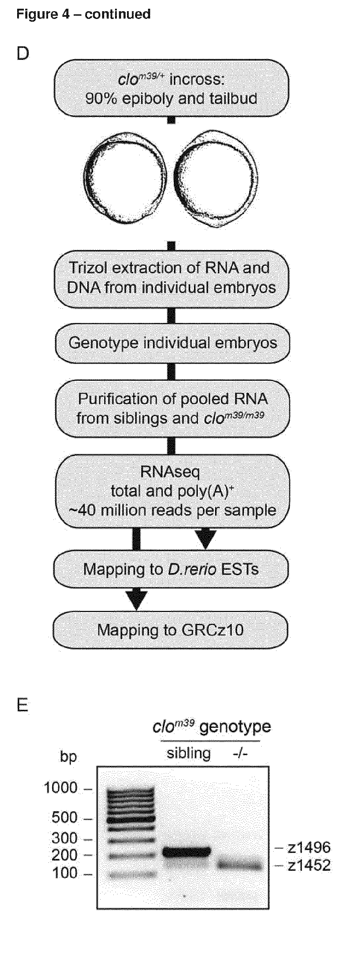

**Figure 4** Identification and analysis of candidate genes in the *cloche* locus. **(A)** Lateral views of *Tg(kdrl:EGFP)* sibling and *clo^{s5}* mutant embryos at 30 hpf. **(B)** Genetic map of the *cloche* locus on chromosome 13 showing meiotic recombination events for the indicated SSLP markers, with the critical region marked in red. **(C)** Physical map of the *cloche* locus based on GRCz10, including a 338kb sub-telomeric region of linkage group 13, which contains more than 25 gaps, and the 25kb unassigned scaffold, KN150101.1. Meiotic recombination events determined by genetic mapping are shown for the indicated markers. **(D)** Overview of the individual steps used to identify novel candidate genes in the *cloche* locus using the *clo^{m39}* deletion allele. **(E)** Genotyping of sibling and *clo^{m39}* mutant embryos using the SSLP markers z1496 (which is deleted in *clo^{m39}* mutants) and z1452 (which is not deleted in *clo^{m39}* mutants). **(F)** RNA-Seq expression differences for genes in *clo^{m39}* mutant versus wild-type sibling embryos, sorted by chromosomal position. **(G)** Table of candidate genes and the respective lesion(s) generated using TALEN or CRISPR/Cas9 technologies. Vascular morphology at 48 hpf was assessed by examining *Tg(kdrl*:EGFP) expression in embryos from F₀ to *clo*^{*m39*/+} as well as F₁ heterozygous matings.

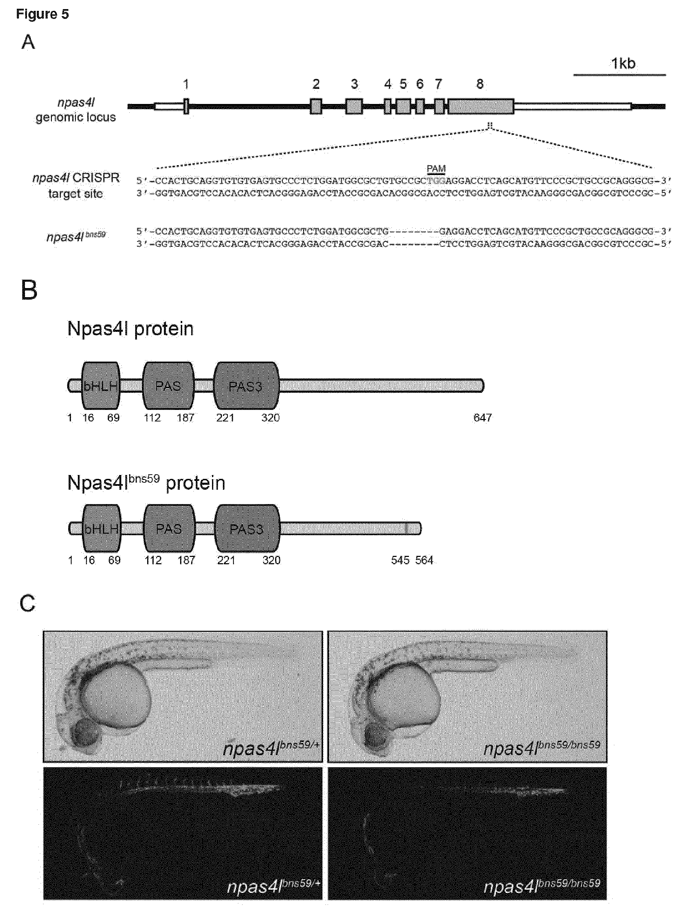

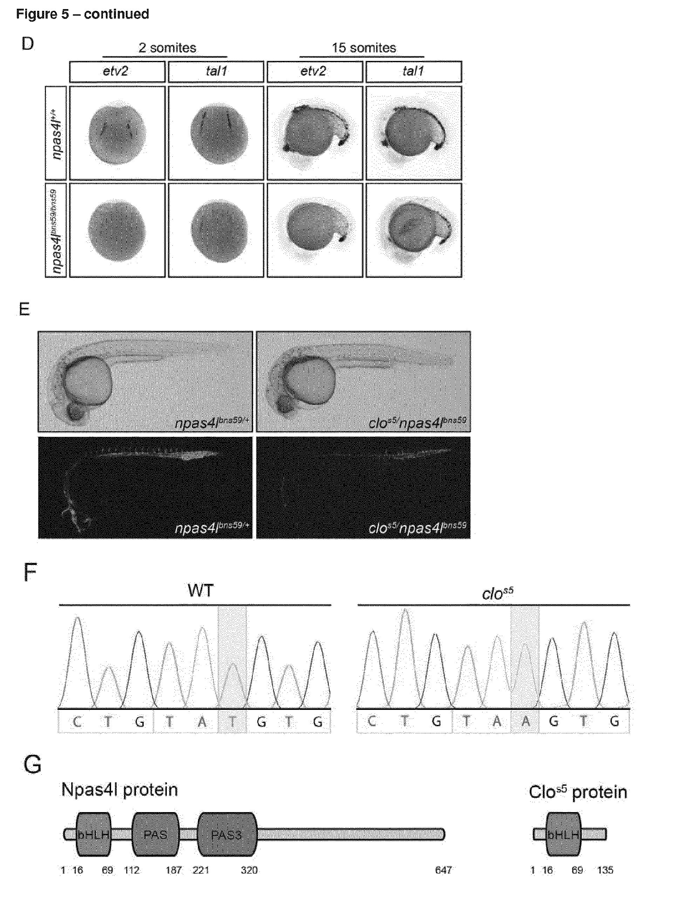

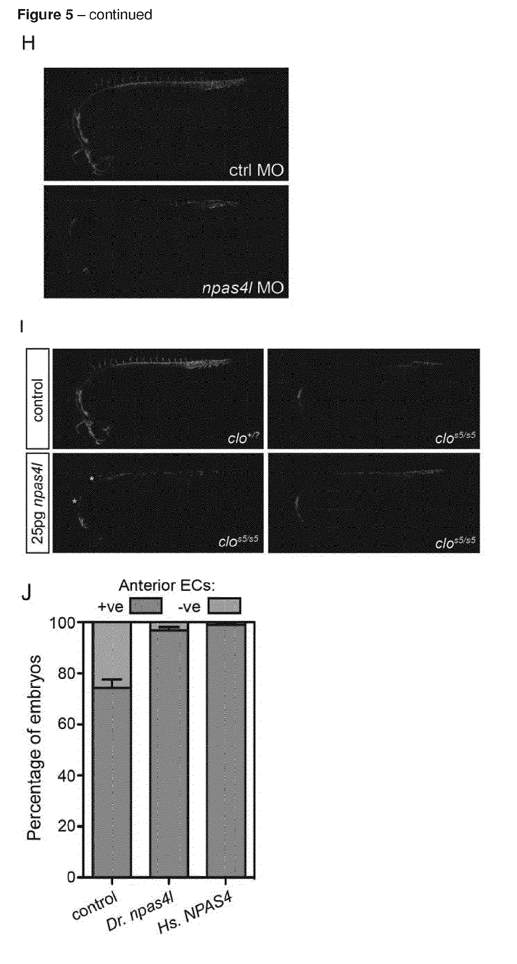

**Figure 5** *npas4I* is *cloche.* **(A)** Schematic representation of the *npas4l* genomic locus showing the CRISPR target site and the Δ8bp *npas4I^{bns59}* allele. **(B)** Illustration of the protein structures of Npas4I and Npas4I^{bns59}; the Δ8bp *npas4I^{bns59}* mutation leads to a premature stop codon after 19 missense amino acids starting at amino acid 545. **(C)** Brightfield and confocal projections of *Tg(kdrl:eGFP)* sibling and *npas4I*^{*bns59*/*bns59*} embryos at 28 hpf. **(D)** Whole-mount *in situ* hybridization showing the expression of *etv2* and *tal1* at 2 and 15-somite stages in *npas4I*^{*+*/+}and *npas4I*^{*bns59*/*bns59*} embryos. **(E)** The *npas4I^{bns59}* mutation fails to complement the *clo^{s5}* mutation; 28 hpf *Tg(kdrl:eGFP)* embryos shown. **(F)** A single base mutation in Y136 leads to a premature stop codon in the *clo*^{s5} allele. **(G)** Illustration of the protein structures of Npas4I and Clo^{s5}. **(H)** Confocal projections of 26 hpf *Tg(kdrl:eGFP)* embryos injected with control or *npas4l* morpholinos. **(I)** Confocal projections of 28 hpf *Tg(kdrl:eGFP) clo*^{*+*/?}, *clo*^{s5/s5} and *clo*^{s5/s5} embryos injected with 25pg *npas4l* mRNA; 2 different *npas4l* mRNA-injected *clo*^{s5/s5} embryos are shown in the images below. The yellow asterisk marks anterior endothelium and the white asterisk marks an anterior intersegmental vessel in a *clo*^{s5/s5} embryo injected with *npas4l* mRNA. The anterior most *Tg(kdrl:eGFP)* expression is non-vascular expression in pharyngeal arch endoderm. **(J)** Quantification of mRNA rescue of *clo*^{s5} mutants; 25pg of zebrafish *npas4l* or human *NPAS4* mRNA was injected into one-cell stage embryos and the presence of anterior endothelial cells based on *Tg(kdrl:eGFP)* expression was recorded at 28 hpf.

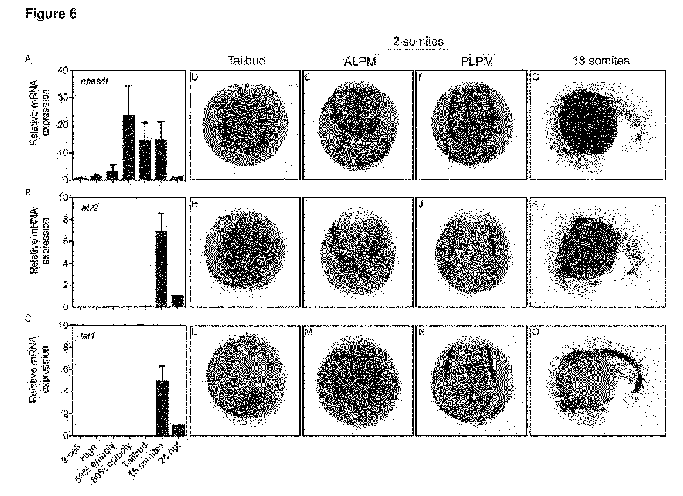

**Figure 6** *npas4l* is expressed in endothelial and hematopoietic precursor cells and precedes the expression of the earliest endothelial and blood markers *etv2* and *tall:* **(A-C)** RT-qPCR at various developmental stages for *npas4I, etv2* and *tal1*, normalized to 24 hpf. **(D-O)** Whole-mount *in situ* hybridization for *npas4I, etv2* and *tal1* at tailbud, 2- and 18-somite stages; the posterior part of the tailbud stage embryos is shown in D, H and L. ALPM, anterior lateral plate mesoderm; PLPM, posterior lateral plate mesoderm. Asterisk marks a unique rostromedial population of *npas4l* positive cells. Error bars represent SEM (n=3 biological replicates).

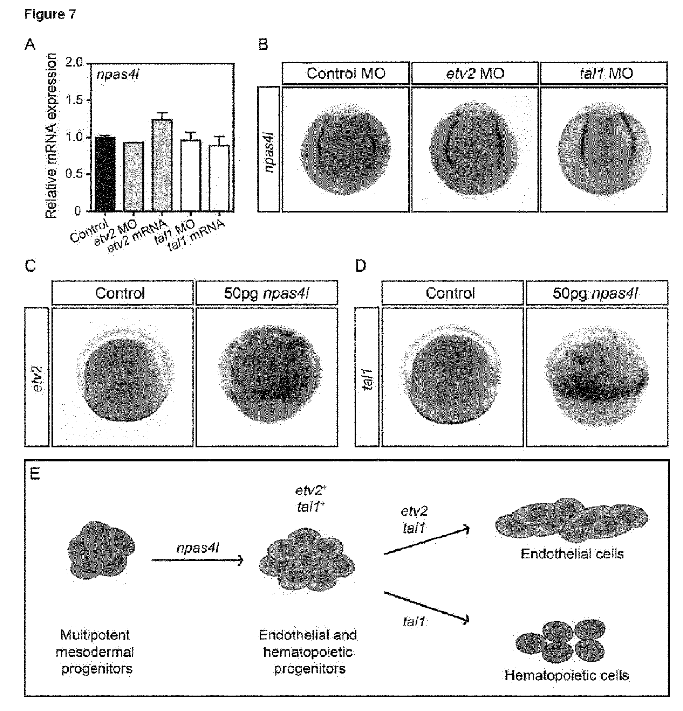

**Figure 7** Npas4I is a master regulator of the endothelial differentiation cascade: **(A)** RT-qPCR analysis of *npas4l* expression in tailbud stage embryos injected with *etv2* MO, *etv2* mRNA, *tal1* MO or *tal1* mRNA, normalized to uninjected embryos. **(B)** Whole-mount *in situ* hybridization for *npas4l* expression at the 2-somite stage in control, *etv2* and *tal1* morphant embryos. **(C-D)** Whole-mount *in situ* hybridization for *etv2* **(C)** and *tal1* **(D)** at shield stage in control and *npas4l* mRNA injected wild-type embryos. **(E)** Schematic illustration of Npas4I function; *cloche*/*npas4*/ is sufficient and necessary for the expression of early endothelial and hematopoietic markers such as *etv2* and *tal1.* Error bars represent SEM (n=3 biological replicates).

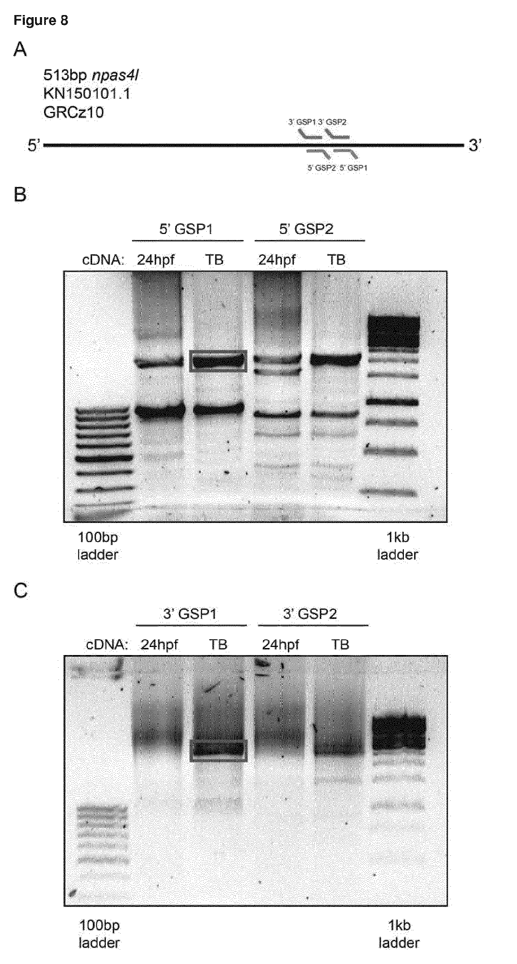

**Figure 8** 5' and 3' RACE PCR for *npas4I*: **(A)** Primer design was based on a 513bp fragment from GRCz10 that is deleted in the *clo^{m39}* allele. Agarose gels showing the products of 5' **(B)** and 3' (C) RACE PCRs using cDNAs generated from 24 hpf or tailbud mRNAs. Multiple fragments were cloned for sequencing and bands that extended the *npas4l* sequence to the 5' **(B)** and 3' (C) are highlighted in blue boxes.

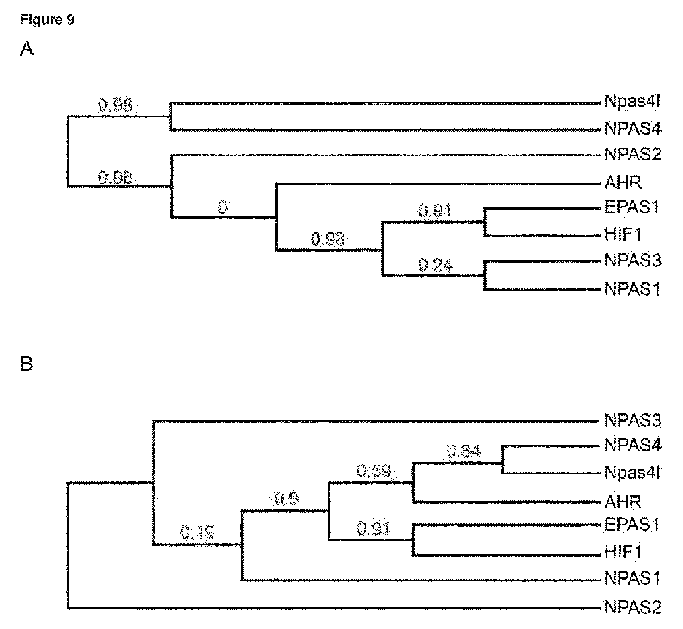

**Figure 9** Phylogenetic analysis of Cloche/Npas4I: Phylogenetic tree based on the amino acid sequence of the bHLH domain **(A)** and the first PAS domain **(B)** of the human proteins NPAS1-NPAS4, HIF1, EPAS1 and AHR together with the protein encoded by *cloche.*

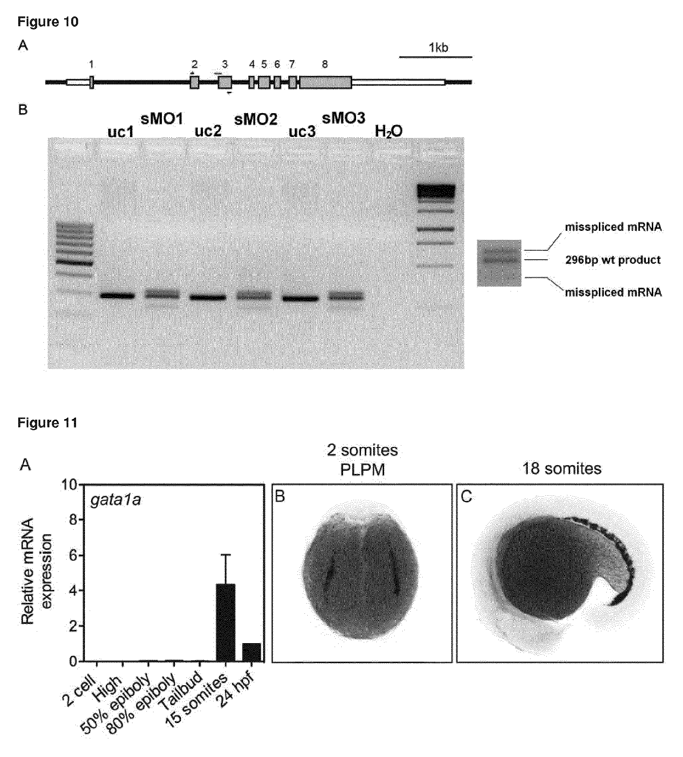

**Figure 10** Morpholino knockdown of *npas4I:* Knockdown of Npas4I was achieved by co-injecting an ATG morpholino targeting the *npas4l* pre-mRNA at its translational start site and a splice morpholino targeting the intron-exon splice site I2E3. **(A)** Schematic representation of the *npas4l* genomic locus including the I2E3 splice morpholino (red) and the primers used to test for splicing defects (black). **(B)** Analysis of the efficiency of the splice morpholino by RT-PCR shows a significant reduction of the *npas4I* wild-type mRNA at tailbud stage after injecting 6 ng of the I2E3 morpholino at the one cell stage (3 different experiments, sMO1-sMO3 are shown). uc= uninjected control, sMO=splice morpholino injected, H₂O= water control. Size of wild-type product = 296bp.
**Figure 11** *gata1* expression during early development: **(A)** RT-qPCR at various developmental stages for *gata1.* Whole mount *in situ* hybridization for *gata1* on wild-type embryos at 2 **(B)** and 18 somite stages **(C).**

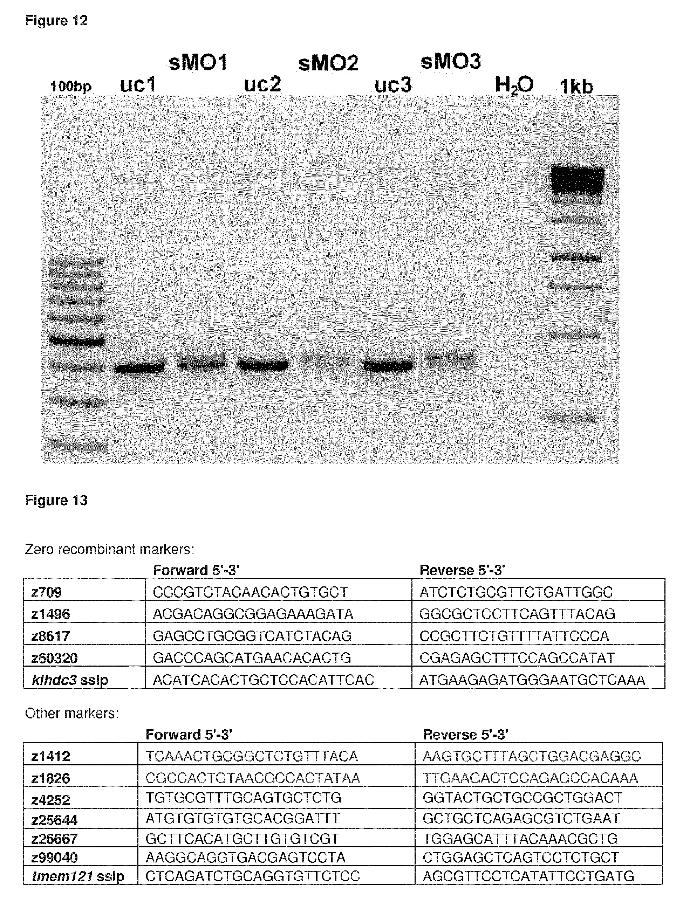

**Figure 12** Morpholino knockdown of *tal1:* Knockdown of Tal1 was achieved by injecting a previously published splice morpholino (Dooley et al. (2005), Dev Biol., 15; 277(2):522-36). **(B)** Analysis of the efficiency of the splice morpholino by RT-PCR shows a significant reduction of the *tal1* wild-type mRNA at the 2 somite stage after injecting 6.5 ng of the morpholino at the one cell stage. uc= uninjected control, sMO=splice morpholino injected, H₂O= water control. Size of wild-type product = 401 bp.
**Figure 13** Genotyping primer sequences used herein for genetic mapping.

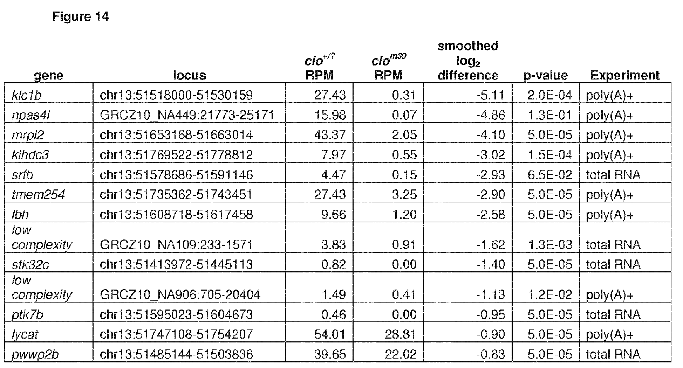

**Figure 14** Genomic loci with reduced expression in *clo^{m39}* mutants - mapping of sequencing reads from total and polyA enriched mRNA of *clo^{m39}* mutants and wild-type siblings identified 13 genomic loci of interest with reduced expression in *clo^{m39}* mutants. 11 of these loci correspond to identifiable genes (coding sequences/conserved domains). 10 of 13 loci are annotated in the telomeric region of Chr. 13, the others on unassembled scaffolds, including KN150101.1 (GRCZ10_NA449), a scaffold that contains an sslp marker (*tmem121*) with strong linkage to the *cloche* mutation and the 3'end of the *npas4I* gene.

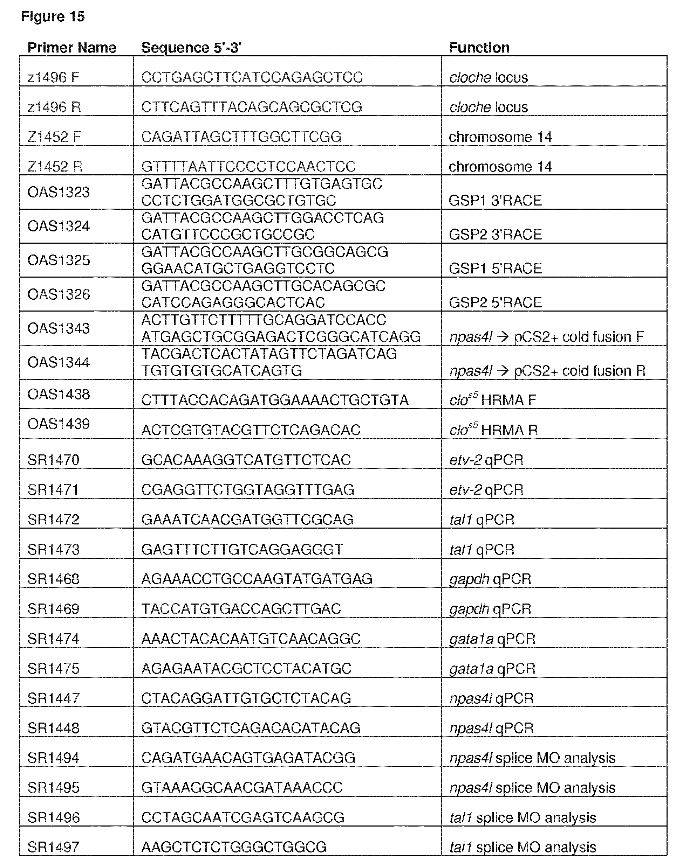

**Figure 15** Cloning and qRT-PCR Primers used herein listed by name, sequence, and function. The examples illustrate the invention.

### Example 1 - Material and Methods

### Zebrafish handling

All zebrafish husbandry was performed under standard conditions in accordance with institutional (UCSF and MPG) and national ethical and animal welfare guidelines. Mutant lines were generated in the *Tg(kdrl:GFP)^{s843}* background (Jin et al., 2005, Development;132:5199-209). Complementation tests were performed with *clo*^{s5} and *clo*^{m39} mutant lines. Overexpression studies were performed in AB embryos.

### Genetic mapping of the clo^{s5} allele and RNA-seq analysis of clo^{m39}

For genetic mapping, fine mapping of the *clo^{s5}* allele was performed by genotyping homozygous mutant embryos with simple sequence length polymorphisms (SSLPs) identified in the locus. Individual genotyping primer sequences can be found in Fig. 13. For RNA-seq analysis, individual embryos from *clo*^{m39} heterozygous intercrosses were collected at 90% epiboly and tailbud. Trizol extraction was perfomed on individual embryos to yield DNA and RNA. Briefly, single embryos were homogenized in Trizol reagent (Invitrogen) and phases were separated with chloroform. For DNA extraction, DNA was precipitated from the organic phase with ethanol, washed twice with 0.1M sodium citrate in 10% ethanol and once with 75% ethanol. The SSLP markers z1496 (0/7920) and z1452 (chr.16) were used for genotyping (Fig. 15). Embryos positive for the z1452 PCR product but negative for z1496 were considered homozygous for the *clo^{m39}* deletion. Embryos positive for both products were considered wild-type siblings. For RNA extraction, the aqueous phase extracted from sibling or *clo*^{*m39*/*m39*} embryos was pooled separately. RNA was precipitated using isopropanol and washed with 70% ethanol. Genomic DNA was removed by DNase treatment and total RNA was isolated using the Zymo Research RNA Clean & Concentrator kit. Random primed libraries were prepared from either total (DSN) or polyA selected RNA with an insert size of 200bp. Paired end sequencing was performed on an Illumina HighSeq 2000 instrument resulting in -40 million reads per condition.

### RNA-Seq data processing

Data from poly(A)+ and total RNA sequencing were treated separately. Sequencing reads were aligned non-strand specifically to the zebrafish GRCz10 genome using Tophat v2.0.13 (Kim et al. (2013), Genome Biol, 14(4), R36) with Bowtie v2.2.3.0 (Langmead & Salzberg (2012), Nat Methods, 9(4), 357-359), guided by splice junctions defined by cDNA-to-genomic alignments from NCBI Release 104. Transcriptional units were defined separately for each sample using Cufflinks v2.2.1 (Roberts et al. (2011), Bioinformatics, 27(17):2325-2329), and the resulting transcript GTF files were combined between samples (sibling and *clo*^{*m39*/*m39*}) and reconciled with NCBI gene annotations using the Cufflinks tool *cuffmerge.* To perform differential expression between sibling and *clo*^{*m39*/*m39*}, read counts per gene for each sample were calculated using the Bedtools v2.21.0 *intersect* command (Quinlan & Hall, 2010) between the alignment BAM file and the exons defined above. Only reads with a MAPQ score > 0 aligning at least 5 nt of an exon were counted. Log2 fold change between sibling and *clo*^{*m39*/*m39*} was calculated per gene using counts normalized per million aligned reads (RPM), with a pseudocount of 0.5 added to each count. To generate p values, the Cufflinks tool *cuffdiff* was used on the Cufflinks FPKM values of sibling versus *clo*^{*m39*/*m39*}. Sequencing reads were also aligned to *Danio rerio* EST sequences from GenBank dbEST using Bowtie, and differential expression per EST between sibling and *clo*^{*m39*/*m39*} was similarly calculated.

### Targeted mutagenesis using TALENs and CRISPR/Cas9

TALEN or CRISPR/Cas9 technology was used to target each of the candidate genes identified. TALEN-binding sites were selected using TALEN Targeter 2.0 (https://tale-nt.cac.cornell.edu/) and constructs were assembled as previously described (Cermak et al. (2011), Nucleic Acids Res, 39(12); Beddell et al. (2012), Nature, 491:114-118). RNA was synthesized with mMESSAGE mMachine kit (Ambion) and between 100-150 pg per TALEN arm was injected into one-cell stage embryos to generate germline carrier founder (F₀) mutants. For CRISPR/Cas9, sgRNAs for candidate genes were designed using the CRISPR Design online toolbox (http://crispr.mit.edu). Guide RNAs were made by ligating annealed primers into the pT7-gRNA vector and the pT3Ts-nCas9n plasmid (Addgene) was used to synthesize Cas-9 mRNA as previously described (Jao et al. (2013), PNAS 110:13904-13909). To generate germline carrier F₀ mutants, between 50-75 pg of sgRNA and 250-500 pg of Cas9 mRNA were co-injected into one-cell stage embryos. To determine the efficacy of individual TALEN or CRISPR/Cas9 injections, DNA was extracted from injected embryos at 48 hpf and subjected to high resolution melting analysis (HRMA) (Eco-Ilumina) and sequencing was used to assess TALEN or CRISPR/Cas9 efficiency. F₀ founders for each gene were identified by outcrossing TALEN or CRISPR/Cas9 injected fish with AB and screening the offspring at 48 hpf using HRMA. PCR products from F₀ founders that showed promising HRMA melt curves were cloned and sequenced, and those that gave interesting mutations (ie. frame-shifts) were grown and backcrossed to *clo^{m39}* in order to assess any possible vascular phenotype. Heterozygous intercrosses of individual adult F₁ *Tg(kdrl:GFP)* mutants were also used to assess any possible vascular phenotype.

### 5' and 3' Rapid Amplification of cDNA Ends (RACE)

5' and 3' RACE PCR was performed using the SMARTer RACE cDNA amplification kit (Clontech). Briefly, the following primers were used to amplify the 5' and 3' ends of the *npas4l* cDNA from 24 hpf and tailbud cDNA (Fig. 15); 3'GSP1 - OAS1323; 3'GSP2 - OAS1324; 5'GSP1 - OAS1325; 5'GSP2 - OAS1326.

### Imaging

Zebrafish embyos were immobilized with a low dose of tricaine and mounted in 1% low melting agarose (GeneMate). Brightfield images were acquired using a Nikon SMZ25 and confocal images were acquired using a Zeiss LSM780 with a W Plan-Apochromat 20X/NA 0.8 DIC objective lens.

### Whole-mount in situ hybridization

Embryos were collected at different developmental stages and fixed with 4% paraformaldehyde in PBS at 4°C overnight. Whole-mount in situ hybridization was performed as described previously (Thisse and Thisse (2008), Nat Protoc., 3(1):59-69). The *npas4-like* probe was generated with the following primers: ACTCGGGCATCAGGAGGATC and GACACCAGCATACGACACACAAC. T7 was used for transcription and digoxigenin for labeling (Roche). Images were acquired using a Nikon SMZ25.

### Expression Analysis

RT-qPCR was performed in the CFX Connect Real-Time System (BIORAD). RNA was isolated utilizing Trizol (Sigma) and cDNA was synthesized using the Superscript III First-Strand synthesis system (Invitrogen). Histograms show the results of three different experiments (3 pools of embryos per developmental stage or treatment) and each pool consisted of 20-40 embryos. Primers were designed using PerlPrimer, and *gapdh* was used for normalization.

### Overexpression and rescue studies

Full-length *npas4l* cDNA was amplified from cDNA and cloned into pCS2⁺. mRNA was synthesized using the mMESSAGE mMACHINE kit and injected at different concentrations into 1-cell stage *Tg(kdrl:eGFP)* zebrafish embryos. Concentrations between 25-250 pg were tested to rescue *clo*^{s5} and *clo*^{m39}. As *clo*^{s5} and *clo*^{m39} mutants entirely lack anterior endothelium at 28 hpf, the presence of *Tg(kdrl:eGFP)* positive endothelial cells in the anterior of embryos was used to quantify the ability of each mRNA to rescue the *clo* phenotype. For overexpression studies, between 25-50 pg of *npas4l* mRNA was injected into AB embryos at the 1-cell stage.

### Phylogenetic Analysis

A phylogenetic tree was calculated using *Phylogeny.fr* (Phylogeny.fr: robust phylogenetic analysis for the non-specialist) based on the amino acid sequence of the bHLH domain and the first PAS domain of the human proteins NPAS1-NPAS4, HIF1, EPAS1 and AHR together with the protein encoded by *cloche* with highly similar results (close similarity to NPAS4). The online tool was run with standard settings.

### Microinjection of morpholinos

The following morpholinos (MOs) were purchased from GeneTools LLC (Philomath, OR) and injected at the indicated amounts:
*npas4I* ATG MO (1 ng): GAGTCTCCGCAGCTCATCTCACA (SEQ ID NO: 33)
*npas4I* I2E3 MO (6 ng): CACCTGGAACACACAGTGGAGGATT (SEQ ID NO: 34)
*tal1* MO (6.5 ng): AATGCTCTTACCATCGTTGATTTCA (SEQ ID NO: 35) (Dooley et al. (2005), loc. lit.)
*etv2* MO1 (4 ng): TTGGTACATTTCCATATCTTAAAGT (SEQ ID NO: 36) (Sumanas and Lin (2006), PLos Biol. 4:e10)
*etv2* MO2 (4 ng): CACTGAGTCCTTATTTCACTATATC (SEQ ID NO: 37) (Sumanas and Lin (2006), loc. lit.)

### Genotyping

High resolution melt analysis (HRMA) was used to genotype both the *npas4I^{bns59}* and *clo^{s5}* alleles. Embryos or fin-clips were placed in PCR tubes, with 50 µl of elution buffer (10 mM Tris-HCl, pH 8.5) containing 1 mg/ml Proteinase K and incubated at 55° C for 2 hrs. The samples were then heated to 95°C for 10 min to inactivate the Proteinase K. DyNAmo SYBR green (Thermo Fisher Scientific, Waltham, MA) was used to perform PCR and HRMA in an Eco Real-Time PCR System (Illumina, San Diego, CA). PCR protocols: 95°C for 15 sec, then 40 cycles of 95°C for 2 sec, 60°C for 2 sec, and 72°C for 2 sec. Following the PCR, a high-resolution melt curve was generated by collecting SYBR-green fluorescence data in the 65-95°C range. The analyses of genotype were performed on plots of normalised derivative fluorescence.

### Example 2 - Cloning and identification of the cloche gene npas4l

The *cloche* locus was previously mapped to one of the telomeres of chromosome 13 as a CA repeat marker, z1496, which is tightly linked to the locus based on genotyping 2359 mutant embryos was identified⁴. In order to provide higher genetic resolution to the *cloche* region and narrow down the critical interval that contains the *cloche* mutation, an additional 7920 mutant embryos were genotyped for a total of 10,279, which allowed to identify additional zero recombinant markers as well as closely linked flanking markers (Fig. 4B; Fig. 13). Searching the available genomic databases led to the identification of several genes in the *cloche* locus including *Iclat* (aka *lycat*)¹³, *lbh, klhdc3,* and *inpp5a.* These genes were sequenced in multiple different ENU-induced *cloche* alleles looking for significant genetic alterations and also carried out functional studies including morpholino knockdowns and mRNA rescues, but failed to generate fully convincing data that any of them corresponded to the *cloche* gene. Because the physical assembly of the *cloche* region did not match the available genetic map, and due to the large number of gaps in the assembly of the critical locus (Fig. 4C), it was reasoned that additional genes were located within the region and that one of them could be the *cloche* gene.

In order to identify these missing genes, the *cloche* deletion allele *m39* was used, which was determined as lacking a large number of genetic markers around the *cloche* locus². *In situ* hybridization analyses examining the expression of the earliest known regulators of endothelial and hematopoietic cell differentiation, the ETS domain protein gene *etv2*⁶ and the bHLH protein gene *tal1*^{3,19-22} had determined that the earliest time one could observe differences between *cloche* mutants and their wild-type siblings was during early somitogenesis^{3,7} (data not shown). Thus a large number of single embryos were collected from *clo^{m39}* heterozygous intercrosses between 90% epiboly and tail bud stages, reasoning that the gene should be expressed prior to the onset of the phenotype. From these individual embryos, both DNA and RNA was extracted, and after genotyping them with z1496, which is deleted in *clo^{m39}* mutants, and z1452, which is not deleted in *clo^{m39}* mutants (Fig. 4D, E), pooled wild-type and mutant RNA. Sequencing total RNA and polyA enriched RNA generated -40 million reads per sample, which were compared to the entire collection of *Danio rerio* expressed sequence tags (ESTs), and also mapped to GRCz10, the latest zebrafish genome assembly at the time. This extensive bioinformatics analysis led to the identification of several new candidate genes as well as previously identified genes in the *cloche* region (Fig. 4F; Fig. 14), which were then mutated using TALEN or CRISPR/Cas9 technologies. For each of these genes, identified founders carrying likely deleterious mutations were crossed to the *clo^{m39}* allele for complementation testing, and once available, the F1's were intercrossed to assess vascular morphology by examining *Tg(kdrl:eGFP)* expression in F2 embryos. Out of the 17 individual mutants tested in this manner, only *npas4l* mutants failed to complement the *clo^{m39}* allele, and displayed a vascular phenotype in the F2 generation (Fig. 4G). Confirming the speculation, *npas4I* did not map to the physical assembly of the *cloche* region but to a small, unassigned scaffold (KN150101.1, Fig. 4C). From the RNA-seq data, a sequence translating into a continuous reading frame of 293 amino acids was identified that shows an identity of 33% over a length of 113 amino acids to the C-terminal region of Npas4 from *Cynoglossus semilaevis.* This sequence was used to design a CRISPR sgRNA to induce a molecular lesion in *npas4I.* To isolate a full-length *npas4l* cDNA, the sequence of this fragment was extended using 5' and 3' RACE (Fig. 8) and a clone of 4286 bp encoding a PAS domain containing bHLH protein of 647 amino acids was assembled. This protein shares highest homology with NPAS4 (Fig. 9), a key regulator of activity-regulated neuronal gene programs and memory formation in mouse¹⁴⁻¹⁶. The NPAS4 ortholog in *Drosophila,* Dysfusion, regulates the development of the trachea¹⁷, the fly vascular system, and Trachealess, an NPAS4 paralog, is a master regulator of tracheal cell fates^{18,19}, indicating the importance of bHLH-PAS domain proteins in vascular development throughout evolution. The zebrafish genome comprises three *npas4* genes, only two of which had been previously identified. Also the genomic region for the *npas4l* gene was assembled; since KN150101.1 did not contain the full *npas4l* sequence, whole-genome PacBio and Illumina sequencing data was used to extend the scaffold from a length of 25 kbp to 35 kbp. Both mapped RNA-seq reads and gene prediction by AUGUSTUS show that *npas4I,* like other *npas4* genes, consists of eight exons (Fig. 5A), extending from 24 kbp to 29 kbp on the improved scaffold. In addition, the coding sequence for *klhdc3* could be aligned in part to both a region upstream of *npas4l* and the end of the GRCz10 chromosome 13 sequence, confirming that the extended version of KN150101.1 should be placed in reverse-complementary orientation downstream of *klhdc3* on chromosome 13. To further test whether *npas4l* is indeed the *cloche* gene, first the phenotype of the CRISPR-generated allele (*npas4I^{bns59}*) was analyzed, which carries an 8 bp deletion in the 3' end of the gene, leading to a premature stop codon at amino acid 545 after 19 missense amino acids (Fig. 5B) and vascular and blood gene expression defects (Fig. 5C, D) were observed, clearly recapitulating the *cloche* phenotype¹⁻³. These epistasis experiments also revealed that *npas4l* acts upstream of *etv2* and *tal1*. Complementation analysis between *npas4I^{bns59}* and the ENU-induced allele *clo*^{*s5* 20} showed that these two mutations fail to complement (Fig. 5E), indicating that they are allelic. Sequencing the *npas4I* cDNA from *clo^{s5}* mutants revealed a stop codon at position 135, i.e., within the first PAS domain (Fig. 5F, G). Also a combination of ATG and splice site *npas4l* morpholinos was injected into wild-type embryos (Extended Data Fig. 6), and similarly severe blood vessel development defects were observed as those exhibited by *clo* mutants (Fig. 5H). Finally, it was found that injection of 25 pg of zebrafish *npas4l* or human *NPAS4* mRNA into embryos from *clo^{s5}* heterozygous intercrosses at different concentrations rescued the anterior population of *Tg(kdrl:eGFP)* expressing endothelial cells in *clo^{s5}* mutants (Fig. 5I, J). Thus, the results from the genetic mapping, complementation studies with a CRISPR-generated allele, identification of a severe molecular lesion in an ENU-induced allele, morpholino knockdown and mRNA rescue experiments, clearly indicate that *npas4l* is the *cloche* gene.

### Example 3 - Functional studies on npas4I and its human ortholog NPAS4

Next the spatio-temporal expression pattern of *npas4l* in comparison with *etv2* and *tal1* was *examined.* RT-qPCR experiments showed that *npas4l* expression peaks prior to the end of gastrulation and is only weakly expressed by 24 hours post fertilization (hpf) (Fig. 6A), the stage when the heart starts beating, whereas *etv2* and *tal1* expression appear to peak at midsomitogenes is stages (Fig. 6B, C). Analysis of existing RNA-seq data sets²¹ confirmed these observations (Fig. 11). Whole-mount in situ hybridization studies show that *npas4l* expression precedes the onset of *etv2* and *tal1* expression (Fig. 6D, H, L), and that all three genes display a very similar expression pattern in the anterior and posterior lateral plate mesoderm, the tissue containing the endothelial and hematopoietic precursors. This expression pattern in the posterior lateral plate mesoderm is also similar to that exhibited by *gata1a*, which marks erythroid lineage cells and is clearly visible by the 2-somite stage (Fig. 12). Notably, *npas4l* expression at early stages is uniquely observed in a rostromedial population of cells in the anterior lateral plate mesoderm (Fig. 6E; asterisk) that is initially negative for *etv2* and *tal1* expression (Fig. 6I, M), and likely corresponds to myeloid precursors. Similarly, in the posterior lateral plate mesoderm, *npas4l* expression appears to extend more posteriorly (Fig. 6F) compared to *etv2* and *tal1* expression (Fig. 6J, N), which may reflect its earlier activation in these endothelial and hematopoietic precursors. By the 18-somite stage, the levels of *npas4l* expression are clearly decreasing (Fig. 6G), in agreement with the RT-qPCR data, while the levels of *etv2* and *tal1* expression remain high (Fig. 6K, O). Thus, the onset of *clo*/*npas4I* expression precedes that of *etv2* and *tal1*; *clo*/*npas4I* expression is also observed in cells that are negative for *etv2* or *tal1* expression, and after peaking at late gastrulation/early somitogenesis stages it is quickly down-regulated. In order to position *clo*/*npas4I* in the hierarchy of endothelial and hematopoietic regulators, gain- and loss-of-function experiments with *etv2* and *tal1* were conducted. Injection of *etv2* or *tal1* mRNA into one-cell stage embryos had no effect on *npas4l* expression when assessed by RT-qPCR (Fig. 7A). Similarly, morpholino-mediated knock-down of *etv2* or *tal1* (Fig. 12) had no effect on *npas4l* expression when assessed by RT-qPCR (Fig. 7A) or whole-mount *in situs* (Fig. 7B). Conversely, injection of *npas4l* mRNA into one-cell stage embryos led to a broad upregulation of *etv2* (Fig. 7C) and *tal1* (Fig. 7D) expression at very early developmental stages. Altogether, these data show that Clo/Npas4I regulates the expression of the earliest known regulators of endothelial and hematopoietic differentiation, placing it at the top of the hierarchy (Fig. 7E).

The identification of the Cloche protein as a transcriptional regulator has allowed its further functional analysis. Extensive loss- and now gain-of-function studies clearly show that Cloche functions at the top of a transcriptional cascade that drives the commitment of multipotent mesodermal cells to the endothelial and hematopoietic lineages. Considering its highly restricted expression pattern, it is likely that investigating the extrinsic signals that induce *clo*/*npas4I* expression will lead to additional insights into the control of mesoderm diversification including the commitment to the endothelial and hematopoietic lineages, and away from the cardiomyocyte lineage. Similarly, the identification of the Cloche protein will facilitate the discovery of additional regulators of endothelial and hematopoietic development.

It has been reported that an NPAS4, the aryl hydrocarbon receptor AHR, can modulate the expansion and differentiation of human hematopoietic progenitors²¹. These reports, together with fly studies¹⁷⁻¹⁹ and the data herein showing that zebrafish as well as human NPAS4 can regulate the expression of early endothelial and hematopoietic lineages strongly implicate members of this family in controlling the formation of the hemato-vascular system throughout evolution. More broadly, the elucidation of Cloche/NPAS4 provides new avenues towards developing novel strategies for lineage reprogramming of stem/progenitor cells into endothelium and blood for regenerative medicine.

### REFERENCES

1 Stainier, D. Y., Weinstein, B. M., Detrich, H. W., 3rd, Zon, L. I. & Fishman, M. C. Cloche, an early acting zebrafish gene, is required by both the endothelial and hematopoietic lineages. Development 121, 3141-3150 (1995).
2 Liao, W. et al. The zebrafish gene cloche acts upstream of a flk-1 homologue to regulate endothelial cell differentiation. Development 124, 381-389 (1997).
3 Liao, E. C. et al. SCL/Tal-1 transcription factor acts downstream of cloche to specify hematopoietic and vascular progenitors in zebrafish. Genes Dev 12, 621-626 (1998).
4 Liao W., Ho C. Y., Yan Y. L., Postlethwait J., Stainier D. Y. Hhex and scl function in parallel to regulate early endothelial and blood differentiation in zebrafish. Development 127, 4303-13 (2000).
5 Schoenebeck J. J., Keegan B. R., Yelon D. Vessel and blood specification override cardiac potential in anterior mesoderm. Dev Cell 13, 254-67 (2007).
6 Sumanas, S., Jorniak, T. & Lin, S. Identification of novel vascular endothelial-specific genes by the microarray analysis of the zebrafish cloche mutants. Blood 106, 534-541(2005).
7 Liu, F., Walmsley, M., Rodaway, A. & Patient, R. FIi1 acts at the top of the transcriptional network driving blood and endothelial development. Curr Biol 18, 1234-1240 (2008).
8 Begley, C. G. et al. The gene SCL is expressed during early hematopoiesis and encodes a differentiation-related DNA-binding motif. Proc Natl Acad Sci USA 86, 10128-32 (1989).
9 Endoh, M., Ogawa, M., Orkin, S. & Nishikawa, S. SCL/tal-1-dependent process determines a competence to select the definitive hematopoietic lineage prior to endothelial differentiation. EMBO J 21, 6700-6708 (2002).
10 Van Handel B. et al. ScI represses cardiomyogenesis in prospective hemogenic endothelium and endocardium. Cell 150, 590-605 (2012).
11 Org T. et al. ScI binds to primed enhancers in mesoderm to regulate hematopoietic and cardiac fate divergence. EMBO J. 34, 759-77 (2015).
12 Vogeli, K. M., Jin, S. W., Martin, G. R. & Stainier, D. Y. A common progenitor for haematopoietic and endothelial lineages in the zebrafish gastrula. Nature 443, 337-39 (2006).
13 Xiong, J. W., Yu, Q., Zhang, J. & Mably, J. D. An acyltransferase controls the generation of hematopoietic and endothelial lineages in zebrafish. Circ Res 102, 1057-1064 (2008).
14 Lin, Y. et al. Activity-dependent regulation of inhibitory synapse development by Npas4. Nature 455, 1198-1204 (2008).
15 Ooe, N., Saito, K., Mikami, N., Nakatuka, I. & Kaneko, H. Identification of a novel basic helix-loop-helix-PAS factor, NXF, reveals a Sim2 competitive, positive regulatory role in dendritic-cytoskeleton modulator drebrin gene expression. Mol Cell Biol 24, 608-616 (2004).
16 Spiegel, I. et al. Npas4 regulates excitatory-inhibitory balance within neural circuits through cell-type-specific gene programs. Cell 157, 1216-1229 (2014).
17 Jiang, L. & Crews, S. T. The Drosophila dysfusion basic helix-loop-helix (bHLH)-PAS gene controls tracheal fusion and levels of the trachealess bHLH-PAS protein. Mol Cell Biol 23, 5625-5637 (2003).
18 Wilk, R., Weizman, I., & Shilo, B. Z. trachealess encodes a bHLH-PAS protein that is an inducer of tracheal cell fates in Drosophila. Genes Dev 10, 93-102 (1996).
19 Isaac, D. D. & Andrew, D. J. Tubulogenesis in Drosophila: a requirement for the trachealess gene product. Genes Dev 10, 103-117 (1996).
20 Field, H. A., Dong, P. D., Beis, D. & Stainier, D. Y. Formation of the digestive system in zebrafish. II. Pancreas morphogenesis. Dev Biol 261, 197-208 (2003).
21 Pauli A. et al. Systematic identification of long noncoding RNAs expressed during zebrafish embryogenesis. Genome Res. 22, 577-91 (2012).

## Claims

1. A method for producing endothelial cells from progenitor cells, said method comprising inducing Neuronal PAS domain protein 4 (NPAS4) function in the progenitor cells.

2. A method for producing hematopoietic cells from progenitor cells, said method comprising inducing NPAS4 function in the progenitor cells.

3. The method of claim 1, wherein the progenitor cells are embryonic stem cells (ESCs), induced pluripotent stem cells (iPS), mesodermal progenitor cells, hemangioblasts, endothelial progenitor cells (EPCs), fibroblasts or a mixture thereof.

4. The method of claim 2, wherein the progenitor cells are embryonic stem cells (ESCs), induced pluripotent stem cells (iPSs), mesodermal progenitor cells, hemangioblasts, angioblasts, hematopoietic stem cells (HSCs), or a mixture thereof.

5. The method of any one of claims 1 to 4, wherein the NPAS4 function is induced by
(a) a polypeptide comprising or consisting of human NPAS4 or a species ortholog thereof; or
(b) a nucleic acid molecule encoding the amino acid sequence of (a).

6. The method of any one of claims 1 to 4, wherein the NPAS4 function is induced by
(i) a polypeptide comprising or consisting of an amino acid sequence being selected from the group consisting of SEQ ID NOs 1 to 31;
(ii) a polypeptide being at least 60% identical to an amino acid sequence being selected from the group consisting of SEQ ID NOs: 1 to 31;
(iii) a polypeptide comprising at least 600 amino acids, wherein the N-terminal portion of said polypeptide is at least 80% identical to the N-terminal half of an amino acid sequence being selected from the group consisting of SEQ ID NOs: 1 to 31;
(iv) a polypeptide being at least 20% identical to an amino acid sequence being selected from the group consisting of SEQ ID NOs 1 to 31 outside the bHLH domain, the PAS1 domain and the PAS2 domain, being at least 75% identical to an amino acid sequence being selected from the group consisting of SEQ ID NOs 1 to 31 inside the bHLH domain, and being at least 50% identical to an amino acid sequence being selected from the group consisting of SEQ ID NOs 1 to 31 inside the PAS1 domain and the PAS2 domain;
(v) a nucleic acid molecule encoding the polypeptide of any one of (i) to (iv); and/or
(vi) a vector comprising the nucleic acid molecule of (v).

7. A method for producing cardiomyocytes from progenitor cells, said method comprising inhibiting NPAS4 function in the progenitor cell.

8. The method of claim 7, wherein the progenitor cells are embryonic stem cells (ESCs), induced pluripotent stem cells (iPSs), mesodermal progenitor cells, cardiomyocyte progenitor cells, or a mixture thereof.

9. The method of claim 7 or 8, wherein the NPAS4 function is inhibited by
(a) a dominant negative mutant of NPAS4 or a species ortholog thereof or a nucleic acid molecule encoding the same;
(b) an aptamer, a ribozyme, an antibody, a protein drug, or a small molecule inhibitor against NPAS-4 or a species ortholog thereof; and/or
(c) a siRNA, a shRNA, a morpholino, a microRNA, or an antisense nucleic acid specifically interacting with a nucleic acid molecule encoding NPAS-4 or a species ortholog thereof.

10. The method of any one of claims 1 to 9 being an *ex vivo* or *in vitro* method.

11. A cell produced by the method of any one of claims 1 to 10.

12. A nucleic acid molecule encoding a transcription factor having Npas4-like function, wherein the nucleic acid molecule is selected from the group consisting of
(i) a nucleic acid molecule encoding the amino acid sequence of SEQ ID NO: 25;
(ii) a nucleic acid molecule comprising or consisting of SEQ ID NO: 32;
(iii) a nucleic acid molecule encoding an amino acid sequence being at least 70% identical to SEQ ID NO: 25;
(iv) a nucleic acid molecule encoding an amino acid sequence comprising at least 600 amino acids, wherein the N-terminal portion of said polypeptide is at least 80% identical to the N-terminal half of the amino acid sequence of SEQ ID NO: 32;
(v) a nucleic acid molecule encoding an amino acid sequence being at least 60% identical to the amino acid sequence of SEQ ID NO: 25 outside the bHLH domain, the PAS1 domain and the PAS2 domain, being at least 90% identical to the amino acid sequence of SEQ ID NO: 25 inside the bHLH domain, and being at least 80% identical to the amino acid sequence of SEQ ID NO: 25 inside the PAS1 domain and the PAS2 domain; or
(vi) a vector comprising the nucleic acid molecule of any one of (i) to (v).

13. A polypeptide encoded by the nucleic acid molecule of claim 12.

14. A pharmaceutical composition comprising
(i) the polypeptide and/or the nucleic acid molecule as defined in claim 5; or the polypeptide, the nucleic acid molecule, and/or the vector as defined in claim 6; and/or
(ii) the cell of claim 11, the nucleic acid molecule of claim 12, the polypeptide of claim 13, or a combination thereof.

15. The polypeptide and/or the nucleic acid molecule as defined in claim 5; or the polypeptide, the nucleic acid molecule, the vector as defined in claim 6; or the cell of claim 11, the nucleic acid molecule of claim 12, the polypeptide of claim 13, or a combination thereof for use in (i) inducing angiogenesis, or (ii) treating a disease selected from atherosclerotic diseases, peripheral arterial disease, wound healing disorders, stroke, infertility, scleroderma, ulcers, diabetic vascular complications, erectile dysfunction and heart disease.
